(19) Europäisches Patentamt
European Patent Office
Office européen des brevets

(11) **EP 4 782 434 A1**

(12) **EUROPEAN PATENT APPLICATION**
published in accordance with Art. 153(4) EPC

(43) Date of publication:
**29.07.2026 Bulletin 2026/31**

(21) Application number: **24867382.4**

(22) Date of filing: **14.09.2024**

(51) International Patent Classification (IPC):
***C07D 213/81*** *(2006.01)* ***A61P 35/00*** *(2006.01)*
***C07D 257/08*** *(2006.01)* ***A61K 31/444*** *(2006.01)*

(52) Cooperative Patent Classification (CPC):
**A61K 31/444; A61P 35/00; C07D 213/81; C07D 257/08**

(86) International application number:
**PCT/CN2024/119017**

(87) International publication number:
**WO 2025/060974 (27.03.2025 Gazette 2025/13)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **18.09.2023 CN 202311202521**
**29.03.2024 CN 202410375286**

(71) Applicant: **Nutshell Therapeutics (Shanghai) Co., Ltd.**
**Shanghai 201210 (CN)**

(72) Inventors:
- **ZHOU, Guoqiang**
**Shanghai 201210 (CN)**
- **ZHANG, Chengyi**
**Shanghai 201210 (CN)**

(74) Representative: **Hirons, Daniel Stuart**
**Withers & Rogers LLP**
**2 London Bridge**
**London SE1 9RA (GB)**

(54) **N-SULFONYL CARBOXAMIDE COMPOUND, AND PREPARATION METHOD THEREFOR AND USE THEREOF**

(57) Disclosed are a N-sulfonyl carboxamide compound as shown in a formula (I) for treating cancers, an isotopic marker, an enantiomer, a diastereoisomer, a solvate or a pharmaceutically acceptable salt thereof. The compound in the present invention has a good inhibitory activity on DNA polymerase θ, and has good application prospects.

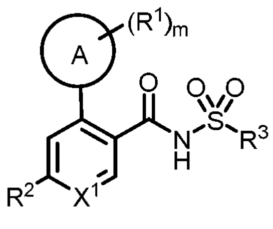

(I)

EP 4 782 434 A1

## Description

The present application claims priority to Chinese Patent Application No. 2023112025210 filed on September 18, 2023, and Chinese Patent Application No. 2024103752865 filed on March 29, 2024. The present application incorporates the entire content of the above-mentioned Chinese patent application by reference.

TECHNICAL FIELD

The present disclosure relates to a DNA polymerase θ inhibitor, and a preparation method thereof, and a use thereof, specifically to a class of N-sulfonylformamide compounds, a composition thereof, and a use thereof in the treatment and prevention of cancer.

BACKGROUND

Tumor cells exhibit genomic instability and often harbor mutations in DNA damage repair-related genes, necessitating compensatory DNA repair mechanisms to sustain survival (Hanahan, D.; Weinberg, R.A. Hallmarks of cancer: The next generation. Cell 2011, 144, 646-674.). Targeting DNA damage repair has also been clinically proven to be an effective means of tumor therapy. For example, PARP (poly(ADP-ribose) polymerase) inhibitors developed using the concept of synthetic lethality have been successfully used in the clinical treatment of tumors such as breast cancer, prostate cancer, and ovarian cancer with BRCA1/2 germline mutations (Maddison Rose et al. PARP Inhibitors: Clinical Relevance, Mechanisms of Action and Tumor Resistance. Front. Cell Dev. Biol. 8:564601.).

Regular repair of DNA damage is crucial for mammalian cells to maintain the genetic stability of the genome and ensure cell survival. Among these, DNA double-strand breaks (DSBs) represent the most severe type of DNA damage and are primarily repaired through three main pathways. The majority of DSBs are repaired via canonical nonhomologous end joining (c-NHEJ), which does not require a homologous template or requires only minimal homologous template. This process involves the ligation of DNA ends by inserting or deleting a few base pairs at the break site (Chang HHY et al. Non-homologous DNA end joining and alternative pathways to double-strand break repair. Nat Rev Mol Cell Biol. 2017, 18(8):495-506). During the S and G2 phases of the cell cycle in mitosis, sister chromatids can serve as repair templates, primarily facilitating the second error-free repair pathway known as homologous recombination (HR). The third DSB repair pathway is microhomology-mediated end joining (TMEJ/MMEJ, polymerase theta-mediated end joining/microhomology-mediated end joining) mediated primarily by DNA polymerase θ (Polθ, encoded by POLQ), which was originally named alternative pathway for non-homologous end joining (alt-EJ) and serves as a backup pathway for DSB repair when c-NHEJ or HR is impaired. However, a growing body of research indicates that TMEJ also plays a role in other DNA repair pathways and is indispensable for certain specific types of DNA damage, such as the collapse of replication forks caused by replication-blocking lesions (e.g., interstrand crosslinks) in sister chromatids. (Sfeir A, Symington LS. Microhomology-Mediated End Joining: A Back-up Survival Mechanism or Dedicated Pathway? Trends Biochem Sci. 2015, 40(11):701-714).

Polθ is a Family A multifunctional DNA polymerase that has been identified as a key protein in the TMEJ pathway (Kent T et al. Mechanism of microhomology-mediated end-joining promoted by human DNA polymerase θ. Nat Struct Mol Biol. 2015, 22(3):230-7;). The human Polθ protein (2590 amino acids) is primarily composed of three domains: a C-terminal polymerase domain (POLθ-pol, 1819-2590aa) having DNA polymerase activity, an N-terminal helicase-like domain (POLθ-hel, 32-899aa) simultaneously having ATPase activity and helicase activity, and an unstructured central domain connecting the two. Currently, POLθ has been found to be the only polymerase in eukaryotic animals with helicase activity. Both POLθ-hel and POLθ-pol are essential for TMEJ repair, and inhibiting either enzymatic activity will inhibit TMEJ activity; these are the two main directions for POLθ inhibitors currently under development (Beagan K et al. Drosophila DNA polymerase theta utilizes both helicase-like and polymerase domains during microhomology-mediated end joining and interstrand crosslink repair. PLoS Genet. 2017, 13(5):e1006813).

POLθ-hel possesses ATPase activity and helicase activity, and includes a Rad51-binding site. In an ATP-dependent manner, POLθ-hel causes the dissociation of Rad51 or RPA proteins bound to ssDNA overhangs, thereby promoting the annealing of ssDNA strands (a key step in TMEJ), ultimately leading to the inhibition of the HR pathway and effectively promoting the TMEJ process (Mateos-Gomez et al. The Helicase Domain of Polθ Counteracts RPA to Promote Alt-NHEJ. Nat. Struct. Mol. Biol. 2017, 24 (12), 1116-1123). Therefore, inhibiting the ATPase activity of POLθ-hel can effectively suppress the TMEJ repair activity of POLθ, which has become a crucial direction in the development of POLθ inhibitors (Ceccaldi, R. et al. Homologous-recombination-deficient tumours are dependent on Polθ-mediated repair. Nature 2015, 518, 258-262).

Polθ is non-essential in healthy cells but is upregulated in breast, lung, and ovarian cancers. Its overexpression is associated with homologous recombination repair deficiency and poor clinical outcomes (Ceccaldi R et al. Homologous-recombination-deficient tumours are dependent on Polθ-mediated repair. Nature. 2015, 518(7538):258-62). In the context

of HR deficiency, loss of POLθ has been shown to impair cell viability, revealing a synthetic lethal relationship between POLθ and HR factors (e.g., POLθ and FA/BRCA deficiency are synthetically lethal) (Shima N et al. The mouse genomic instability mutation chaos1 is an allele of Polq that exhibits genetic interaction with Atm. Mol Cell Biol. 2004, 24(23):10381-9; Wang Z et al., DNA polymerase θ (POLQ) is important for repair of DNA double-strand breaks caused by fork collapse. J Biol Chem. 2019, 294(11):3909-3919). More importantly, inhibition/deficiency of Polθ has shown synergistic effects with PARP inhibitors in killing HR-deficient tumor cells, and does not overlap with PARP resistance mechanisms, which holds promise for expanding the clinical application scope of PARP inhibitors and overcoming their resistance issues (Ceccaldi R et al. Homologous-recombination-deficient tumors are dependent on Polθ-mediated repair. Nature. 2015, 518(7538):258-62; Mateos-Gomez PA et al. Nature. 2015, 518(7538):254-7). In addition, inhibiting POLθ promotes the enrichment of DNA damage at the replication fork, thereby enhancing sensitivity to inhibitors such as ATR or topoisomerase. Furthermore, since p53-deficient tumors tend to utilize NHEJ and TMEJ pathways to repair DSBs, the combination of a POLθ inhibitor and a DNA-PK inhibitor has become an important combination therapy strategy for targeting p53-mutant tumors (Z. Wang, et al. DNA polymerase (POLQ) is important for repair of DNA double-strand breaks caused by fork collapse. J. Biol. Chem., 294 (2019), pp. 3909-3919; R.J. Kumar, et al. Hyperactive end joining repair mediates resistance to DNA damaging therapy in p53- deficient cells. bio Rxiv., Published online April 3, 2020).

Therefore, for cancer types with DNA repair deficiencies, Polθ is a very attractive new target for synthetic lethal therapy. Developing small-molecule inhibitors that inhibit the POLθ-hel/ATPase activity is an effective means of targeting Polθ and is of significant importance.

SUMMARY

The present disclosure provides a class of compounds, as well as a preparation method thereof and a use thereof, to overcome the defects in the prior art. The compound according to the present disclosure has a good inhibitory activity on DNA polymerase 0.

The present disclosure provides a compound of formula (I), or an isotopically labeled compound, enantiomer, diastereoisomer, or solvate thereof, or a pharmaceutically acceptable salt thereof, wherein the compound of formula (I) is selected from the following structures:

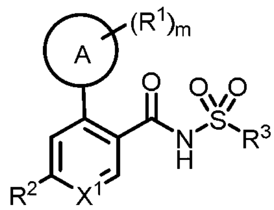

(I)

wherein

- $X^1$ is CH or N;
- ring A is phenyl or a six-membered heteroaryl containing 1 to 3 N atoms;
- m is 0, 1, 2, 3, or 4;
- each $R^1$ is independently selected from deuterium, halogen, C1-C6 alkyl, C3-C6 cycloalkyl, C1-C6 haloalkyl, deuterated C1-C6 alkyl, C1-C6 alkoxy, C3-C6 cycloalkoxy, C1-C6 haloalkoxy, deuterated C1-C6 alkoxy, hydroxyl, cyano, C2-C6 alkynyl, and $-NR^{1x}R^{1y}$; $R^{1x}$ and $R^{1y}$ are each independently H, C1-C6 alkyl, C1-C6 haloalkyl, or C3-C6 cycloalkyl;
- $R^2$ is selected from hydrogen, cyano, hydroxyl, $-NR^{2x}R^{2y}$, $-CONR^{2x}R^{2y}$, $-NR^{2x}COR^{2y}$, C1-C6 alkyl unsubstituted or substituted with one or more substituents selected from Group A, C1-C6 alkoxy unsubstituted or substituted with one or more substituents selected from Group A, C2-C6 alkenyl unsubstituted or substituted with one or more substituents selected from Group A, C2-C6 alkynyl unsubstituted or substituted with one or more substituents selected from Group A, 3- to 8-membered heterocyclyl unsubstituted or substituted with one or more substituents selected from Group A, C3-C6 cycloalkyl unsubstituted or substituted with one or more substituents selected from Group A, C6-C10 aryl unsubstituted or substituted with one or more substituents selected from Group A, and 5- to 6-membered heteroaryl unsubstituted or substituted with one or more substituents selected from Group A;
- the substituents of Group A include: oxo, deuterium, halogen, C1-C6 alkyl, C3-C6 cycloalkyl unsubstituted or substituted with one or more substituents selected from Group A1, C6-C10 aryl unsubstituted or substituted with one or more substituents selected from Group A1, 3- to 8-membered heterocyclyl unsubstituted or substituted with

one or more substituents selected from Group A1, 5- to 6-membered heteroaryl unsubstituted or substituted with one or more substituents selected from Group A1, C1-C6 haloalkyl, deuterated C1-C6 alkyl, C1-C6 alkoxy, C3-C6 cycloalkoxy, C1-C6 haloalkoxy, deuterated C1-C6 alkoxy, hydroxyl, cyano, - $NR^{3x}R^{3y}$, $-CONR^{3x}R^{3z}$, and $-NR^{3x}COR^{3z}$;

the substituents of Group A1 include: halogen, hydroxyl, cyano, C1-C6 alkyl, C1-C6 haloalkyl, deuterated C1-C6 alkyl, C1-C6 alkoxy, and C1-C6 haloalkoxy;

$R^{2x}$, $R^{2y}$, $R^{3x}$, $R^{3y}$, and $R^{3z}$ are each independently H, C1-C6 alkyl, C1-C6 haloalkyl, deuterated C1-C6 alkyl, or C3-C6 cycloalkyl;

or $R^{2x}$ and $R^{2y}$, together with the N atom to which they are attached, form a 4- to 6-membered heterocyclyl containing 1 to 2 N atoms; said 4- to 6-membered heterocyclyl may be unsubstituted or substituted with one or more substituents selected from oxo, C1-C6 alkyl, C3-C6 cycloalkyl, hydroxyl, amino, halogen, and cyano;

or $R^{3x}$ and $R^{3y}$, together with the N atom to which they are attached, form a 4- to 6-membered heterocyclyl containing 1 to 2 N atoms; said 4- to 6-membered heterocyclyl may be unsubstituted or substituted with one or more substituents selected from oxo, C1-C6 alkyl, C3-C6 cycloalkyl, hydroxyl, amino, halogen, and cyano;

$R^3$ is $-(CR_4R_5)_nR^6$;

$R^4$ and $R^5$ are each independently selected from hydrogen, deuterium, halogen, C1-C6 alkyl, deuterated C1-C6 alkyl, and C1-C6 haloalkyl;

n is 0, 1, 2, or 3;

$R^6$ is C1-C6 alkyl unsubstituted or substituted with one or more substituents selected from Group B, 3- to 8-membered heterocyclyl unsubstituted or substituted with one or more substituents selected from Group B, 5- to 10-membered heteroaryl unsubstituted or substituted with one or more substituents selected from Group B, C3-C6 cycloalkyl unsubstituted or substituted with one or more substituents selected from Group B, or C6-C10 aryl unsubstituted or substituted with one or more substituents selected from Group B;

the substituents of Group B include: oxo, deuterium, halogen, hydroxyl, cyano, mercapto, C2-C6 alkenyl, C2-C6 alkynyl, C1-C6 alkyl unsubstituted or substituted with one or more substituents selected from Group C, C1-C6 alkoxy unsubstituted or substituted with one or more substituents selected from Group C, $-(CH_2)_iNR^7R^8$, $-(CH_2)_iCONR^7R^8$, $-(CH_2)_iNR^7COR^8$, $-(CH_2)_iSO_2R^7$, $-(CH_2)_iSO_2NR^7R^8$, $-(CH_2)_iOR^7$, C6-C10 aryl, C3-C6 cycloalkyl, 3- to 8-membered heterocyclyl, and 5- to 10-membered heteroaryl;

i is 0, 1, 2, or 3;

$R^7$ and $R^8$ are each independently H, deuterium, C1-C6 alkyl, C1-C6 haloalkyl, or C3-C6 cycloalkyl;

the substituents of Group C include: deuterium, halogen, hydroxyl, C1-C6 alkoxy, cyano, amino, C3-C6 cycloalkyl, and deuterated C1-C6 alkoxy;

the heteroatoms in the 3- to 8-membered heterocyclyl, 5- to 10-membered heteroaryl, and 5- to 6-membered heteroaryl are independently selected from 1, 2, or 3 of N, O, and S.

In a preferred technical solution, ring A is phenyl or pyridyl.

In a preferred technical solution,

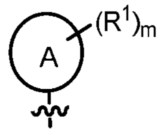

is

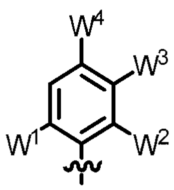,
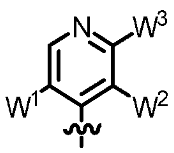,
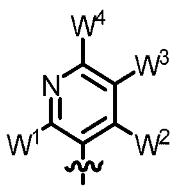, or
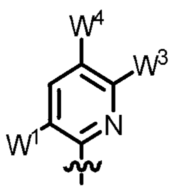;

preferably,

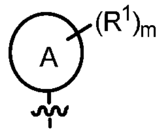

is

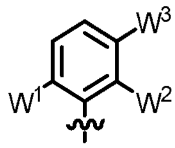

or

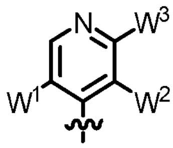

;

$W^1$, $W^2$, $W^3$, and $W^4$ are each independently selected from hydrogen, deuterium, halogen, C1-C6 alkyl, C3-C6 cycloalkyl, C1-C6 haloalkyl, deuterated C1-C6 alkyl, C1-C6 alkoxy, C3-C6 cycloalkoxy, C1-C6 haloalkoxy, deuterated C1-C6 alkoxy, hydroxyl, cyano, C2-C6 alkynyl, and $-NR^{1x}R^{1y}$; $R^{1x}$ and $R^{1y}$ are each independently H, C1-C6 alkyl, C1-C6 haloalkyl, or C3-C6 cycloalkyl;

further preferably,

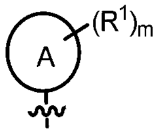

is

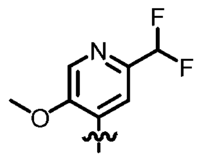

,

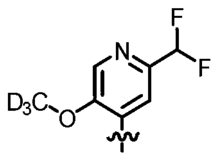

,

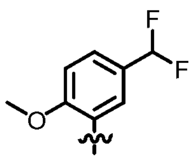

,

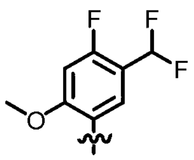

,

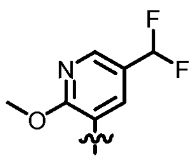

,

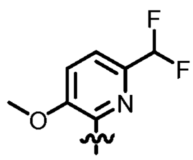

,

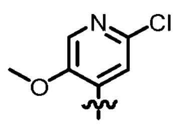

,

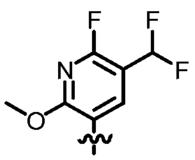

, or

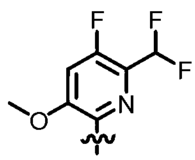

; preferably,

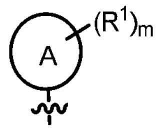

is

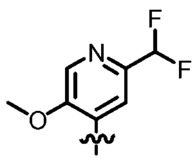

or

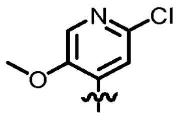.

In a preferred technical solution, $R^2$ is selected from C1-C6 alkyl unsubstituted or substituted with one or more substituents selected from Group A, C1-C6 alkoxy unsubstituted or substituted with one or more substituents selected from Group A, C2-C6 alkynyl unsubstituted or substituted with one or more substituents selected from Group A, 3- to 8-membered heterocyclyl unsubstituted or substituted with one or more substituents selected from Group A, C3-C6 cycloalkyl unsubstituted or substituted with one or more substituents selected from Group A, C6-C10 aryl unsubstituted or substituted with one or more substituents selected from Group A, and 5- to 6-membered heteroaryl unsubstituted or substituted with one or more substituents selected from Group A;

the substituents of Group A include: oxo, deuterium, halogen, C1-C6 alkyl, C3-C6 cycloalkyl unsubstituted or substituted with one or more substituents selected from Group A1, C6-C10 aryl unsubstituted or substituted with one or more substituents selected from Group A1, 3- to 8-membered heterocyclyl unsubstituted or substituted with one or more substituents selected from Group A1, 5- to 6-membered heteroaryl unsubstituted or substituted with one or more substituents selected from Group A1, C1-C6 haloalkyl, deuterated C1-C6 alkyl, C1-C6 alkoxy, C3-C6 cycloalkoxy, C1-C6 haloalkoxy, deuterated C1-C6 alkoxy, hydroxyl, cyano, - $NR^{3x}R^{3y}$, $-CONR^{3x}R^{3z}$, and $-NR^{3x}COR^{3z}$;

the substituents of Group A1 include: halogen, hydroxyl, cyano, C1-C6 alkyl, C1-C6 haloalkyl, deuterated C1-C6 alkyl, C1-C6 alkoxy, and C1-C6 haloalkoxy;

$R^{3x}$, $R^{3y}$, and $R^{3z}$ are each independently H, C1-C6 alkyl, C1-C6 haloalkyl, deuterated C1-C6 alkyl, or C3-C6 cycloalkyl;

or $R^{3x}$ and $R^{3y}$, together with the N atom to which they are attached, form a 4- to 6-membered heterocyclyl containing 1 to 2 N atoms; said 4- to 6-membered heterocyclyl may be unsubstituted or substituted with one or more substituents selected from oxo, C1-C6 alkyl, C3-C6 alkyl, hydroxyl, amino, halogen, and cyano.

In a preferred technical solution, $R^2$ is selected from C1-C6 alkyl unsubstituted or substituted with one or more substituents selected from Group A, C1-C6 alkoxy unsubstituted or substituted with one or more substituents selected from Group A, C2-C6 alkynyl unsubstituted or substituted with one or more substituents selected from Group A, C3-C6 cycloalkyl unsubstituted or substituted with one or more substituents selected from Group A,

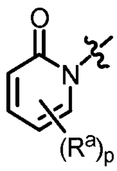, 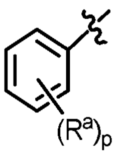, 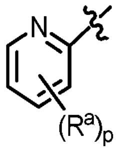, 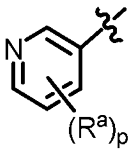, 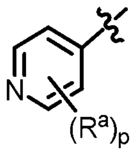, 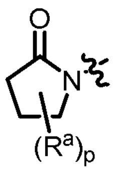, 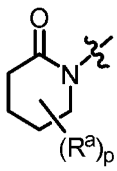, 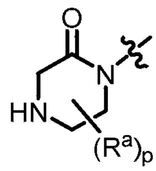,

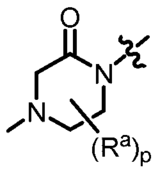, 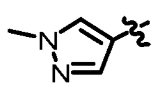, 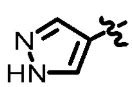, 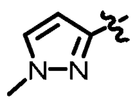, 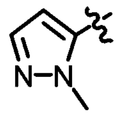, 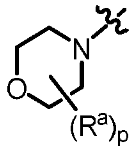, 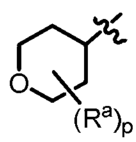,

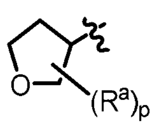, 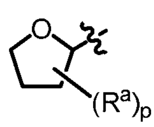, and 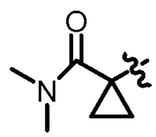;

wherein

the substituents of Group A include: deuterium, halogen, C1-C6 alkyl, C3-C6 cycloalkyl unsubstituted or substituted with one or more substituents selected from Group A1, C6-C10 aryl unsubstituted or substituted with one or more substituents selected from Group A1, 3- to 8-membered heterocyclyl unsubstituted or substituted with one or more

substituents selected from Group A1, 5- to 6-membered heteroaryl unsubstituted or substituted with one or more substituents selected from Group A1, C1-C6 haloalkyl, deuterated C1-C6 alkyl, C1-C6 alkoxy, deuterated C1-C6 alkoxy, C3-C6 cycloalkoxy, C1-C6 haloalkoxy, hydroxyl, cyano, - $NR^{3x}R^{3y}$, -$CONR^{3x}R^{3z}$, and -$NR^{3x}COR^{3z}$;

the substituents of Group A1 include: halogen, hydroxyl, cyano, C1-C6 alkyl, C1-C6 haloalkyl, deuterated C1-C6 alkyl, C1-C6 alkoxy, and C1-C6 haloalkoxy;

$R^{3x}$, $R^{3y}$, and $R^{3z}$ are each independently H, C1-C6 alkyl, C1-C6 haloalkyl, deuterated C1-C6 alkyl, or C3-C6 cycloalkyl;

or $R^{3x}$ and $R^{3y}$, together with the N atom to which they are attached, form a 4- to 6-membered heterocyclyl containing 1 to 2 N atoms; said 4- to 6-membered heterocyclyl may be unsubstituted or substituted with one or more substituents selected from oxo, C1-C6 alkyl, C3-C6 alkyl, hydroxyl, amino, halogen, and cyano;

$R^a$ is selected from hydrogen, halogen, hydroxyl, amino, cyano, C1-C6 alkyl, C1-C6 haloalkoxy, and C1-C6 alkoxy;

p is 1, 2, or 3.

In a preferred technical solution, $R^2$ is selected from C1-C6 alkyl, C1-C6 haloalkyl, deuterated C1-C6 alkyl, C1-C6 alkoxy, C1-C6 alkoxy-substituted C1-C6 alkyl,

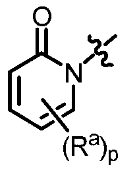,

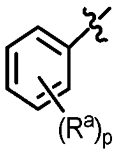, 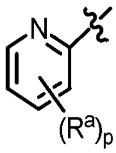, 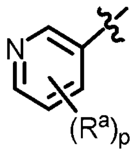, 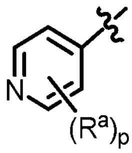, 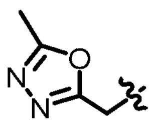, and 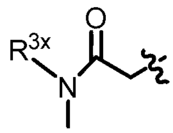;

wherein

$R^a$ is selected from hydrogen, C1-C6 alkyl, C1-C6 haloalkyl, or C1-C6 alkoxy; p is 1, 2, or 3; $R^{3x}$ is H, C1-C6 alkyl, C1-C6 haloalkyl, deuterated C1-C6 alkyl, or C3-C6 cycloalkyl.

In a preferred technical solution, $R^2$ is selected from methyl, methoxy, -$CF_3$, -$CD_3$, - $CH_2CF_3$, -$CH_2OCH_3$,

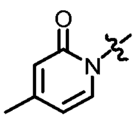, 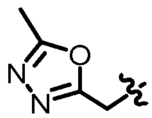, 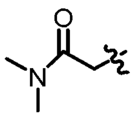, and 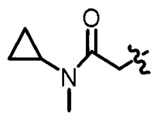.

In a preferred technical solution, $R^2$ is selected from methyl and -$CD_3$.

In a preferred technical solution,

$R^3$ is -$(CH_2)_nR^6$;

n is 0, 1, 2, or 3;

$R^6$ is 3- to 8-membered heterocyclyl unsubstituted or substituted with one or more substituents selected from Group B, 5- to 10-membered heteroaryl unsubstituted or substituted with one or more substituents selected from Group B, C3-C6 cycloalkyl unsubstituted or substituted with one or more substituents selected from Group B, or C6-C10 aryl unsubstituted or substituted with one or more substituents selected from Group B;

the substituents of Group B include: oxo, deuterium, halogen, hydroxyl, cyano, mercapto, C2-C6 alkenyl, C2-C6 alkynyl, C1-C6 alkyl unsubstituted or substituted with one or more substituents selected from Group C, C1-C6 alkoxy unsubstituted or substituted with one or more substituents selected from Group C, -$(CH_2)_iNR^7R^8$, -$(CH_2)_iCONR^7R^8$, -$(CH_2)_iNR^7COR^8$, -$(CH_2)_iSO_2R^7$, -$(CH_2)_iSO_2NR^7R^8$, and -$(CH_2)_iOR^7$;

i is 0, 1, 2, or 3;

$R^7$ and $R^8$ are each independently H, C1-C6 alkyl, C1-C6 haloalkyl, deuterated C1-C6 alkyl, or C3-C6 cycloalkyl;

the substituents of Group C include: deuterium, halogen, hydroxyl, cyano, amino, C1-C6 alkoxy, C3-C6 cycloalkyl, and deuterated C1-C6 alkoxy;

the heteroatoms in the 5- to 10-membered heteroaryl and 5- to 6-membered heteroaryl are independently selected

from 1, 2, or 3 of N, O, and S.

In a preferred technical solution,

$R^3$ is $-(CH_2)_nR^6$;
n is 1 or 2;
$R^6$ is C6-C10 aryl group (such as phenyl) unsubstituted or substituted with one or more substituents selected from Group B;
the substituents of Group B include: oxo, deuterium, halogen, hydroxyl, cyano, mercapto, C2-C6 alkenyl, C2-C6 alkynyl, C1-C6 alkyl unsubstituted or substituted with one or more substituents selected from Group C, C1-C6 alkoxy unsubstituted or substituted with one or more substituents selected from Group C, $-(CH_2)_iNR^7R^8$, $-(CH_2)_iCONR^7R^8$, $-(CH_2)_iNR^7COR^8$, $-(CH_2)_iSO_2R^7$, $-(CH_2)_iSO_2NR^7R^8$, and $-(CH_2)_iOR^7$;
i is 0, 1, 2, or 3;
$R^7$ and $R^8$ are each independently H, C1-C6 alkyl, C1-C6 haloalkyl, deuterated C1-C6 alkyl, or C3-C6 cycloalkyl;
the substituents of Group C include: deuterium, halogen, hydroxyl, cyano, amino, C1-C6 alkoxy, C3-C6 cycloalkyl, and deuterated C1-C6 alkoxy;
the heteroatoms in the 5- to 10-membered heteroaryl and 5- to 6-membered heteroaryl are independently selected from 1, 2, or 3 of N, O, and S.

In a preferred technical solution, $R^3$ is selected from -C1-C3 alkyl,

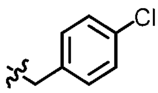,

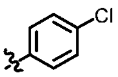, 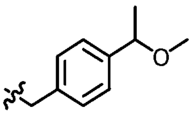, 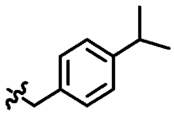, 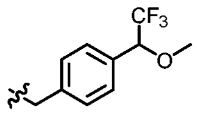, 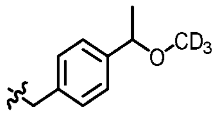

(such as

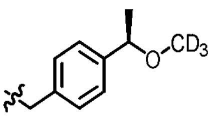 or 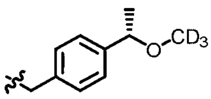), and 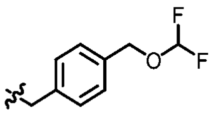.

In a preferred technical solution, $R^3$ is

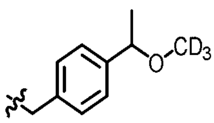.

In a preferred technical solution, the compound of formula (I) is selected from the following structures:

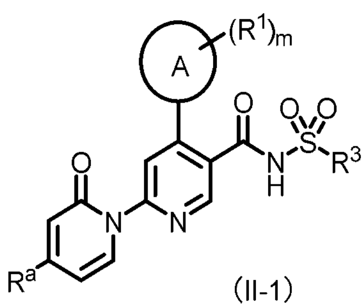 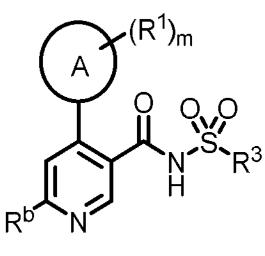 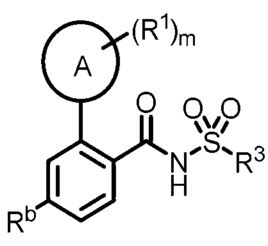

(II-1) (II-2) (II-3)

wherein

$R^a$ is selected from hydrogen, halogen, hydroxyl, amino, cyano, C1-C6 alkyl, C1-C6 haloalkyl, and C1-C6 alkoxy; $R^b$ is selected from C1-C6 alkyl unsubstituted or substituted with one or more substituents selected from Group A and C1-C6 alkoxy unsubstituted or substituted with one or more substituents selected from Group A; the substituents of Group A include: deuterium, halogen, C1-C6 alkyl, C1-C6 haloalkyl, deuterated C1-C6 alkyl, C1-C6 alkoxy, C1-C6 haloalkoxy, deuterated C1-C6 alkoxy, hydroxyl, cyano, amino, -CONR$^{3x}$R$^{3z}$, -NR$^{3x}$COR$^{3z}$, and unsubstituted or C1-C6 alkyl-substituted 5- to 6-membered heteroaryl;
ring A, $R^1$, $R^3$, $R^{3x}$, $R^{3z}$, and m are as defined above.

In a preferred technical solution, the compound of formula (I) is selected from the following structures:

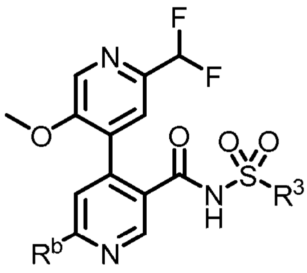
(III-1)

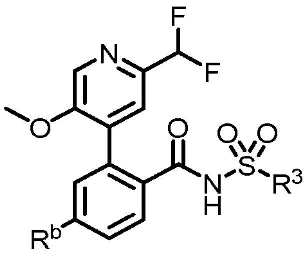
(III-2)

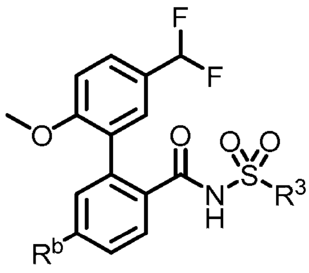
(III-3)

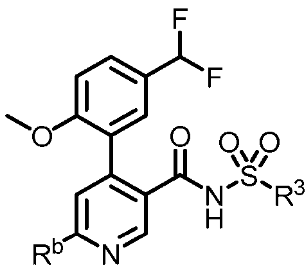
(III-4)

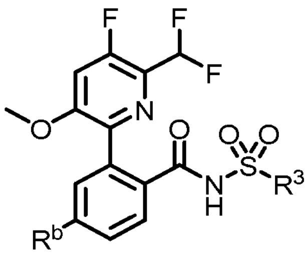
(III-5)

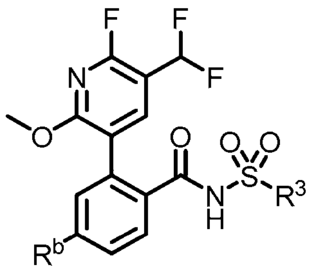
(III-6)

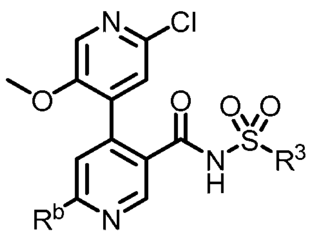
(III-7)

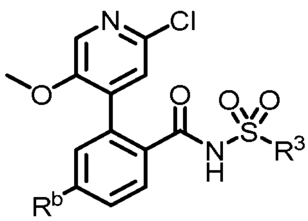
(III-8)

wherein

$R^b$ is selected from C1-C6 alkyl unsubstituted or substituted with one or more substituents selected from Group A and C1-C6 alkoxy unsubstituted or substituted with one or more substituents selected from Group A; the substituents of Group A include: deuterium, halogen, C1-C6 alkyl, C1-C6 haloalkyl, deuterated C1-C6 alkyl, C1-C6 alkoxy, C1-C6 haloalkoxy, deuterated C1-C6 alkoxy, hydroxyl, cyano, amino, -CONR$^{3x}$R$^{3z}$, -NR$^{3x}$COR$^{3z}$, and unsubstituted or C1-C6 alkyl-substituted 5- to 6-membered heteroaryl; $R^{3x}$ and $R^{3z}$ are each independently H, C1-C6 alkyl, C1-C6 haloalkyl, deuterated C1-C6 alkyl, or C3-C6 cycloalkyl;
$R^3$ is $-(CR_4R_5)_nR^6$;
$R^4$ and $R^5$ are each independently selected from hydrogen, deuterium, halogen, C1-C6 alkyl, deuterated C1-C6 alkyl, and C1-C6 haloalkyl;

n is 0, 1, 2, or 3;

$R^6$ is C1-C6 alkyl unsubstituted or substituted with one or more substituents selected from Group B, 3- to 8-membered heterocyclyl unsubstituted or substituted with one or more substituents selected from Group B, 5- to 10-membered heteroaryl unsubstituted or substituted with one or more substituents selected from Group B, C3-C6 cycloalkyl unsubstituted or substituted with one or more substituents selected from Group B, or C6-C10 aryl unsubstituted or substituted with one or more substituents selected from Group B;

the substituents of Group B include: oxo, deuterium, halogen, hydroxyl, cyano, mercapto, C2-C6 alkenyl, C2-C6 alkynyl, C1-C6 alkyl unsubstituted or substituted with one or more substituents selected from Group C, C1-C6 alkoxy unsubstituted or substituted with one or more substituents selected from Group C, $-(CH_2)_iNR^7R^8$, $-(CH_2)_iCONR^7R^8$, $-(CH_2)_iNR^7COR^8$, $-(CH_2)_iSO_2R^7$, $-(CH_2)_iSO_2NR^7R^8$, $-(CH_2)_iOR^7$, C6-C10 aryl, C3-C6 cycloalkyl, 3- to 8-membered heterocyclyl, and 5- to 10-membered heteroaryl;

i is 0, 1, 2, or 3;

$R^7$ and $R^8$ are each independently H, deuterium, C1-C6 alkyl, C1-C6 haloalkyl, or C3-C6 cycloalkyl;

the substituents of Group C include: deuterium, halogen, hydroxyl, cyano, amino, C1-C6 alkoxy, C3-C6 cycloalkyl, and deuterated C1-C6 alkoxy;

the heteroatoms in the 3- to 8-membered heterocyclyl and 5- to 10-membered heteroaryl are independently selected from 1, 2, or 3 of N, O, and S.

In a further preferred technical solution,

$R^b$ is selected from C1-C6 alkyl unsubstituted or substituted with one or more substituents selected from Group A and C1-C6 alkoxy unsubstituted or substituted with one or more substituents selected from Group A; the substituents of Group A include: deuterium, halogen, C1-C6 alkyl, C1-C6 haloalkyl, deuterated C1-C6 alkyl, C1-C6 alkoxy, deuterated C1-C6 alkoxy, hydroxyl, cyano, amino, $-CONR^{3x}R^{3z}$, $-NR^{3x}COR^{3z}$, and unsubstituted or C1-C6 alkyl-substituted 5- to 6-membered heteroaryl; $R^{3x}$ and $R^{3z}$ are each independently H, C1-C6 alkyl, C1-C6 haloalkyl, deuterated C1-C6 alkyl, or C3-C6 cycloalkyl;

$R^3$ is $-(CR_4R_5)_nR^6$;

$R^4$ and $R^5$ are each independently selected from hydrogen, deuterium, halogen, and methyl;

n is 0, 1, 2, or 3;

$R^6$ is C1-C6 alkyl unsubstituted or substituted with one or more substituents selected from Group B, 3- to 8-membered heterocyclyl unsubstituted or substituted with one or more substituents selected from Group B, 5- to 10-membered heteroaryl unsubstituted or substituted with one or more substituents selected from Group B, C3-C6 cycloalkyl unsubstituted or substituted with one or more substituents selected from Group B, or C6-C10 aryl unsubstituted or substituted with one or more substituents selected from Group B;

the substituents of Group B include: oxo, deuterium, halogen, hydroxyl, cyano, mercapto, C2-C6 alkenyl, C2-C6 alkynyl, C1-C6 alkyl unsubstituted or substituted with one or more substituents selected from Group C, C1-C6 alkoxy unsubstituted or substituted with one or more substituents selected from Group C, $-(CH_2)_iNR^7R^8$, $-(CH_2)_iCONR^7R^8$, $-(CH_2)_iNR^7COR^8$, $-(CH_2)_iSO_2R^7$, $-(CH_2)_iSO_2NR^7R^8$, and $-(CH_2)_iOR^7$;

i is 0, 1, 2, or 3;

$R^7$ and $R^8$ are each independently H, deuterium, C1-C6 alkyl, C1-C6 haloalkyl, or C3-C6 cycloalkyl;

the substituents of Group C include: deuterium, halogen, hydroxyl, cyano, amino, C1-C6 alkoxy, C3-C6 cycloalkyl, and deuterated C1-C6 alkoxy;

the heteroatoms in the 3- to 8-membered heterocyclyl and 5- to 10-membered heteroaryl are independently selected from 1, 2, or 3 of N, O, and S.

In a further preferred technical solution,

$R^b$ is selected from C1-C6 alkyl unsubstituted or substituted with one or more substituents selected from Group A and C1-C6 alkoxy unsubstituted or substituted with one or more substituents selected from Group A; the substituents of Group A include: deuterium, halogen, C1-C6 alkyl, C1-C6 alkoxy, $-CONR^{3x}R^{3z}$, $-NR^{3x}COR^{3z}$, and unsubstituted or C1-C6 alkyl-substituted 5- to 6-membered heteroaryl; $R^{3x}$ and $R^{3z}$ are each independently H, C1-C6 alkyl, C1-C6 haloalkyl, deuterated C1-C6 alkyl, or C3-C6 cycloalkyl;

$R^3$ is $-(CH_2)_nR^6$;

n is 0, 1, or 2;

$R^6$ is C1-C6 alkyl unsubstituted or substituted with one or more substituents selected from Group B, 3- to 6-membered heterocyclyl unsubstituted or substituted with one or more substituents selected from Group B, six-membered heteroaryl unsubstituted or substituted with one or more substituents selected from Group B, C3-C6 cycloalkyl unsubstituted or substituted with one or more substituents selected from Group B, or phenyl unsubstituted or

substituted with one or more substituents selected from Group B;
the substituents of Group B include: deuterium, halogen, hydroxyl, cyano, C1-C6 alkyl unsubstituted or substituted with one or more substituents selected from Group C, C1-C6 alkoxy, C1-C6 haloalkoxy, deuterated C1-C6 alkoxy, amino, $-(CH_2)_iOR^7$; the substituents of Group C include: deuterium, halogen, hydroxyl, cyano, amino, C1-C6 alkoxy, C3-C6 cycloalkyl, deuterated C1-C6 alkoxy; i is 1 or 2; $R^7$ is C1-C6 haloalkyl;
the heteroatoms in the 3- to 6-membered heterocyclyl and six-membered heteroaryl are independently selected from 1, 2, or 3 of N, O, and S.

In a further preferred technical solution,

$R^b$ is selected from C1-C6 alkyl unsubstituted or substituted with one or more substituents selected from Group A, C1-C6 alkoxy unsubstituted or substituted with one or more substituents selected from Group A; the substituents of Group A include: deuterium, halogen, C1-C6 alkyl, C1-C6 alkoxy, $-CONR^{3x}R^{3z}$, and $-NR^{3x}COR^{3z}$; $R^{3x}$ and $R^{3z}$ are each independently H, C1-C6 alkyl, C1-C6 haloalkyl, deuterated C1-C6 alkyl, C3-C6 cycloalkyl; preferably, $R^b$ is C1-C6 alkyl or deuterated C1-C6 alkyl, such as $CH_3$, $CD_3$;
$R^3$ is $-(CH_2)_nR^6$;
n is 0 or 1;
$R^6$ is C1-C6 alkyl unsubstituted or substituted with one or more substituents selected from Group B, 3- to 6-membered heterocyclyl unsubstituted or substituted with one or more substituents selected from Group B, six-membered heteroaryl unsubstituted or substituted with one or more substituents selected from Group B, C3-C6 cycloalkyl unsubstituted or substituted with one or more substituents selected from Group B, or phenyl unsubstituted or substituted with one or more substituents selected from Group B;
the substituents of Group B include: deuterium, halogen, hydroxyl, cyano, C1-C6 alkyl, C1-C6 haloalkyl, deuterated C1-C6 alkyl,

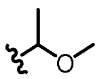 , 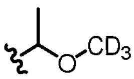 , 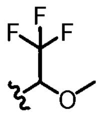 ,

and

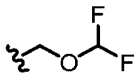 ;

the heteroatoms in the 3- to 6-membered heterocyclyl and six-membered heteroaryl are independently selected from 1, 2, or 3 of N, O, and S.

In a preferred technical solution, the compound of formula (I) is selected from the following structures:

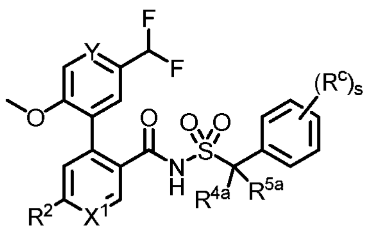

(IV-1)

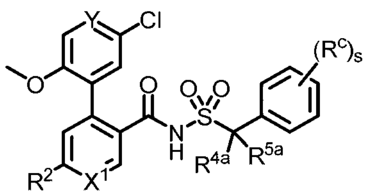

(IV-1a)

wherein

$X^1$ and Y are each independently N or CH; preferably, $X^1$ is CH and Y is N;
$R^{4a}$ and $R^{5a}$ are each independently selected from hydrogen, deuterium, C1-C6 alkyl, and deuterated C1-C6 alkyl;
s is 0, 1, 2, 3, or 4;
each $R^c$ is independently selected from deuterium, halogen, hydroxyl, cyano, mercapto, C2-C6 alkenyl, C2-C6 alkynyl, C1-C6 alkyl unsubstituted or substituted with one or more substituents selected from Group C, C1-C6 alkoxy

unsubstituted or substituted with one or more substituents selected from Group C, $-(CH_2)_iNR^7R^8$, $-(CH_2)_iCONR^7R^8$, $-(CH_2)_iNR^7COR^8$, $-(CH_2)_iSO_2R^7$, $-(CH_2)_iSO_2NR^7R^8$, and $-(CH_2)_iOR^7$;
i is 0, 1, 2, or 3;
$R^7$ and $R^8$ are each independently H, deuterium, C1-C6 alkyl, C1-C6 haloalkyl, or C3-C6 cycloalkyl;
the substituents of Group C include: deuterium, halogen, hydroxyl, cyano, amino, C1-C6 alkoxy, C3-C6 cycloalkyl, and deuterated C1-C6 alkoxy;
$R^2$ is as defined above; preferably, $R^2$ is $CH_3$ or $CD_3$.

In a preferred technical solution, the compound of formula (I) is selected from the following structures:

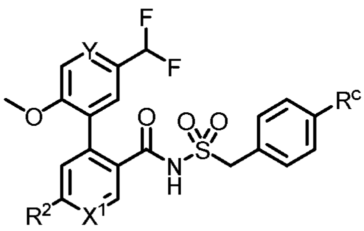
(IV-2)

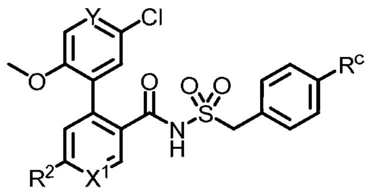
(IV-2a)

wherein

$X^1$ and Y are each independently N or CH; preferably, $X^1$ is CH and Y is N;
$R^c$ is selected from halogen, hydroxyl, cyano, mercapto, C1-C6 alkyl unsubstituted or substituted with one or more substituents selected from Group C, C1-C6 alkoxy unsubstituted or substituted with one or more substituents selected from Group C, and $-(CH_2)_iOR^7$;
i is 0, 1, 2, or 3;
$R^7$ and $R^8$ are each independently H, deuterium, C1-C6 alkyl, C1-C6 haloalkyl, or C3-C6 cycloalkyl;
the substituents of Group C include: deuterium, halogen, hydroxyl, cyano, amino, C1-C6 alkoxy, C3-C6 cycloalkyl, and deuterated C1-C6 alkoxy;
$R^2$ is as defined above; preferably, $R^2$ is $CH_3$ or $CD_3$.

In a preferred technical solution, the compound of formula (I) is selected from the following compounds:

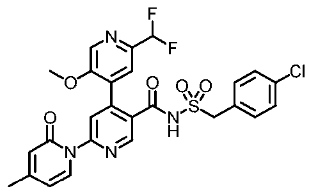 , 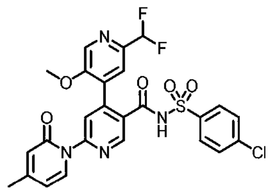 , 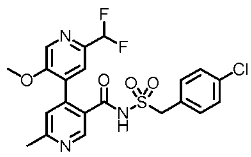 , 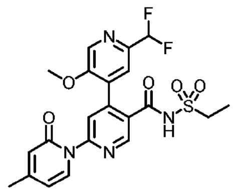 ,

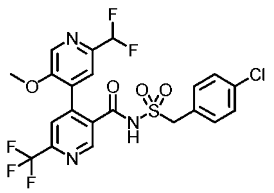 , 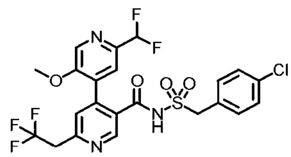 , 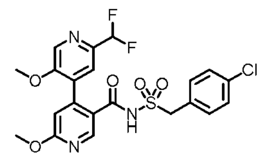 , 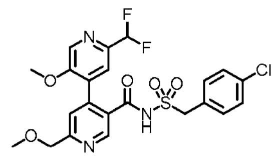 ,

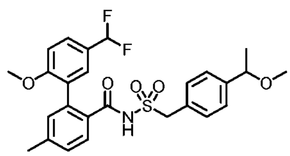 , 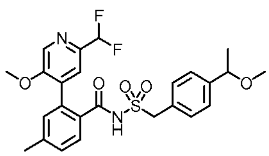 , 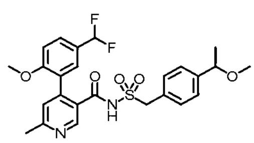 , 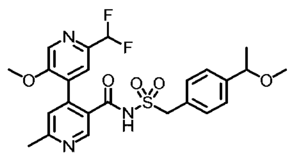 ,

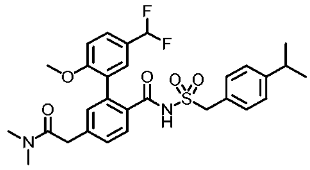 , 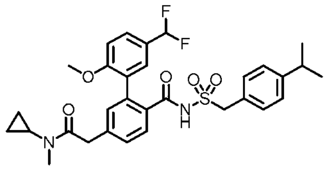 , 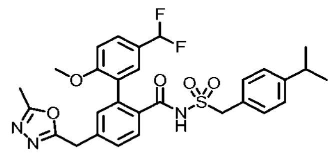 ,

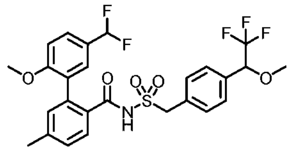 , 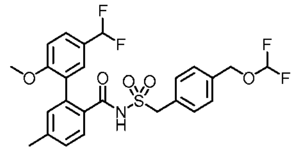 , 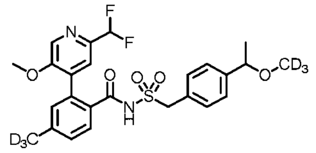 ,

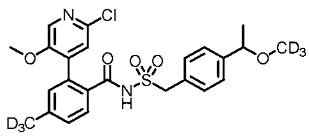 , 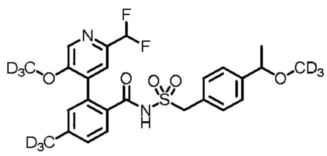 , 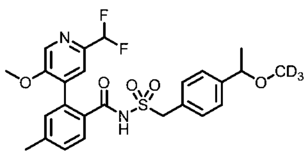 ,

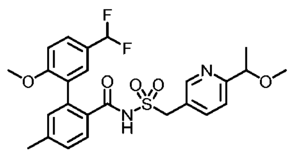 , 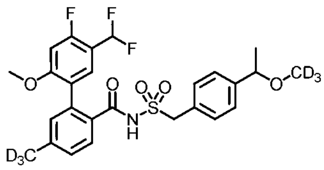 , 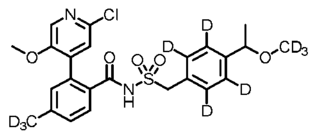 ,

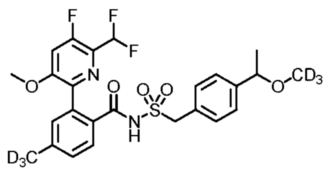 , and 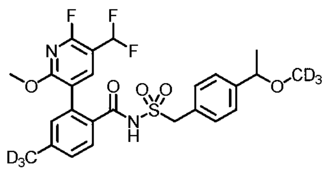 .

In a preferred technical solution, the compound of formula (I) is further selected from the following compounds:

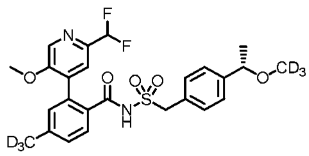 , 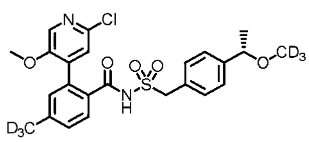 , 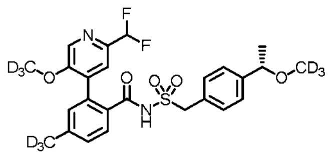 ,

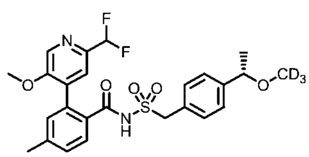 , 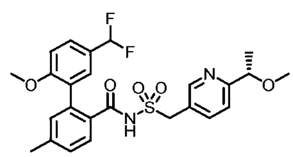 , 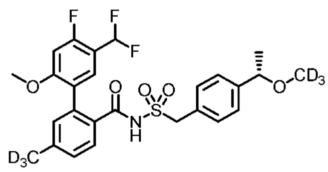 ,

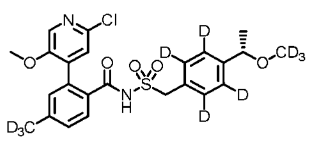 , 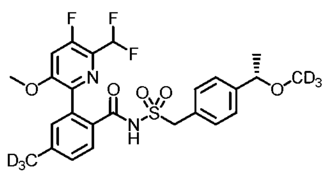 , and 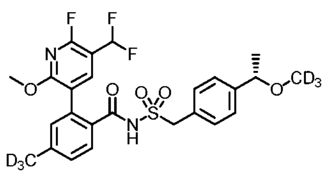 .

The present disclosure further provides a method of preparing the compound of formula (I), or an isotopically

labeled compound, enantiomer, diastereoisomer, or solvate thereof, or a pharmaceutically acceptable salt thereof. The method comprises several methods as follows:

Method one, comprising the following steps:

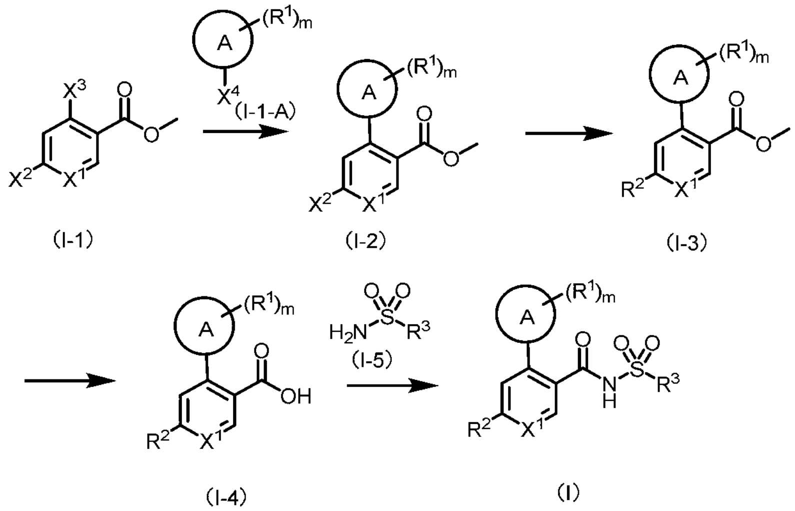


(I-1) (I-2) (I-3) (I-4) (I)

(1) subjecting a compound of formula (I-1) to a coupling or nucleophilic substitution reaction with a compound of formula (I-1-A) in the presence of a catalyst and/or a base to obtain a compound of formula (I-2);
(2) subjecting the compound of formula (I-2) to a coupling or nucleophilic substitution reaction with a compound $R^2$-$X^5$ in the presence of a catalyst and/or a base to obtain a compound of formula (I-3);
(3) subjecting the compound of formula (I-3) to hydrolysis in the presence of a base to obtain a compound of formula (I-4);
(4) reacting the compound of formula (I-4) with a compound of formula (I-5) in a solvent in the presence of a condensing agent or a base to obtain the compound of formula (I);

wherein ring A, $R^1$, $R^2$, $R^3$, m, and $X^1$ are as previously defined; $X^2$ and $X^3$ are each independently halogen or trifluoromethanesulfonate; $X^4$ is a boronic acid group, a boronate ester group, a zinc bromide group, a tributyltin group, or the like; $X^5$ is hydrogen, a boronic acid group, a boronate ester group, a potassium trifluoroborate, or a zinc bromide group, halogen, etc.

Preferably,

the catalyst in step (1) is a palladium catalyst, such as [1,1'-bis(diphenylphosphino)ferrocene]palladium dichloride; the base may be an inorganic base (such as potassium acetate, etc.) or an organic base (such as N,N-diisopropylethylamine, etc.);
the catalyst in step (2) is a palladium catalyst, such as [1,1'-bis(diphenylphosphino)ferrocene]palladium dichloride; the base may be an inorganic base (such as potassium acetate, etc.) or an organic base (such as N,N-diisopropylethylamine, etc.);
the base in step (3) is selected from sodium hydroxide, lithium hydroxide, and potassium hydroxide, etc.
the condensing agent in step (4) is selected from (3H-1,2,3-triazolo[4,5-b]pyridin-3-yloxy)tris-1-pyrrolidinylphosphonium hexafluorophosphate and 2-(7-azabenzotriazol-1-yl)-N,N,N',N'-tetramethyluronium hexafluorophosphate, etc.; the base is selected from N,N-diisopropylethylamine, etc.; and the solvent is selected from N,N-dimethylformamide, etc.

Method two, comprising the following steps:

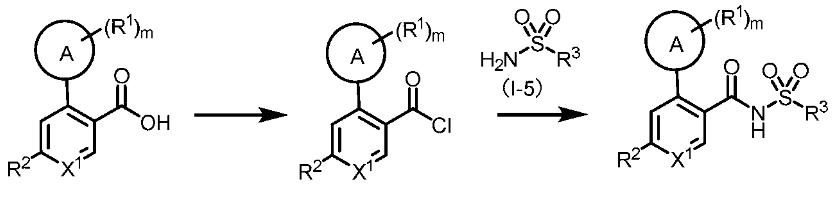


(I-4) (I-6) (I)

(1) subjecting a compound of formula (I-4) to a carboxylic acid acyl chlorination reaction with an acyl chlorinating reagent to obtain a compound of formula (I-6);
(2) reacting the compound of formula (I-6) with a compound of formula (I-5) in a solvent in the presence of a base to obtain the compound of formula (I);

wherein ring A, $R^1$, $R^2$, $R^3$, m, and $X^1$ are as defined above; $X^2$ and $X^3$ are each independently halogen or trifluoromethanesulfonate.

Preferably,

the acyl chlorinating reagent in step (1) is selected from thionyl chloride, phosphorus oxychloride, and oxalyl chloride, etc.;
the base in step (2) is selected from N,N-diisopropylethylamine and pyridine, etc.; the solvent is selected from N,N-dimethylformamide and pyridine, etc.

The present disclosure further provides a pharmaceutical composition comprising the compound of formula (I) as described above, or an isotopically labeled compound, enantiomer, diastereoisomer, or solvate thereof, or a pharmaceutically acceptable salt thereof, as well as a pharmaceutical excipient.

In the pharmaceutical composition, the compound of formula (I), or an isotopically labeled compound, enantiomer, diastereoisomer, or solvate thereof, or a pharmaceutically acceptable salt thereof is present in a therapeutically effective amount.

The present disclosure further provides a use of the compound of formula (I) as described above, or an isotopically labeled compound, enantiomer, diastereoisomer, or solvate thereof, or a pharmaceutically acceptable salt thereof in the manufacture of a DNA polymerase $\theta$ inhibitor or a medicament; said medicament is one for treating and/or preventing diseases by inhibiting DNA polymerase $\theta$.

The present disclosure further provides a use of the compound of formula (I) as described above, or an isotopically labeled compound, enantiomer, diastereoisomer, or solvate thereof, or a pharmaceutically acceptable salt thereof in the manufacture of a medicament for treating and/or preventing cancer.

The present disclosure further provides a method of treating and/or preventing cancer, comprising administering to a patient a therapeutically effective amount of the compound of formula (I) as described above, or an isotopically labeled compound, enantiomer, diastereoisomer, or solvate thereof, or a pharmaceutically acceptable salt thereof.

The term "pharmaceutically acceptable" refers to being relatively non-toxic, safe, and suitable for use in patients.

The term "pharmaceutically acceptable salt" refers to a salt obtained by reacting the compound with a pharmaceutically acceptable acid or base. When the compound contains a relatively acidic functional group, a base addition salt can be obtained by contacting the compound with a sufficient amount of a pharmaceutically acceptable base in a suitable inert solvent. When the compound contains a relatively basic functional group, an acid addition salt can be obtained by contacting the compound with a sufficient amount of a pharmaceutically acceptable acid in a suitable inert solvent. For details, please refer to Handbook of Pharmaceutical Salts: Properties, Selection, and Use (P. Heinrich Stahl, Camille G. Wermuth, 2011, 2nd Revised Edition).

The term "solvate" refers to a substance formed by the combination of a compound with a solvent (including, but not limited to: water, methanol, ethanol, etc.). Solvates are classified into stoichiometric solvates and non-stoichiometric solvates.

The term "solvate of a pharmaceutically acceptable salt" refers to a substance formed by the combination of a compound with a pharmaceutically acceptable acid or base, and a solvent (including but not limited to: water, methanol, ethanol, etc.). The amount of the solvent may be stoichiometric or non-stoichiometric.

The "-" at the end of a group indicates that the group is connected to the rest of the molecule through this site. For example, $CH_3$-C(=O)- refers to an acetyl group.

When a wavy line "∿∿" is present on the valence bond of a group, for example in

"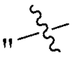",

the wavy line indicates the point of attachment of the group to the rest of the molecule.

The term "halogen" refers to fluorine, chlorine, bromine, or iodine.

The term "oxo" refers to =O, where an oxygen atom replaces two hydrogen atoms on the same carbon atom, that is, replacing a methylene group with a carbonyl group.

The term "alkyl" refers to a straight or branched, saturated monovalent hydrocarbon group having the specified number of carbon atoms (e.g., $C_1$-$C_6$). Alkyl groups include, but are not limited to: methyl, ethyl, n-propyl, isopropyl, n-butyl, isobutyl, sec-butyl, tert-butyl, n-pentyl, n-hexyl, etc.

The term "alkoxy" refers to the group $R^X$-O-, wherein $R^X$ is defined as in the term "alkyl". Alkoxy groups include, but are not limited to: methoxy, ethoxy, n-propoxy, isopropoxy, etc.

The term "alkenyl" refers to a straight or branched, unsaturated monovalent hydrocarbon group having the specified number of carbon atoms (e.g., $C_2$-$C_6$) and containing one or more (e.g., one, two, or three) carbon-carbon $sp^2$ double bonds. Alkenyl groups include, but are not limited to: vinyl,

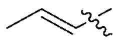, 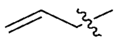,

etc.

The term "alkynyl" refers to a straight-chain or branched, unsaturated monovalent hydrocarbon group having a specified number of carbon atoms (e.g., $C_2$-$C_6$) and having one or more (e.g., one, two, or three) carbon-carbon triple bonds. Alkenyl groups include, but are not limited to, ethynyl,

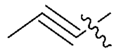, 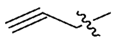,

etc.

The term "cycloalkyl" refers to a cyclic, saturated monovalent hydrocarbon group having the specified number of carbon atoms (e.g., $C_3$-$C_6$), which is monocyclic. Cycloalkyl groups include, but are not limited to:

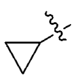, 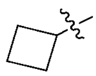, 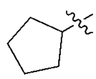, 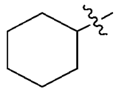,

etc.

The term "cycloalkoxy" refers to the group $R^Y$-O-, wherein $R^Y$ is as defined in the term "cycloalkyl". Cycloalkoxy groups include, but are not limited to, cyclopropoxy, etc.

The term "aryl" refers to a cyclic, unsaturated monovalent hydrocarbon group having the specified number of carbon atoms (e.g., $C_6$-$C_{10}$), which is monocyclic or polycyclic (e.g., 2 or 3 rings). When polycyclic, the monocycles share two atoms and one bond, and each ring is aromatic. Aryl groups include but are not limited to: phenyl, naphthyl, etc.

In the present disclosure, the term "heterocyclyl" refers to a non-aromatic cyclic group comprising at least one carbon atom and at least one (e.g., 1 to 3) ring heteroatom selected from N, O, and S, wherein the sulfur atom may optionally be oxidized or aminated. Examples of "heterocyclyl" may specifically be groups formed by replacing one or more ring carbons of a cycloalkyl group as defined in the present disclosure with moieties selected from -O-, -N=, - NR-, -S-, -S(=O)-, -S(=O)$_2$-, and

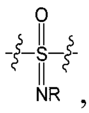,

wherein R is hydrogen, $C_{1-4}$ alkyl, $C_{3-6}$ cycloalkyl, or a nitrogen protecting group (e.g., benzyloxycarbonyl, p-methoxybenzyl carbonyl, tert-butoxycarbonyl, acetyl, benzoyl, benzyl, p-methoxybenzyl, p-methoxyphenyl, 3,4-dimethoxybenzyl, etc.). "Heterocyclyl" includes monocyclic and fused ring, bridged ring, spiro ring, and other bicyclic structures, and may be partially or fully saturated, such as 4- to 10-membered saturated or unsaturated heterocyclyl, 4- to 6-membered saturated or unsaturated heterocyclyl, 3- to 8-membered heterocyclyl, 3- to 6-membered heterocyclyl, etc.; for example, tetrahydrofuranyl, pyrrolidinyl, oxetanyl, oxanyl, azetidinyl, oxiranyl, aziridinyl, thietanyl, 1,2-dithietanyl, 1,3-dithietanyl,

azepanyl, oxepanyl, etc. For example, the heterocyclyl according to the present disclosure may preferably be selected from the following groups:

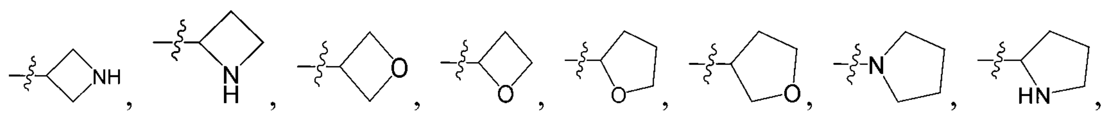
, , , , , , , ,

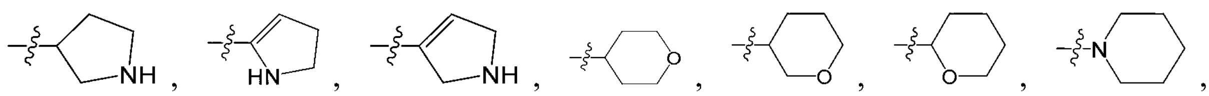
, , , , , , ,

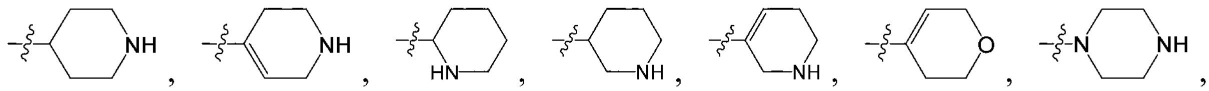
, , , , , , ,

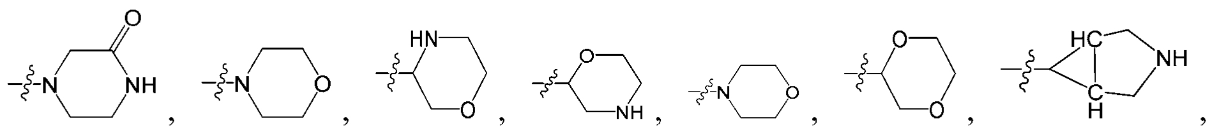
, , , , , , ,

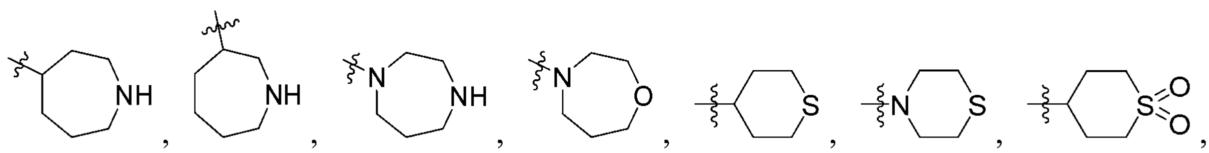
, , , , , , ,

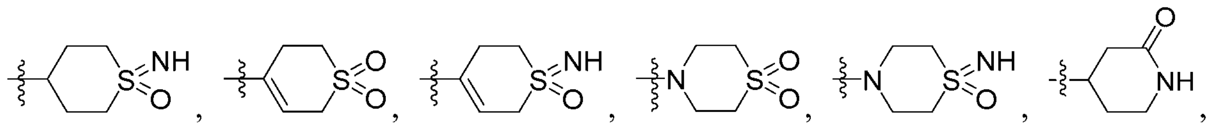
, , , , , , ,

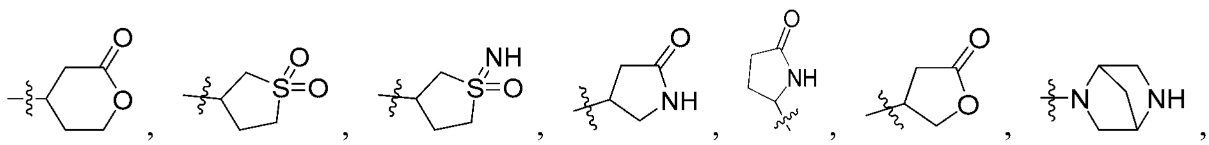
, , , , , , ,

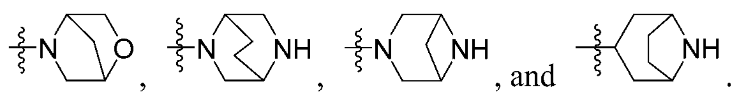
, , , and .

In the present disclosure, the term "heteroaryl" refers to a monocyclic or bicyclic or fused polycyclic aromatic hydrocarbon group having a specified number of ring atoms (e.g., 5-to 10-membered), which contains at least one (e.g., 1 to 3) ring heteroatom independently selected from N, O, and S (e.g., N) in the ring, with the remaining ring atoms being carbon atoms; such as imidazolyl, pyridyl, pyrrolyl, thiazolyl, furyl, oxazolyl, isoxazolyl, pyrazolyl, thienyl, pyrimidinyl, 1,2,4-triazolyl, benzoxazolyl,imidazopyridyl,triazolopyridyl, benzofuryl, pyrazolopyrimidinyl, benzodioxolyl, indolyl, quinolinyl, isoquinolinyl, etc.

The term "isotopically labeled compound" refers to a compound in which one or more atoms are replaced by atoms having an atomic mass or mass number different from the atomic mass or mass number typically found in nature, compared to the nitrogen-containing compound represented by formula (I). Examples of isotopes that can be incorporated into compounds according to the present disclosure include isotopes of H, C, N, O, S, F, and Cl, such as $^{2}H$, $^{3}H$, $^{13}C$, $^{11}C$, $^{14}C$, $^{15}N$, $^{18}O$, $^{17}O$, $^{32}P$, $^{35}S$, $^{18}F$, and $^{36}Cl$, respectively. A compound according to the present disclosure containing the above-mentioned isotopes and/or other isotopes of other atoms, a pharmaceutically acceptable salt, a solvate, a solvate of the pharmaceutically acceptable salt, or a prodrug thereof are within the scope of the present disclosure. Certain isotopically labeled compounds according to present disclosure, for example, those incorporating radioactive isotopes such as $^{3}H$ and $^{14}C$, may be useful in drug and/or substrate tissue distribution assays. Tritium (i.e., $^{3}H$) and carbon-14 (i.e.,

$^{14}C$) isotopes are particularly preferred due to their ease of preparation and detectability. Moreover, substitution with heavier isotopes, such as deuterium (i.e., $^{2}H$ or D), may offer certain therapeutic advantages derived from greater metabolic stability (e.g., increased in vivo half-life or reduced dosage requirements) and thus may be preferred in some circumstances. The compounds according to the present disclosure as claimed in the claims may specifically be defined as being substituted with deuterium or tritium. In addition, the absence of explicit mention of deuterium or tritium in the substituents does not exclude deuterium or tritium; rather, deuterium or tritium may also be included.

The term "therapeutically effective amount" refers to an amount administered to a patient that is sufficient to effectively treat a disease. The therapeutically effective amount will vary depending on the type of compound, the type of disease, the severity of the disease, the age of the patient, etc., but can be adjusted by those skilled in the art as appropriate.

The term "pharmaceutical excipient" refers to all substances included in a pharmaceutical preparation other than the active pharmaceutical ingredient, and is generally divided into two major categories: excipients and additives. Specific reference may be made to the Pharmacopoeia of the People's Republic of China (2020 Edition) and the Handbook of Pharmaceutical Excipients (Paul J Sheskey, Bruno C Hancock, Gary P Moss, David J Goldfarb, 2020, 9th Edition).

The term "treatment" refers to eliminating the cause of a disease or alleviating symptoms.

The term "prevention" refers to reducing the risk of occurrence of a disease.

The term "patient" refers to any animal in need of treatment for or prevention of a disease, usually a mammal, such as a human. Mammals include, but are not limited to: cattle, horses, sheep, pigs, cats, dogs, mice, rats, rabbits, guinea pigs, monkeys, humans, etc.

The term "PG" represents a protecting group, such as a Boc protecting group or the like.

On the basis of complying with common knowledge in the art, the above-mentioned preferred conditions can be combined arbitrarily to obtain the various preferred embodiments of the present disclosure.

The reagents and raw materials used in the present disclosure are all commercially available.

The positive progressive effect of the present disclosure lies in that: the compounds according to the present disclosure have superior inhibitory activity against DNA polymerase 0.

DETAILED DESCRIPTION OF THE PREFERRED EMBODIMENT

The following further illustrates the present disclosure by way of examples, without thereby limiting the present disclosure to the scope of the described examples. In the following examples, experimental methods without specified conditions are selected according to conventional methods and conditions or based on product instructions.

In various examples, $^{1}$HNMR was recorded using a BRUKER AVANCE NEO 400 MHz, JNM-ECZ400s nuclear magnetic resonance spectrometer, with chemical shifts expressed in $\delta$ (ppm); Liquid chromatography-mass spectrometry (LCMS) was recorded using Shimadzu LC-20AD, Agilent 1260, and Agilent 1200 mass spectrometers; Preparative HPLC separations were performed using WATERS Autop and Shimadzu LC20AR liquid chromatographs.

In the following experimental descriptions, the following abbreviations may be used:

| | |
|---|---|
| DCM | Dichloromethane |
| DAST | Diethylaminosulfur trifluoride |
| Dioxane | 1,4-dioxane |
| DMF | N,N-Dimethylformamide |
| PyAOP | (3H-1,2,3-Triazolo[4,5-b]pyridin-3-yloxy)tri-1-pyrrolidinophosphonium hexafluorophosphate |
| DIPEA | N,N-Diisopropylethylamine |
| $Pd(dppf)Cl_2$ | 1,1'-Bis(diphenylphosphino)ferrocene palladium chloride |
| EDCI | 1-Ethyl-(3-dimethylaminopropyl)carbonyldiimide hydrochloride |
| DMAP | 4-Dimethylaminopyridine |

**Preparation of intermediate compound INT-1**

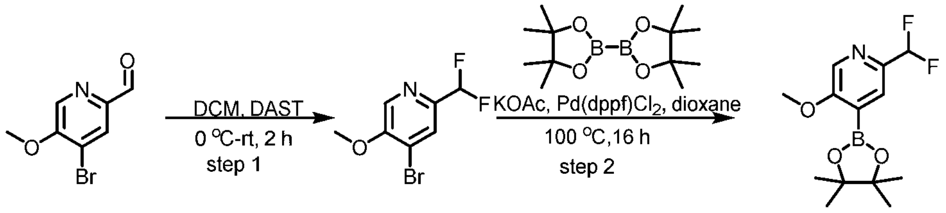


1 2 INT-1

**Step 1: 4-Bromo-2-(difluoromethyl)-5-methoxypyridine**

To a solution of 4-bromo-5-methoxypicolinaldehyde (1.0 g, 4.63 mmol) in dichloromethane (10 mL) at 0°C was added dropwise diethylaminosulfur trifluoride (1.49 g, 9.26 mmol). The mixture was reacted at room temperature for 2 hours. After completion of the reaction, water (10 mL) was added, followed by extraction with dichloromethane (10 mL × 3). The combined organic phase was dried over anhydrous sodium sulfate, filtered, and concentrated to obtain a residue. The residue was purified by silica gel column chromatography (ethyl acetate:petroleum ether = 10% to 20%) to obtain 4-bromo-2-(difluoromethyl)-5-methoxypyridine (800 mg, yield 85.2%). LCMS calc. for $C_7H_7BrF_2NO$ $[M+H]^+$: m/z = 238.0; Found: 237.9, 239.9.

**Step 2: 2-(Difluoromethyl)-5-methoxy-4-(4,4,5,5-tetramethyl-1,3,2-dioxaborolan-2-yl)pyridine (INT-1)**

To a solution of 4-bromo-2-(difluoromethyl)-5-methoxypyridine (800 mg, 3.36 mmol) in 1,4-dioxane (10 mL) at room temperature were added 4,4,4',4',5,5,5',5'-octamethyl-2,2'-bi(1,3,2-dioxaborolane) (1.7 g, 6.72 mmol), [1,1'-bis(diphenylphosphino)ferrocene]dichloropalladium (244 mg, 0.336 mmol), and potassium acetate (989 mg, 10.1 mmol). Under a nitrogen atmosphere, the reaction mixture was purged three times, then heated to 100°C and reacted for 16 hours, followed by cooling to room temperature. The reaction mixture was filtered, and the filtrate was concentrated to obtain a residue. The residue was purified by silica gel column chromatography (ethyl acetate:petroleum ether = 20% to 50%) to obtain 2-(difluoromethyl)-5-methoxy-4-(4,4,5,5-tetramethyl-1,3,2-dioxaborolan-2-yl)pyridine (INT-1) (700 mg, yield 65.8%). LCMS calc. for $C_{13}H_{19}BF_2NO_3$ $[M+H]^+$: m/z =286.1; Found: 204.1. (The molecular ion peak of (2-(difluoromethyl)-5-methoxypyridin-4-yl)boronic acid was detected.)

**Preparation of intermediate compound INT-2**

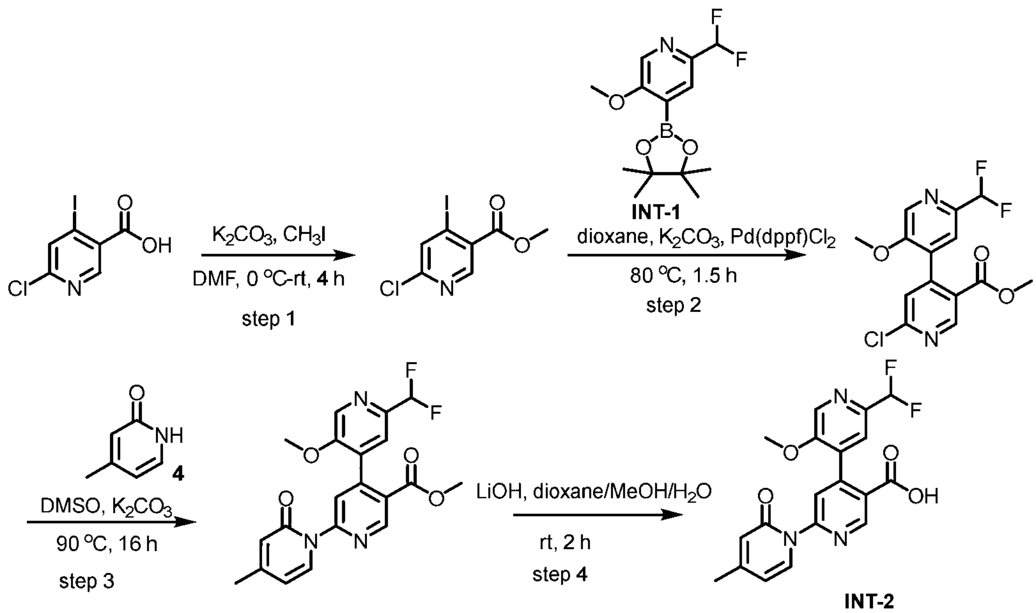


INT-2

**Step 1:** Methyl **6-chloro-4-iodonicotinate**

To a solution of 6-chloro-4-iodonicotinic acid (2.0 g, 7.1 mmol) and potassium carbonate (2.94 g, 21.3 mmol) in N,N-dimethylformamide (10 mL) at 0°C was added methyl iodide (1.6 g, 11.3 mmol). The mixture was reacted at room temperature for 4 hours. After completion of the reaction, water (30 mL) was added, followed by extraction with ethyl acetate (50 mL × 3). The combined organic phase was dried over anhydrous sodium sulfate, filtered, and concentrated to obtain a residue. The residue was purified by silica gel column chromatography (ethyl acetate:petroleum ether = 5% to 10%) to obtain methyl 6-chloro-4-iodonicotinate (2.05 g, yield 97.8%). LCMS calc. for $C_7H_5ClINO_2$ $[M+H]^+$: m/z = 297.9; Found: 297.7.

**Step 2: Methyl 6-chloro-2'-(difluoromethyl)-5'-methoxy-[4,4'-bipyridine]-3-carboxylate**

To a solution of methyl 6-chloro-4-iodonicotinate (711 mg, 2.49 mmol) and 2-(difluoromethyl)-5-methoxy-4-(4,4,5,5-tetramethyl-1,3,2-dioxaborolan-2-yl)pyridine (700 mg, 2.35 mmol) in 1,4-dioxane (10 mL) at room temperature were added [1,1'-bis(diphenylphosphino)ferrocene]palladium(II) dichloride (171 mg, 0.235 mmol) and potassium carbonate (813 mg, 5.88 mmol). Under a nitrogen atmosphere, the reaction mixture was purged three times, then heated to 80°C and reacted for 1.5 hours, followed by cooling to room temperature. The reaction mixture was filtered, and the filtrate was concentrated to obtain a residue. The residue was purified by silica gel column chromatography (ethyl acetate:petroleum ether = 20% to 50%) to obtain methyl 6-chloro-2'-(difluoromethyl)-5'-methoxy-[4,4'-bipyridine]-3-carboxylate (600 mg, yield 69.8%). LCMS calc. for $C_{14}H_{12}ClF_2N_2O_3$ $[M+H]^+$: m/z =329.0; Found: 329.0.

**Step 3: Methyl 2"-(difluoromethyl)-5"-methoxy-4-methyl-2-oxo-2H-[1,2':4',4"-terpyridine]-5'-carboxylate**

To a solution of methyl 6-chloro-2'-(difluoromethyl)-5'-methoxy-[4,4'-bipyridine]-3-carboxylate (300 mg, 0.913 mmol) and 4-methylpyridin-2(1H)-one (99.6 mg, 0.913 mmol) in dimethyl sulfoxide (4 mL) at room temperature was added potassium carbonate (253 mg, 1.73 mmol). Under a nitrogen atmosphere, the reaction mixture was purged three times, then heated to 90°C and reacted for 16 hours, followed by cooling to room temperature. The mixture was added with a saturated aqueous ammonium chloride solution (10 mL) and extracted with ethyl acetate (20 mL x 3). The combined organic phase was dried over anhydrous sodium sulfate, filtered, and concentrated to obtain a residue. The residue was purified by silica gel column chromatography (methanol:dichloromethane = 0% to 10%) to obtain methyl 2"-(difluoromethyl)-5"-methoxy-4-methyl-2-oxo-2H-[1,2':4',4"-terpyridine]-5'-carboxylate (200 mg, yield 49.1%). LCMS calc. for $C_{20}H_{18}F_2N_3O_4$ $[M+H]^+$: m/z = 402.1; Found: 402.0.

**Step 4: 2"-(Difluoromethyl)-5"-methoxy-4-methyl-2-oxo-2H-[1,2':4',4"-terpyridine]-5'-carboxylic acid (INT-2)**

Methyl 2"-(difluoromethyl)-5"-methoxy-4-methyl-2-oxo-2H-[1,2':4',4"-terpyridine]-5'-carboxylate (150 mg, 0.374 mmol) was dissolved in 1,4-dioxane (3 mL), methanol (1 mL), and water (1 mL), followed by addition of lithium hydroxide (57.2 mg, 2.39 mmol). The reaction mixture was reacted at room temperature for 3 hours. After the reaction was completed, the pH of the mixture was adjusted to 4-5 with 1 N dilute hydrochloric acid, resulting in precipitation of a solid. The solid was filtered off and dried under vacuum to obtain 2"-(difluoromethyl)-5"-methoxy-4-methyl-2-oxo-2H-[1,2':4',4"-terpyridine]-5'-carboxylic acid (INT-2) (90 mg, yield 62.3%). LCMS calc. for $C_{19}H_{16}F_2N_3O_4$ $[M+H]^+$: m/z = 388.1; Found: 388.0.

**Preparation of intermediate compound INT-3**

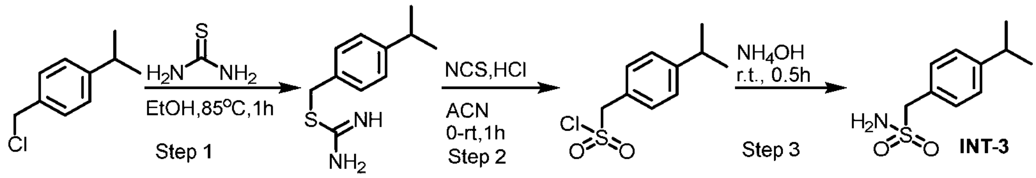


INT-3

**Step 1: 4-Isopropylbenzyl carbamimidothioate**

1-(Chloromethyl)-4-isopropylbenzene (2.0 g, 11.9 mmol) was dissolved in ethanol (20 mL). Thiourea (0.99 g, 13.0 mmol) was added to the reaction mixture, and the reaction mixture was heated to 80°C and reacted for 1 hour. After the

reaction was completed, the reaction mixture was concentrated to obtain 4-isopropylbenzyl carbamimidothioate (2.47 g, crude yield: >99%). LCMS calc. for $C_{11}H_{17}N_2S$ $[M+H]^+$: m/z = 209.1; Found: 209.2.

**Step 2: (4-Isopropylphenyl)methanesulfonyl chloride**

N-Chlorosuccinimide (6.33 g, 47.4 mmol) was dissolved in acetonitrile (20 mL) and 1 N dilute hydrochloric acid (5 mL). The reaction mixture was reacted at 0°C for 15 minutes, and then 4-isopropylbenzyl carbamimidothioate (2.47 g, 11.8 mmol) was added to the reaction mixture. The reaction mixture was reacted at room temperature for 1 hour. After the reaction was completed, the mixture was extracted with ethyl acetate (50 mL x 3). The combined organic phases were dried over anhydrous sodium sulfate, filtered, and concentrated to obtain (4-isopropylphenyl)methanesulfonyl chloride (2.5 g, crude yield: 90.6%).

**Step 3: (4-Isopropylphenyl)methanesulfonamide**

(4-Isopropylphenyl)methanesulfonyl chloride (2.5 g, 10.7 mmol) was dissolved in tetrahydrofuran (10 mL) and aqueous ammonia (10 mL), and the reaction mixture was reacted at room temperature for 1 hour. After the reaction was completed, water (20 mL) was added to quench the reaction. The mixture was extracted with ethyl acetate (100 mL $\times$ 3). The combined organic phases were dried over anhydrous sodium sulfate, filtered, and concentrated to obtain a residue. The residue was purified by silica gel column chromatography (methanol:dichloromethane = 0% to 5%) to obtain (4-isopropylphenyl)methanesulfonamide (2.0 g, yield: 87.3%). LCMS calc. for $C_{10}H_{14}NO_2S$ $[M-H]^-$: m/z =212.1; Found: 212.1.

**Example 1: Preparation of compound cpd-1**

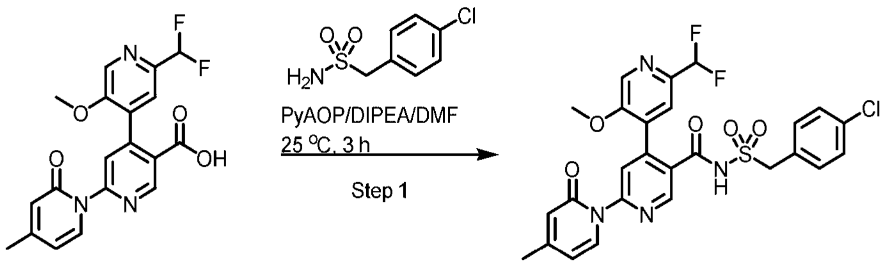


INT-2 cpd-1

**Step 1: *N*-((4-Chlorobenzyl)sulfonyl)-2"-(difluoromethyl)-5"-methoxy-4-methyl-2-oxo-2H-[1,2':4',4"-terpyridine]-5' -carboxamide**

To a solution of 2"-(difluoromethyl)-5"-methoxy-4-methyl-2-oxo-2H-[1,2':4',4"-terpyridine]-5'-carboxylic acid (INT-2) (60 mg, 0.15 mmol), (7-azabenzotriazol-1-yloxy)tripyrrolidinophosphonium hexafluorophosphate (105 mg, 0.20 mmol), and (4-chlorophenyl)methanesulfonamide (38 mg, 0.19 mmol) in N,N-dimethylformamide (2 mL) at room temperature was added dropwise N,N-diisopropylethylamine (40 mg, 0.31 mmol). The mixture was reacted at 25 °C for 3 hours, after which the reaction mixture was concentrated, and the residue was purified by preparative high-performance liquid chromatography (C18 column, Mobile Phase A: water (0.01% HCOOH), Mobile Phase B: acetonitrile, Gradient: 5% - 90% (%B)) to obtain *N*-((4-chlorobenzyl)sulfonyl)-2"-(difluoromethyl)-5"-methoxy-4-methyl-2-oxo-2H-[1,2':4',4"-terpyridine]-5'-carboxamide (30 mg, yield: 33.6%). LCMS calc. for $C_{26}H_{22}ClF_2N_4O_5S$ $[M+H]^+$: m/z =575.1; Found: 575.1. $^1H$ NMR (400 MHz, DMSO-d6) $\delta$ 12.31 (br s, 1H), 8.70 (s, 1H), 8.53 (s, 1H), 7.91-7.85 (m, 2H), 7.61 (s, 1H), 7.44-7.42 (m, 2H), 7.33-7.31 (m, 2H), 6.97 (t, J = 55.2 Hz, 1H), 6.35 (s, 1H), 6.30-6.28 (m, 1H), 4.72-4.43 (m, 2H), 3.90 (s, 3H), 2.21 (s, 3H).

**Example 2: Preparation of compound cpd-2**

The same synthetic route as that of compound **cpd-2** was adopted, except that the starting material (4-chlorophenyl)methanesulfonamide was replaced with ethanesulfonamide.

**2”-(Difluoromethyl)-N-(ethylsulfonyl)-5”-methoxy-4-methyl-2-oxo-2H-[1,2’:4’,4"-terpyridine]-5’-carboxamide**

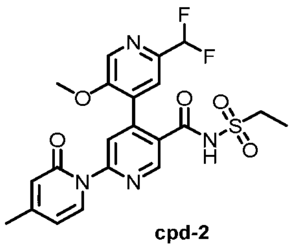

**cpd-2**

LCMS calc. for $C_{21}H_{21}F_2N_4O_5S$ $[M+H]^+$: m/z =479.1; Found: 478.8 $^1H$ NMR (400 MHz, DMSO-$d_6$) $\delta$ 12.81-12.12 (m, 1H), 8.86 (s, 1H), 8.48 (s, 1H), 7.90 (d, *J* = 6.8 Hz, 1H), 7.76 (s, 1H), 7.54 (s, 1H), 6.95 (t, *J* = 55.2 Hz, 1H), 6.33 (s, 1H), 6.29-6.27 (m, 1H), 3.88 (s, 3H), 3.06 - 3.04 (m, 2H), 2.20 (s, 3H), 1.10 (t, *J* = 7.2 Hz, 3H).

**Example 3: Preparation of compound cpd-3**

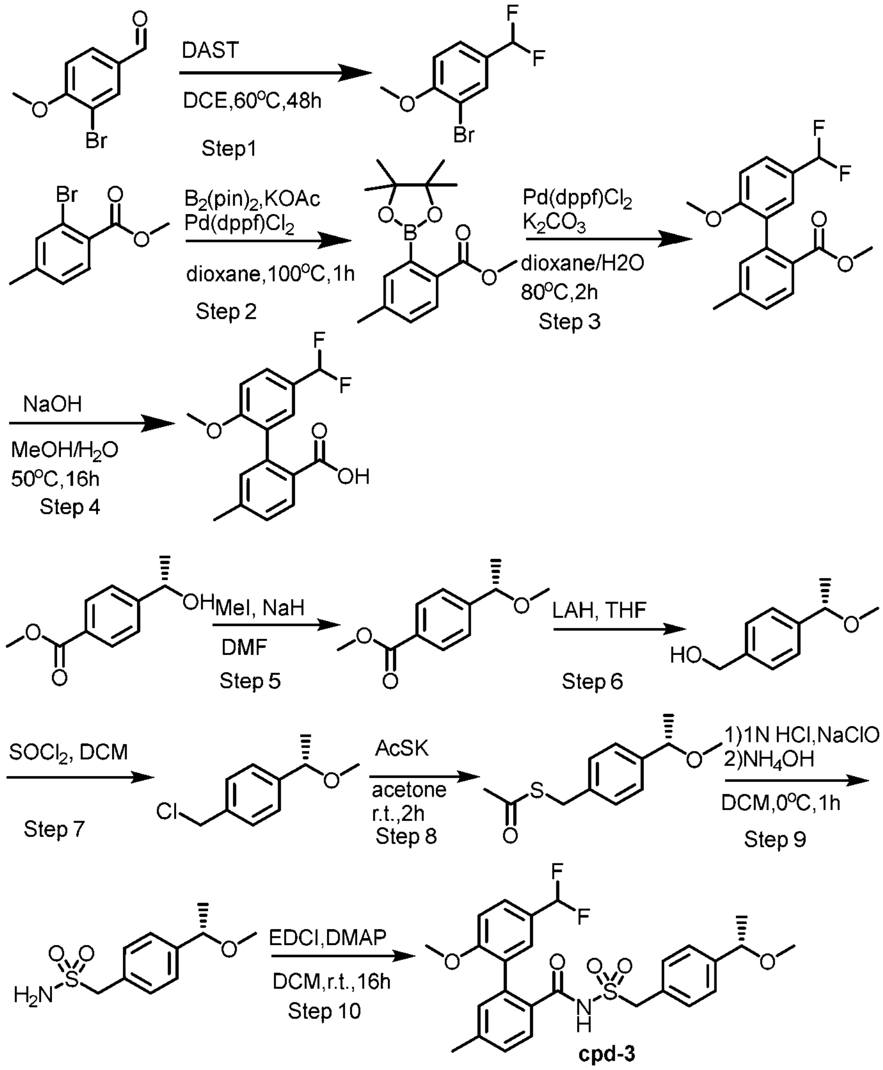


**cpd-3**

**Step 1: 2-Bromo-4-(difluoromethyl)-1-methoxybenzene**

At room temperature, 3-bromo-4-methoxybenzaldehyde (20 g, 93.0 mmol) was dissolved in 1,2-dichloroethane (100 mL). Diethylaminosulfur trifluoride (60 g, 372 mmol) was added to the reaction mixture. The reaction mixture was heated to 60°C and then reacted for 48 hours. After the reaction was completed, the reaction mixture was slowly poured into ice water and extracted with ethyl acetate (200 mL × 2). The organic phase was washed once with saturated sodium bicarbonate solution. The combined organic phases were dried over anhydrous sodium sulfate, filtered, and concentrated to obtain a residue. The residue was purified by silica gel column chromatography (ethyl acetate:petroleum ether = 0% to 10%) to obtain 2-bromo-4-(difluoromethyl)-1-methoxybenzene (15.0 g, yield: 68%).

**Step 2: Methyl 4-methyl-2-(4,4,5,5-tetramethyl-1,3,2-dioxaborolan-2-yl)benzoate**

Methyl 2-bromo-4-methylbenzoate (6.0 g, 26.2 mmol) was dissolved in dioxane (60 mL), and pinacol boronate (9.98 g, 39.3 mmol), potassium acetate (5.14 g, 52.4 mmol), and 1,1'-bis(diphenylphosphino)ferrocene palladium dichloride (1.92 g, 2.62 mmol) were added to the reaction mixture. The reaction mixture was heated to 100°C and reacted under an argon atmosphere for 1 hour. After the reaction was completed, the reaction mixture was concentrated to obtain a residue. The residue was purified by silica gel column chromatography (ethyl acetate:petroleum ether = 0% to 20%) to obtain methyl 4-methyl-2-(4,4,5,5-tetramethyl-1,3,2-dioxaborolan-2-yl)benzoate (5.5 g, yield: 76%). LCMS calc. for $C_{15}H_{22}BO_4$ $[M+H]^+$: m/z = 277.2; Found:277.2

**Step 3: Methyl 5'-(difluoromethyl)-2'-methoxy-5-methyl-[1,1'-biphenyl]-2-carboxylate**

Methyl 4-methyl-2-(4,4,5,5-tetramethyl-1,3,2-dioxaborolan-2-yl)benzoate (5.5 g, 19.9 mmol) was dissolved in dioxane (50 mL) and water (10 mL). 2-bromo-4-(difluoromethyl)-1-methoxybenzene (5.67 g, 23.9 mmol), potassium carbonate (8.26 g, 59.8 mmol), and 1,1'-bis(diphenylphosphino)ferrocene palladium dichloride (1.46 g, 1.99 mmol) were added to the reaction mixture. The reaction mixture was heated to 80°C and reacted for 2 hours under an argon atmosphere. After the reaction was completed, the reaction mixture was quenched with water, then extracted with ethyl acetate (100 mL × 2). The combined organic phases were dried over anhydrous sodium sulfate, filtered, and concentrated to obtain a residue. The residue was purified by silica gel column chromatography (ethyl acetate:petroleum ether = 0% to 30%) to obtain methyl 5'-(difluoromethyl)-2'-methoxy-5-methyl-[1,1'-biphenyl]-2-carboxylate (5.4 g, yield: 88.5%).

**Step 4: 5'-(Difluoromethyl)-2'-methoxy-5-methyl-[1,1'-biphenyl]-2-carboxylic acid**

Methyl 5'-(difluoromethyl)-2'-methoxy-5-methyl-[1,1'-biphenyl]-2-carboxylate (5.4 g, 17.6 mmol) was dissolved in methanol (50 mL) and water (8 mL). Sodium hydroxide (2.12 g, 52.9 mmol) was added to the reaction mixture, which was then heated to 50 °C and reacted for 16 hours. After the reaction was completed, the reaction mixture was adjusted to pH = 5 with 1N dilute hydrochloric acid, and extracted with ethyl acetate (100 mL × 3). The combined organic phases were dried over anhydrous sodium sulfate, filtered, and concentrated to obtain a residue. The residue was purified by silica gel column chromatography (methanol:dichloromethane = 0% to 10%) to obtain 5'-(difluoromethyl)-2'-methoxy-5-methyl-[1,1'-biphenyl]-2-carboxylic acid (5.0 g, yield: 97%). LCMS calc. for $C_{16}H_{13}F_2O_3$ $[M-H]^+$: m/z = 291.1; Found:291.2.

**Step 5: Methyl (S)-4-(1-methoxyethyl)benzoate**

Methyl (*S*)-4-(1-hydroxyethyl)benzoate (1.0 g, 5.55 mmol) was dissolved in N,N-dimethylformamide (10 mL), and then sodium hydride (0.33 g, 8.32 mmol, 60%) was added to the solution at 0°C. The reaction mixture was reacted at 0°C under a nitrogen atmosphere for 0.5 hours. Iodomethane (1.58 g, 11.1 mmol) was added to the reaction mixture, which was reacted at 20°C under a nitrogen atmosphere for another 2 hours. The reaction mixture was quenched with saturated aqueous ammonium chloride solution, then extracted with ethyl acetate (50 mL × 2). The combined organic phases were dried over anhydrous sodium sulfate, filtered, and concentrated to obtain a residue. The residue was purified by silica gel column chromatography (ethyl acetate:petroleum ether = 0% to 20%) to obtain methyl (S)-4-(1-methoxyethyl)benzoate (0.8 g, yield: 74.3%).

**Step 6: (*S*)-(4-(1-methoxyethyl)phenyl)methanol**

Methyl (*S*)-4-(1-methoxyethyl)benzoate (0.8 g, 4.12 mmol) was dissolved in tetrahydrofuran (10 mL), and then a solution of lithium aluminum hydride in tetrahydrofuran (8.3 mL, 8.3 mmol, 1 mol/L) was added to the solution at 0°C. The reaction mixture was reacted at 0°C under a nitrogen atmosphere for 1 hour. After the reaction was completed, decahydrate sodium sulfate was slowly added to the reaction mixture to quench the reaction. The mixture was filtered.

The filter cake was washed 3 times with ethyl acetate, and the combined organic phases were dried over anhydrous sodium sulfate, filtered, and concentrated to obtain a residue. The residue was purified by silica gel column chromatography (ethyl acetate:petroleum ether = 0% to 30%) to obtain (*S*)-(4-(1-methoxyethyl)phenyl)methanol (0.7 g, yield: 93%).

**Step 7: (*S*)-1-(Chloromethyl)-4-(1-methoxyethyl)benzene**

(*S*)-(4-(1-Methoxyethyl)phenyl)methanol (0.7 g, 4.21 mmol) was dissolved in dichloromethane (10 mL), and thionyl chloride (0.92 mL, 12.6 mmol) was added to the reaction mixture. The mixture was reacted at room temperature for 1 hour, and concentrated under reduced pressure to obtain the crude product (S)-1-(chloromethyl)-4-(1-methoxyethyl) benzene (0.8 g, crude yield: >99%).

**Step 8: (*S*)-(4-(1-Methoxyethyl)benzyl)benzylthioacetate**

(*S*)-1-(Chloromethyl)-4-(1-methoxyethyl)benzene (0.78 g, 4.22 mmol) was dissolved in acetone (15 mL), followed by addition of potassium thioacetate (0.58 g, 5.07 mmol). The mixture was reacted at room temperature for 2 hours. After the reaction was completed, the mixture was filtered, and the filtrate was concentrated to obtain a residue. The residue was purified by silica gel column chromatography (ethyl acetate:petroleum ether = 0% to 20%) to obtain (*S*)-(4-(1-methoxyethyl)benzyl)benzylthioacetate (0.8 g, yield: 84.4%).

**Step 9: (*S*)-(4-(1-methoxyethyl)phenyl)methanesulfonamide**

(*S*)-(4-(1-Methoxyethyl)benzyl)benzylthioacetate (0.8 g, 3.57 mmol) was dissolved in dichloromethane (10 mL). 1 N dilute hydrochloric acid (21.4 mL, 21.4 mmol) and then a 10% aqueous sodium hypochlorite solution (15.9 g, 21.4 mmol) were added to the reaction mixture. After reacting for 1 hour, the reaction mixture was added with concentrated aqueous ammonia to adjust the pH to 11, and reacted for an additional 0.5 hours. The reaction mixture was diluted with water (50 mL) and extracted with ethyl acetate (30 mL $\times$ 3). The combined organic phases were dried over anhydrous sodium sulfate, filtered, and concentrated to obtain a residue. The residue was purified by silica gel column chromatography (methanol:dichloromethane = 0% to 10%) to obtain (S)-(4-(1-methoxyethyl)phenyl)methanesulfonamide (0.35 g, yield: 47.5%). LCMS calc. for $C_{10}H_{14}NO_3S$ $[M-H]^+$: m/z =228.1; Found: 228.2.

**Step 10: (*S*)-5'-(Difluoromethyl)-2'-methoxy-N-(4-(1-methoxyethyl)benzyl)sulfonyl)-5-methyl-[1,1'-biphenyl]-2-carboxamide**

5'-(Difluoromethyl)-2'-methoxy-5-methyl-[1,1'-biphenyl]-2-carboxylic acid (100 mg, 0.34 mmol) was dissolved in dichloromethane (6 mL), followed by addition of (S)-(4-(1-methoxyethyl)phenyl)methanesulfonamide (94.1 mg, 0.41 mmol), 1-ethyl-(3-dimethylaminopropyl)carbodiimide hydrochloride (78.7 mg, 0.41 mmol), and 4-dimethylaminopyridine (83.6 mg, 0.68 mmol). The mixture was reacted at room temperature for 16 hours. After the reaction was completed, the reaction mixture was concentrated under reduced pressure. The residue was purified by silica gel column chromatography (dichloromethane:methanol = 0 to 10%) and preparative high-performance liquid chromatography (Column: XBridge Prep C18 5$\mu$m OBD 19*150mm, Mobile phase: A (10 mM $NH_4HCO_3$ in Water) B (ACN), 0-3min: 40%B, 3-7min: 40-85%B, 7-11min: 85-90%B, 11-13min: 90-95%B, 13-16min: 95%B, 16-16.5min: 95-40%B, 16.5-21min: 40%B, Flow =15mL/min) to obtain (S)-5'-(difluoromethyl)-2'-methoxy-N-((4-(1-methoxyethyl)benzyl)sulfonyl)-5-methyl-[1,1'-biphenyl]-2-carboxamide (58 mg, yield: 33.7%). LCMS calc. for $C_{26}H_{26}F_2NO_5S$ $[M-H]^-$: m/z = 502.2; Found: 502.3. $^1H$ NMR (400 MHz, DMSO-d6) $\delta$ 11.79 (s, 1H), 7.62-7.52 (m, 1H), 7.43 (d, J = 2.1 Hz, 1H), 7.34-7.19 (m, 6H), 7.17-6.86 (m, 3H), 4.64 (s, 2H), 4.33 (q, J = 6.4 Hz, 1H), 3.72 (s, 3H), 3.13 (s, 3H), 2.38 (s, 3H), 1.33 (d, J = 6.4 Hz, 3H).

**Example 4: Preparation of compound cpd-4**

The same synthetic route as that of compound **cpd-8** was adopted, except that the starting material (R)-4-(1-methoxyethyl)phenyl)methanesulfonamide in step 3 was replaced with (S)-4-(1-methoxyethyl)phenyl)methanesulfonamide.

**(*S*)-2-(2-(Difluoromethyl)-5-methoxypyridin-4-yl)-N-((4-(1-methoxyethyl)benzyl)sulfonyl)-4-methylbenzamide**

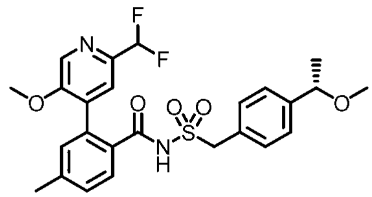

**cpd-4**

LCMS calc. for $C_{25}H_{27}F_2NO_5S$ $[M+H]^+$: m/z = 505.2; Found: 505.4. $^1H$ NMR (400 MHz, DMSO-*d*$_6$) δ 11.91 (s, 1H), 8.44 (s, 1H), 7.56 (s, 1H), 7.39-7.21 (m, 7H), 6.96 (t, *J* = 55.1 Hz, 1H), 4.66 (s, 2H), 4.33 (q, *J* = 6.4 Hz, 1H), 3.86 (s, 3H), 3.13 (s, 3H), 2.39 (s, 3H), 1.32 (d, *J* = 6.4 Hz, 3H).

**Example 5: Preparation of compound cpd-5**

The same synthetic route as that of compound **cpd-9** was adopted, except that the starting material (*R*)-4-(1-methoxyethyl)phenyl)methanesulfonamide in step 3 was replaced with (S)-4-(1-methoxyethyl)phenyl)methanesulfonamide.

**(*S*)-4-(5-(Difluoromethyl)-2-methoxyphenyl)-N-((4-(1-methoxyethyl)benzyl)sulfonyl)-6-methylnicotinamide**

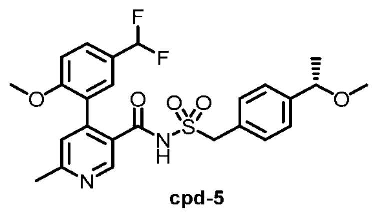

**cpd-5**

LCMS calc. for $C_{25}H_{27}F_2NO_5S$ $[M+H]^+$: m/z = 505.2; Found: 505.3. $^1H$ NMR (400 MHz, DMSO-*d*$_6$) δ 12.03 (s, 1H), 8.35 (s, 1H), 7.62 (d, *J* = 8.6 Hz, 1H), 7.50 (s, 1H), 7.32-6.89 (m, 7H), 4.67 (s, 2H), 4.33 (q, *J* = 6.4 Hz, 1H), 3.74 (s, 3H), 3.13 (s, 3H), 2.54 (s, 3H), 1.33 (d, *J* = 6.4 Hz, 3H).

**Example 6: Preparation of compound cpd-6**

The same synthetic route as that of compound **cpd-10** was adopted, except that the starting material (*R*)-4-(1-methoxyethyl)phenyl)methanesulfonamide in step 3 was replaced with (*S*)-4-(1-methoxyethyl)phenyl)methanesulfonamide.

**(*S*)-2'-(Difluoromethyl)-5'-methoxy-N-((4-(1-methoxyethyl)benzyl)sulfonyl)-6-methyl-[4,4'-bipyridine]-3-carboxamide**

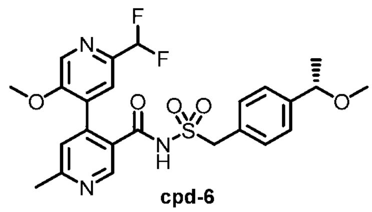

**cpd-6**

LCMS calc. for $C_{24}H_{26}F_2N_3O_5S$ $[M+H]^+$: m/z = 506.2; Found: 506.3 $^1H$ NMR (400 MHz, DMSO-d6) δ 12.16 (s, 1H), 8.52 (s, 1H), 8.45 (s, 1H), 7.65 (s, 1H), 7.38 (s, 1H), 7.35-7.27 (m, 4H), 6.99 (t, J = 55.1 Hz, 1H), 4.69 (s, 2H), 4.33 (q, J = 6.4 Hz, 1H), 3.88 (s, 3H), 3.13 (s, 3H), 2.57 (s, 3H), 1.32 (d, J = 6.4 Hz, 3H).

**Example 7: Preparation of compound cpd-7**

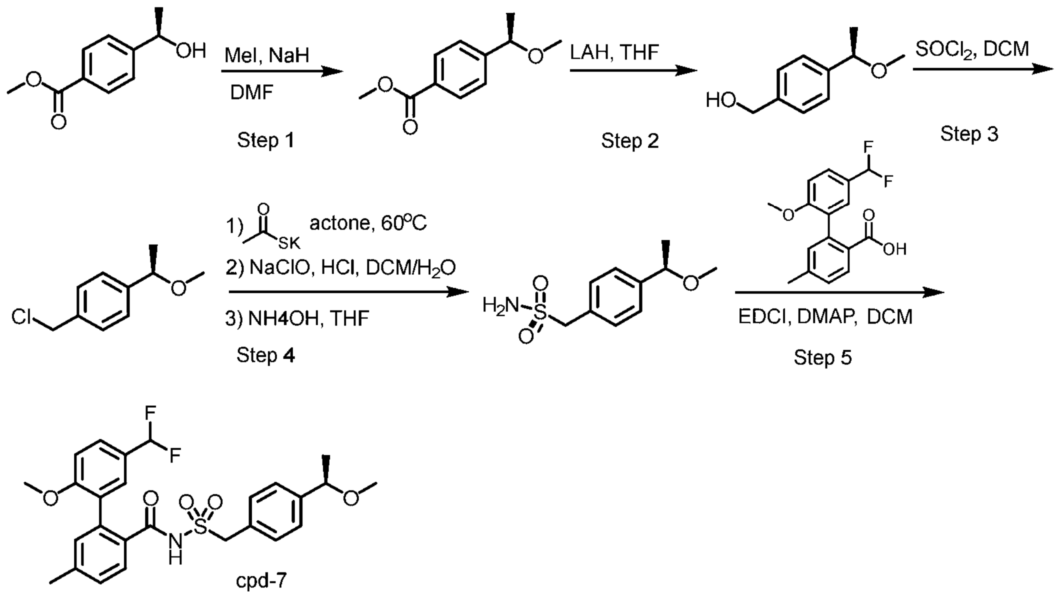


cpd-7

**Step 1: Methyl (*R*)-4-(1-methoxyethyl)benzoate**

Methyl (*R*)-4-(1-hydroxyethyl)benzoate (1.0 g, 5.55 mmol) was dissolved in N,N-dimethylformamide (10 mL), then sodium hydride (0.33 g, 8.32 mmol, 60%) was added to the solution at 0°C. The mixture was reacted at 0°C under a nitrogen atmosphere for 0.5 hours. Iodomethane (1.58 g, 11.1 mmol) was added to the reaction mixture, which was reacted at 20°C under a nitrogen atmosphere for 2 hours. Water (40 mL) was added, and the mixture was extracted twice with ethyl acetate (50 mL). The combined organic phases were sequentially washed with water (100 mL) and saturated brine (100 mL), dried over anhydrous sodium sulfate, filtered, and concentrated to obtain a residue. The residue was purified by silica gel column chromatography (ethyl acetate:petroleum ether = 0% to 30%) to obtain methyl (R)-4-(1-methoxyethyl) benzoate (0.8 g, yield: 74.3%).

**Step 2: (*R*)-4-(1-Methoxyethyl)phenyl)methanol**

To a solution of methyl (*R*)-4-(1-methoxyethyl)benzoate in tetrahydrofuran (40 mL) under a nitrogen atmosphere at 0°C was added lithium aluminum hydride (0.31 g, 8.24 mmol). The mixture was reacted at 0°C for 2 hours. After the reaction was completed, the mixture was cooled to -10°C, and 0.31 mL of water, 0.31 mL of 20% sodium hydroxide solution, and 0.93 mL of water were sequentially added slowly. The mixture was reacted at room temperature for 30 minutes, and filtered under reduced pressure. The filtrate was concentrated to obtain (R)-4-(1-methoxyethyl)phenyl) methanol (0.7 g, crude yield: >99%).

**Step 3: (*R*)-1-(chloromethyl)-4-(1-methoxyethyl)benzene**

(R)-4-(1-Methoxyethyl)phenyl)methanol (0.7 g, 4.21 mmol) was dissolved in dichloromethane (10 mL), and thionyl chloride (1.76 g, 14.8 mmol) was added thereto at 20°C. The mixture was reacted at 20°C for 1 hour. The mixture was concentrated under reduced pressure to obtain (R)-1-(chloromethyl)-4-(1-methoxyethyl)benzene (0.8 g, crude yield: >99%). The crude product was directly used in the next step.

**Step 4: (R)-4-((1-methoxyethyl)phenyl)methanesulfonamide**

(R)-1-(Chloromethyl)-4-(1-methoxyethyl)benzene (0.72 g, 4.21 mmol) was dissolved in acetone (15 mL), followed by addition of potassium thioacetate (0.5 g, 5.05 mmol). The reaction mixture was reacted at 60°C for 1 hour under a nitrogen atmosphere. The reaction mixture was diluted with water (30 mL), and the aqueous phase was extracted twice with ethyl acetate (30 mL). The combined organic phases were washed with water (50 mL) and saturated brine (50 mL), dried over anhydrous sodium sulfate, filtered, and concentrated to obtain a residue. To the concentrated residue was

added dichloromethane (10 mL) and 1N dilute hydrochloric acid (10 mL). The mixture was cooled to 0°C, and a 10% aqueous sodium hypochlorite solution (14 mL) was slowly added dropwise thereto. After reacting for 1 hour, the reaction mixture was added with concentrated aqueous ammonia to adjust the pH to 13, and reacted for an additional 0.5 hours. The reaction mixture was diluted with water (50 mL), and the aqueous phase was extracted twice with ethyl acetate (50 mL). The combined organic phases were washed with water (50 mL) and saturated brine (50 mL), dried over anhydrous sodium sulfate, filtered, and concentrated to afford a residue. The residue was purified by silica gel column chromatography (methanol:dichloromethane = 0% to 5%) to obtain (*R*)-4-(1-methoxyethyl)phenyl)methanesulfonamide (0.4 g, yield: 43.5%). LCMS calc. for $C_{10}H_{16}NO_3S$ $[M+H]^+$: m/z =230.1; Found: 230.2.

**Step 5: (*R*)-5'-(Difluoromethyl)-2'-methoxy-N-((4-(1-methoxyethyl)benzyl)sulfonyl)-5-methyl-[1,1'-biphenyl]-2-carboxamide**

(*R*)-4-(1-Methoxyethyl)phenyl)methanesulfonamide and 5'-(difluoromethyl)-2'-methoxy-5-methyl-[1,1'-biphenyl]-2-carboxylic acid (100 mg, 0.34 mmol) were dissolved in dichloromethane (6 mL). 1-ethyl-(3-dimethylaminopropyl)carbodiimide hydrochloride (131 mg, 0.68 mmol) and 4-dimethylaminopyridine (83.6 mg, 0.68 mmol) were added, and the mixture was reacted at 20°C for 10 hours. The reaction mixture was concentrated under reduced pressure and purified by silica gel column chromatography (dichloromethane:methanol = 0 to 10%) and preparative high-performance liquid chromatography (Column: XBridge Prep C18 5μm OBD 19*150mm, Mobile phase: A(10 mM $NH_4HCO_3$ in Water) B (ACN), 0-3min: 40%B, 3-7min: 40-85%B, 7-11min: 85-90%B, 11-13min: 90-95%B, 13-16min: 95%B, 16-16.5min: 95-40% B, 16.5-21min: 40%B, Flow =15mL/min) to obtain (*R*)-5'-(difluoromethyl)-2'-methoxy-N-((4-(1-methoxyethyl)benzyl)sulfonyl)-5-methyl-[1,1'-biphenyl]-2-carboxamide (52.2 mg, yield: 31.2%). LCMS calc. for $C_{26}H_{26}F_2NO_5S$ $[M-H]^-$: m/z = 502.2; Found: 502.1 $^1H$ NMR (400 MHz, DMSO-$d_6$) δ 11.78 (s, 1H), 7.56 (dd, *J* = 7.6, 2.4 Hz, 1H), 7.43 (d, *J*= 2.4 Hz, 1H), 7.33-7.11 (m, 8H), 7.02 (t, *J* = 56.0 Hz, 1H), 4.64 (s, 2H), 4.34-4.30 (m, 1H), 3.72 (s, 3H), 3.13 (s, 3H) 2.38 (s, 3H), 1.33 (d, *J* = 6.4 Hz, 3H).

**Example 8: Preparation of compound cpd-8**

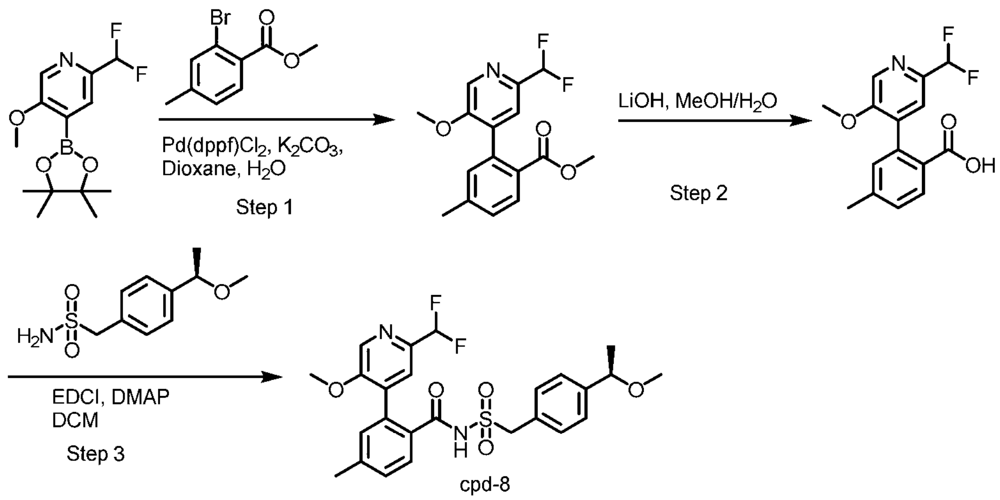

cpd-8

**Step** 1: **Methyl 2-(2-(difluoromethyl)-5-methoxypyridin-4-yl)-4-methylbenzoate**

(2-(Difluoromethyl)-5-methoxy-4-(4,4,5,5-tetramethyl-1,3,2-dioxaborolan-2-yl)pyridine (0.5 g, 1.75 mmol) and methyl 2-bromo-4-methylbenzoate (0.48 g, 2.1 mmol) were dissolved in dioxane (15 mL), followed by addition of 1,1'-bis(diphenylphosphino)ferrocene palladium dichloride (0.13 g, 0.18 mmol), potassium carbonate (0.73 g, 5.26 mmol), and water (3 mL). The mixture was reacted at 100°C under a nitrogen atmosphere for 2 hours. The reaction mixture was cooled and filtered through diatomite, and the filtrate was concentrated to obtain a residue. The residue was purified by silica gel column chromatography (ethyl acetate:petroleum ether = 0% to 30%) to obtain methyl 2-(2-(difluoromethyl)-5-methoxypyridin-4-yl)-4-methylbenzoate (0.5 g, yield: 92.3%). LCMS calc. for $C_{16}H_{16}F_2NO_3$ $[M+H]^+$: m/z = 308.1; Found: 308.2.

**Step 2: 2-(2-(Difluoromethyl)-5-methoxypyridin-4-yl)-4-methylbenzoic acid**

To a solution of methyl 2-(2-(difluoromethyl)-5-methoxypyridin-4-yl)-4-methylbenzoate (0.5 g, 1.63 mmol) in methanol (20 mL) were added water (3 mL) and lithium hydroxide (0.19 g, 8.14 mmol). The mixture was reacted at 40°C for 2 hours. After the reaction was completed, the reaction mixture was added with 4N dilute hydrochloric acid to adjust its pH to 4, then extracted twice with ethyl acetate (20 mL). The organic phases were combined, dried over anhydrous sodium sulfate, filtered, and concentrated to obtain a residue. The residue was purified by silica gel column chromatography (methanol:dichloromethane = 0% to 10%) to obtain 2-(2-(difluoromethyl)-5-methoxypyridin-4-yl)-4-methylbenzoic acid (0.32 g, yield: 67.1%). LCMS calc. for $C_{15}H_{14}F_2NO_3$ $[M+H]^+$: m/z = 294.1; Found: 294.2.

**Step 3: (*R*)-2-(2-(Difluoromethyl)-5-methoxypyridin-4-yl)-N-((4-(1-methoxyethyl)benzyl)sulfonyl)-4-methylbenzamide**

(*R*)-4-(1-Methoxyethyl)phenyl)methanesulfonamide (94 mg, 0.41 mmol) and 2-(2-(difluoromethyl)-5-methoxypyridin-4-yl)-4-methylbenzoic acid (80 mg, 0.27 mmol) were dissolved in dichloromethane (5 mL), followed by addition of 1-ethyl-(3-dimethylaminopropyl)carbodiimide hydrochloride (104 mg, 0.55 mmol) and 4-dimethylaminopyridine (66.6 mg, 0.55 mmol). The mixture was reacted at 20°C for 10 hours. The reaction mixture was concentrated under reduced pressure and purified by silica gel column chromatography (dichloromethane:methanol = 0 to 10%) and preparative high-performance liquid chromatography (Column: XBridge Prep C18 5μm OBD 19*150mm, Mobile phase: A (10 mM $NH_4HCO_3$ in Water) B (ACN), 0-3min: 40%B, 3-7min: 40-85%B, 7-11min: 85-90%B, 11-13min: 90-95%B, 13-16min: 95%B, 16-16.5min: 95-40%B, 16.5-21min: 40%B, Flow =15mL/min) to obtain (*R*)-2-(2-(difluoromethyl)-5-methoxypyridin-4-yl)-N-((4-(1-methoxyethyl)benzyl)sulfonyl)-4-methylbenzamide (46.2 mg, yield: 33.4%). LCMS calc. for $C_{25}H_{27}F_2N_2O_5S$ $[M+H]^+$: m/z = 505.2; Found: 505.3 $^1H$ NMR (400 MHz, DMSO-$d_6$) $\delta$ 11.92 (s, 1H), 8.44 (s, 1H), 7.54 (s, 1H), 7.48-7.09 (m, 7H), 7.02 (t, *J* = 55.2 Hz, 1H), 4.63 (s, 2H), 4.30-4.25 (m, 1H), 3.85 (s, 3H), 3.12 (s, 3H) 2.39 (s, 3H), 1.33 (d, *J* = 6.4 Hz, 3H).

**Example 9: Preparation of compound cpd-9**

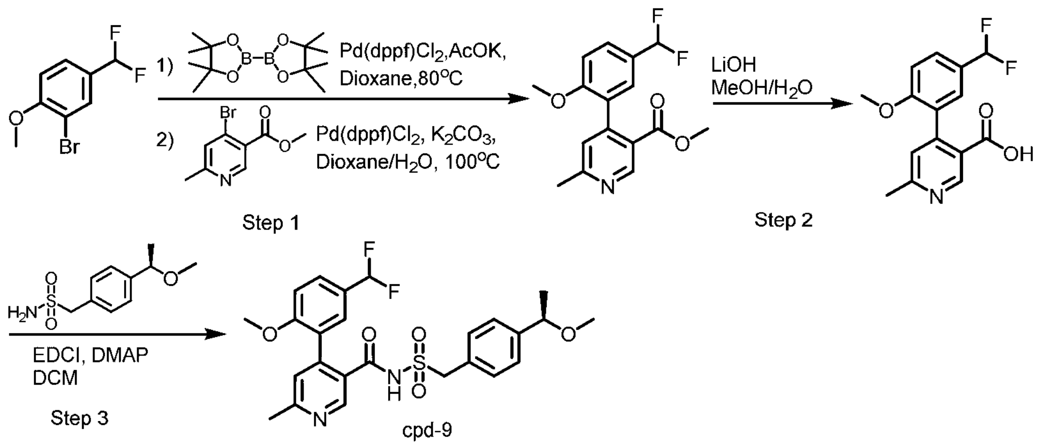

cpd-9

**Step 1: Methyl 4-(5-(difluoromethyl)-2-methoxyphenyl)-6-methylnicotinate**

2-Bromo-4-(difluoromethyl)-1-methoxybenzene (0.62 g, 2.61 mmol) and 4,4,4',4',5,5,5',5'-octamethyl-2,2'-bi(1,3,2-dioxaborolane) (0.83 g, 3.26 mmol) were dissolved in dioxane (15 mL), followed by addition of 1,1'-bis(diphenylphosphino)ferrocene palladium dichloride (0.16 g, 0.22 mmol) and potassium acetate (0.64 g, 6.52 mmol). The mixture was reacted at 100°C under a nitrogen atmosphere for 1 hour. After the reaction mixture was cooled to room temperature, methyl 4-bromo-6-methylnicotinate (0.5 g, 2.17 mmol), 1,1'-bis(diphenylphosphino)ferrocene palladium dichloride (0.16 g, 0.22 mmol), potassium carbonate (0.9 g, 6.52 mmol), and water (2 mL) were added. The mixture was reacted at 100°C under a nitrogen atmosphere for 2 hours. The reaction mixture was cooled and filtered through diatomite, and the filtrate was concentrated to obtain a residue. The residue was purified by silica gel column chromatography (ethyl acetate:petroleum ether = 0% to 100%) to obtain methyl 4-(5-(difluoromethyl)-2-methoxyphenyl)-6-methylnicotinate (0.6 g, yield: 89.8%). LCMS calc. for $C_{16}H_{16}F_2NO_3$ $[M+H]^+$: m/z = 308.1; Found: 308.3.

**Step 2: 4-(5-(Difluoromethyl)-2-methoxyphenyl)-6-methylnicotinic acid.**

To a solution of methyl 4-(5-(difluoromethyl)-2-methoxyphenyl)-6-methylnicotinate (0.6 g, 1.95 mmol) in methanol (10 mL) were added water (3 mL) and lithium hydroxide (0.28 g, 11.7 mmol). The mixture was reacted at 40°C for 2 hours. After the reaction was completed, the reaction mixture was added with 4N dilute hydrochloric acid to adjust its pH to 5, then extracted twice with ethyl acetate (20 mL). The combined organic phases were dried over anhydrous sodium sulfate, filtered, and concentrated to obtain a residue. The residue was purified by silica gel column chromatography (methanol:dichloromethane = 0% to 10%) to obtain 4-(5-(difluoromethyl)-2-methoxyphenyl)-6-methylnicotinic acid (0.4 g, yield: 69.9%). LCMS calc. for $C_{15}H_{14}F_2NO_3$ $[M+H]^+$: m/z = 294.1; Found: 294.2.

**Step 3: (*R*)-4-(5-(Difluoromethyl)-2-methoxyphenyl)-N-((4-(1-methoxyethyl)benzyl)sulfonyl)-6-methylnicotinamide**

To a solution of (*R*)-4-(1-methoxyethyl)phenyl)methanesulfonamide (80 mg, 0.35 mmol) and 4-(5-(difluoromethyl)-2-methoxyphenyl)-6-methylnicotinic acid (80 mg, 0.27 mmol) in dichloromethane (5 mL) were added 1-ethyl-(3-dimethylaminopropyl)carbodiimide hydrochloride (105 mg, 0.55 mmol) and 4-dimethylaminopyridine (66.7 mg, 0.55 mmol). The reaction mixture was stirred at 20°C for 10 hours. The reaction mixture was concentrated under reduced pressure and purified by silica gel column chromatography (dichloromethane:methanol = 0 to 10%) and preparative high performance liquid chromatography (Column: SHIMADZU Prep C18 10μm 20*250mm, Mobile phase: A (0.1%FA in $H_2O$) B (ACN), 0-2min: 40%B, 2-3.5min: 40-75%B, 3.5-6min: 75%B, 6-9min: 75-95%B, 9-11min: 95%B, 11-11.01min: 95-40% B, 11.01-14min: 40%B, Flow=20 mL/min) to obtain (*R*)-4-(5-(difluoromethyl)-2-methoxyphenyl)-N-((4-(1-methoxyethyl) benzyl)sulfonyl)-6-methylnicotinamide (10.2 mg, yield: 7.4%). LCMS calc. for $C_{25}H_{27}F_2N_2O_5S$ $[M+H]^+$: m/z = 505.2; Found: 505.3 $^1H$ NMR (400 MHz, DMSO-$d_6$) δ 12.03 (s, 1H), 8.35 (s, 1H), 8.46 (s, 1H), 7.62 (s, 1H), 7.50 (s, 1H), 7.40-7.18 (m, 6H), 7.03 (t, *J* = 56.0 Hz, 1H), 4.68 (s, 2H), 4.30-4.25 (m, 1H), 3.74 (s, 3H), 3.13 (s, 3H) 2.54 (s, 3H), 1.32 (d, *J* = 6.4 Hz, 3H).

**Example 10: Preparation of compound cpd-10**

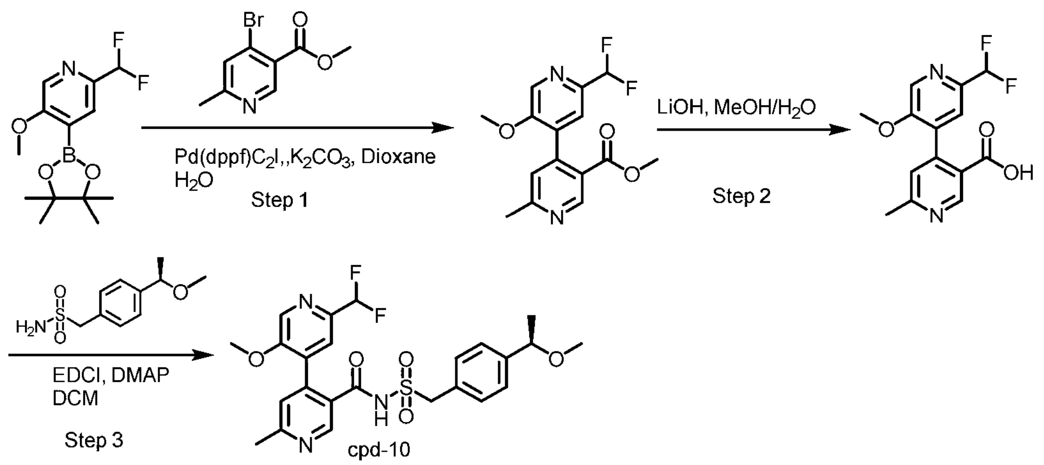


**Step 1: Methyl 2'-(difluoromethyl)-5'-methoxy-6-methyl-[4,4'-bipyridine]-3-carboxylate**

(2-(Difluoromethyl)-5-methoxy-4-(4,4,5,5-tetramethyl-1,3,2-dioxaborolan-2-yl)pyridine (1.0 g, 3.51 mmol) and methyl 4-bromo-6-methylnicotinate (0.97 g, 4.21 mmol) were dissolved in dioxane (15 mL), followed by addition of 1,1'-bis(diphenylphosphino)ferrocene palladium dichloride (0.26 g, 0.35 mmol), potassium carbonate (1.45 g, 10.5 mmol), and water (3 mL). The mixture was reacted at 100°C under a nitrogen atmosphere for 2 hours. The reaction mixture was cooled and filtered through diatomite, and the filtrate was concentrated to obtain a residue. The residue was purified by silica gel column chromatography (ethyl acetate:petroleum ether = 0% to 100%) to obtain methyl 2'-(difluoromethyl)-5'-methoxy-6-methyl-[4,4'-bipyridine]-3-carboxylate (0.7 g, 64.7%). LCMS calc. for $C_{15}H_{15}F_2N_2O_3$ $[M+H]^+$: m/z = 309.1; Found: 309.3.

Step 2: **2'-(Difluoromethyl)-5'-methoxy-6-methyl-[4,4'-bipyridine]-3-carboxylic acid**

To a solution of methyl 2'-(difluoromethyl)-5'-methoxy-6-methyl-[4,4'-bipyridine]-3-carboxylate (0.7 g, 2.27 mmol) in methanol (10 mL) were added water (3 mL) and lithium hydroxide (0.33 g, 13.6 mmol), and the reaction system was stirred at 40°C for 2 hours. After the reaction was completed, the reaction mixture added with 4N dilute hydrochloric acid to adjust its pH to 5 and extracted twice with ethyl acetate (20 mL). The combined organic phases were dried over anhydrous sodium sulfate, filtered, and concentrated to obtain a residue. The residue was purified by silica gel column chromatography (methanol:dichloromethane = 0% to 10%) to obtain 2'-(difluoromethyl)-5'-methoxy-6-methyl-[4,4'-bipyridine]-3-carboxylic acid (0.4 g, yield: 59.9%). LCMS calc. for $C_{14}H_{13}F_2N_2O_3$ $[M+H]^+$: m/z = 295.1; Found: 295.2.

**Step 3: (*R*)-2'-(Difluoromethyl)-5'-methoxy-N-((4-(1-methoxyethyl)benzyl)sulfonyl)-6-methyl-[4,4'-bipyridine]-3-carboxamide**

To a solution of (R)-4-(1-methoxyethyl)phenyl)methanesulfonamide (93.8 mg, 0.41 mmol) and 4-(5-(difluoromethyl)-2-methoxyphenyl)-6-methylnicotinic acid (80 mg, 0.27 mmol) in dichloromethane (5 mL) were added 1-ethyl-(3-dimethylaminopropyl)carbodiimide hydrochloride (105 mg, 0.55 mmol) and 4-dimethylaminopyridine (66.7 mg, 0.55 mmol). The reaction mixture was stirred at 20°C for 10 hours. The reaction mixture was concentrated under reduced pressure and purified by silica gel column chromatography (dichloromethane:methanol = 0 to 10%) and preparative high performance liquid chromatography (Column: SHIMADZU Prep C18 10μm 20*250mm, Mobile phase: A (0.1%FA in $H_2O$) B (ACN), 0-2min: 40%B, 2-3.5min: 40-75%B, 3.5-6min: 75%B, 6-9min: 75-95%B, 9-11min: 95%B, 11-11.01min: 95-40% B, 11.01-14min: 40%B, Flow=20 mL/min) to obtain (R)-2'-(difluoromethyl)-5'-methoxy-N-((4-(1-methoxyethyl)benzyl) sulfonyl)-6-methyl-[4,4'-bipyridine]-3-carboxamide (20.2 mg, yield: 14.5%). LCMS calc. for $C_{24}H_{26}F_2N_3O_5S$ $[M+H]^+$: m/z = 506.2; Found: 506.3 $^1H$ NMR (400 MHz, DMSO-$d_6$) δ 12.15 (s, 1H), 8.51 (s, 1H), 8.46 (s, 1H), 7.63 (s, 1H), 7.50-7.25 (m, 5H), 6.98 (t, *J* = 54.8 Hz, 1H), 4.68 (s, 2H), 4.30-4.25 (m, 1H), 3.88 (s, 3H), 3.12 (s, 3H) 2.56 (s, 3H), 1.32 (d, *J* = 6.8 Hz, 3H).

**Example 11: Preparation of compound cpd-11**

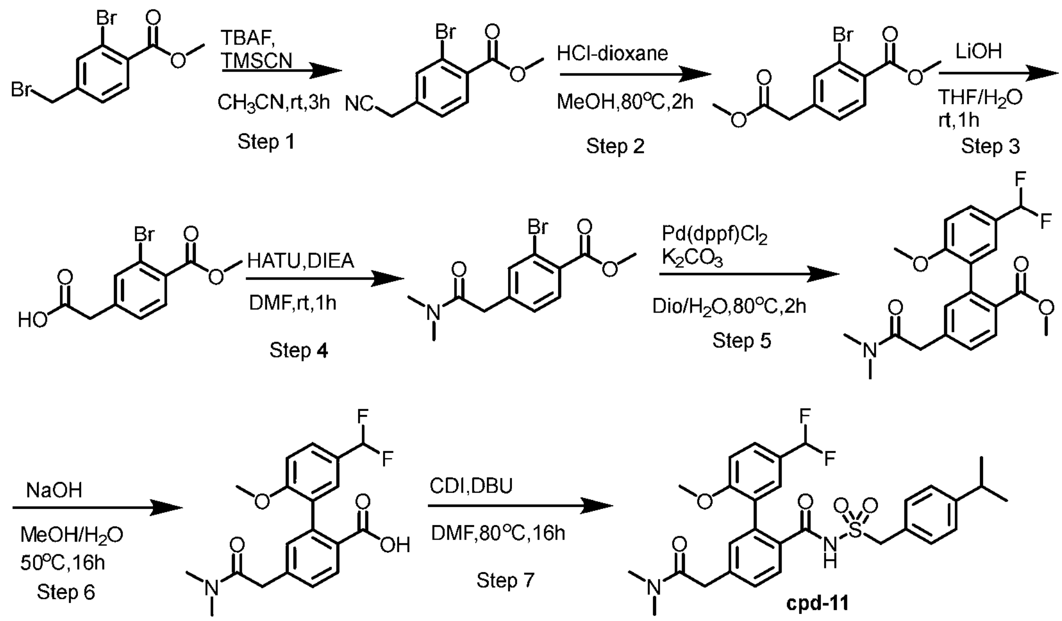


cpd-11

**Step 1: Methyl 2-bromo-4-(cyanomethyl)benzoate**

Methyl 2-bromo-4-(bromomethyl)benzoate (13 g, 42.2 mmol) was dissolved in acetonitrile (100 mL), followed by addition of trimethylsilyl cyanide (15.8 mL, 127 mmol) and a tetrahydrofuran solution of tetrabutylammonium fluoride (127 mL, 1.00 mol/L, 127 mmol). The mixture was reacted at room temperature for 3 hours. After the reaction was completed, water (50 mL) was added to quench the reaction. The mixture was extracted with ethyl acetate (200 mL × 2). The combined organic phases were dried over anhydrous sodium sulfate, filtered, and concentrated to obtain a residue. The residue was purified by silica gel column chromatography (ethyl acetate:petroleum ether = 0% to 30%) to obtain methyl 2-bromo-4-(cyanomethyl)benzoate (8.0 g, yield: 74.6%).

**Step 2: Methyl 2-bromo-4-(2-methoxy-2-oxoethyl)benzoate**

Methyl 2-bromo-4-(cyanomethyl)benzoate (5.0 g, 19.7 mmol) was dissolved in methanol (20 mL) and 4 N HCl in dioxane (30 mL). The mixture was heated to 80 °C and reacted for 2 hours. After the reaction was completed, the reaction mixture was poured into a saturated sodium bicarbonate solution and extracted with ethyl acetate (100 mL x 2). The combined organic phases were dried over anhydrous sodium sulfate, filtered, and concentrated to obtain a residue. The residue was purified by silica gel column chromatography (ethyl acetate:petroleum ether = 0% to 30%) to obtain methyl 2-bromo-4-(2-methoxy-2-oxoethyl)benzoate (4.5 g, yield: 79.7%). LCMS calc. for $C_{11}H_{12}BrO_4$ $[M+H]^+$: m/z = 287.0/289.0; Found: 287.1/289.1.

**Step 3: 2-(3-Bromo-4-(methoxycarbonyl)phenyl)acetic acid**

Methyl 2-bromo-4-(2-methoxy-2-oxoethyl)benzoate (4.5 g, 15.7 mmol) was dissolved in tetrahydrofuran (40 mL) and water (8 mL), followed by addition of lithium hydroxide (1.13 g, 47.0 mmol). The mixture was reacted at room temperature for 1 hour. After the reaction was completed, the reaction mixture was added with 1N dilute hydrochloric acid to adjust its pH to 5, and extracted with ethyl acetate (100 mL × 3). The combined organic phases were dried over anhydrous sodium sulfate, filtered, and concentrated to obtain a residue. The residue was purified by silica gel column chromatography (methanol:dichloromethane = 0% to 10%) to obtain 2-(3-bromo-4-(methoxycarbonyl)phenyl)acetic acid (2.8 g, yield: 88.8%). LCMS calc. for $C_{10}H_{10}BrO_4$ $[M+H]^+$: m/z = 273.0/275.0; Found:273.0/275.0.

**Step 4: Methyl 2-bromo-4-(2-(dimethylamino)-2-oxoethyl)benzoate**

2-(3-Bromo-4-(methoxycarbonyl)phenyl)acetic acid (0.7 g, 2.56 mmol) was dissolved in N,N-dimethylformamide (8 mL), followed by addition of dimethylamine hydrochloride (0.31 g, 3.85 mmol), 2-(7-azabenzotriazol-1-yl)-N,N,N',N'-tetramethyluronium hexafluorophosphate (1.17 g, 3.08 mmol), and N,N-diisopropylethylamine (0.99 g, 7.69 mmol). The mixture was reacted at room temperature for 1 hour. After the reaction was completed, the reaction mixture was quenched with water, then extracted with ethyl acetate (50 mL × 2). The combined organic phases were dried over anhydrous sodium sulfate, filtered, and concentrated to obtain a residue. The residue was purified by silica gel column chromatography (ethyl acetate:petroleum ether = 0% to 50%) to obtain methyl 2-bromo-4-(2-(dimethylamino)-2-oxoethyl) benzoate (0.7 g, yield: 91%). LCMS calc. for $C_{12}H_{15}BrNO_3$ $[M+H]^+$: m/z = 300.0/302.0; Found: 300.1/302.1.

**Step 5: Methyl 5'-(difluoromethyl)-5-(2-(dimethylamino)-2-oxoethyl)-2'-methoxy-[1,1'-biphenyl]-2-carboxylate**

Methyl 2-bromo-4-(2-(dimethylamino)-2-oxoethyl)benzoate (0.63 g, 2.11 mmol) was dissolved in dioxane (8 mL) and water (2 mL), followed by addition of 2-(5-(difluoromethyl)-2-methoxyphenyl)-4,4,5,5-tetramethyl-1,3,2-dioxaborolane (0.6 g, 2.11 mmol), potassium carbonate (0.88 g, 6.34 mmol), and 1,1'-bis(diphenylphosphino)ferrocene palladium dichloride (0.15 g, 0.21 mmol). The mixture was heated to 80°C and reacted for 2 hours under an argon atmosphere. After the reaction was completed, the reaction mixture was quenched with water, then extracted with ethyl acetate (50 mL × 2). The combined organic phases were dried over anhydrous sodium sulfate, filtered, and concentrated to obtain a residue. The residue was purified by silica gel column chromatography (ethyl acetate:petroleum ether = 0% to 50%) to obtain methyl 5'-(difluoromethyl)-5-(2-(dimethylamino)-2-oxoethyl)-2'-methoxy-[1,1'-biphenyl]-2-carboxylate (0.5 g, yield: 62.7%). LCMS calc. for $C_{20}H_{22}F_2NO_4$ $[M+H]^+$: m/z = 378.1; Found: 378.3.

**Step 6: 5'-(Difluoromethyl)-5-(2-(dimethylamino)-2-oxoethyl)-2'-methoxy-[1,1'-biphenyl]-2-carboxylic acid**

Methyl 5'-(difluoromethyl)-5-(2-(dimethylamino)-2-oxoethyl)-2'-methoxy-[1,1'-biphenyl]-2-carboxylate (0.5 g, 1.32 mmol) was dissolved in methanol (8 mL) and water (2 mL). Sodium hydroxide (0.16 g, 3.97 mmol) was added to the reaction mixture, which was heated to 50°C and reacted for 16 hours. After the reaction was completed, the reaction mixture was adjusted to pH = 5 with 1N dilute hydrochloric acid, and extracted with ethyl acetate (50 mL × 3). The combined organic phases were dried over anhydrous sodium sulfate, filtered, and concentrated to obtain a residue. The residue was purified by silica gel column chromatography (methanol:dichloromethane = 0% to 10%) to obtain 5'-(difluoromethyl)-5-(2-(dimethylamino)-2-oxoethyl)-2'-methoxy-[1,1'-biphenyl]-2-carboxylic acid (0.35 g, yield: 72.7%). LCMS calc. for : $C_{19}H_{20}F_2NO_4$ $[M+H]^+$: m/z = 364.1; Found:364.2.

**Step 7: 5'-(Difluoromethyl)-5-(2-(dimethylamino)-2-oxoethyl)-N-((4-isopropylbenzyl)sulfonyl)-2'-methoxy-[1,1'-biphenyl]-2-carboxamide**

5'-(Difluoromethyl)-5-(2-(dimethylamino)-2-oxoethyl)-2'-methoxy-[1,1'-biphenyl]-2-carboxylic acid (100 mg, 0.28 mmol) was dissolved in N,N-dimethylformamide (3 mL), followed by addition of 1,1'-carbonyldiimidazole (53.5 mg, 0.33 mmol). The mixture was reacted at 80°C for 10 minutes. Subsequently, (4-isopropylphenyl)methanesulfonamide (88 mg, 0.41 mmol) and 1,8-diazabicyclo[5.4.0]undec-7-ene (0.08 mL, 0.55 mmol) were added. The mixture was reacted at 80°C for an additional 1 hour. After the reaction was completed, the reaction mixture was quenched with water, then extracted with ethyl acetate (30 mL × 2). The combined organic phases were dried over anhydrous sodium sulfate, filtered, and concentrated to obtain a residue. The residue was purified by preparative high-performance liquid chromatography (Column: XBridge Prep C18 5μm OBD 19*150mm, Mobile phase: A (10 mM $NH_4HCO_3$ in Water) B (ACN), 0-3min: 40%B, 3-7min: 40-85%B, 7-11min: 85-90%B, 11-13min: 90-95%B, 13-16min: 95%B, 16-16.5min: 95-40%B, 16.5-21min: 40%B, Flow =15mL/min) to obtain 5'-(difluoromethyl)-5-(2-(dimethylamino)-2-oxoethyl)-N-((4-isopropylbenzyl)sulfonyl)-2'-methoxy-[1,1'-biphenyl]-2-carboxamide (51.0 mg, yield: 33.2%). LCMS calc. for $C_{29}H_{33}F_2N_2O_5S$ $[M+H]^+$: m/z = 559.2; Found: 559.2. $^1H$ NMR (400 MHz, DMSO-d6) δ 11.80 (s, 1H), 7.56 (d, J = 8.5 Hz, 1H), 7.41 (s, 1H), 7.31-6.89 (m, 9H), 4.60 (s, 2H), 3.79 (s, 2H), 3.73 (s, 3H), 3.04 (s, 3H), 2.90 (p, J = 6.9 Hz, 1H), 2.83 (s, 3H), 1.20 (d, J = 6.9 Hz, 6H).

**Example 12: Preparation of compound cpd-12**

The same synthetic route as that of compound **cpd-11** was adopted, except that the starting material dimethylamine hydrochloride in step 4 was replaced with N-methylcyclopropylamine hydrochloride.

**5-(2-(Cyclopropyl(methyl)amino)-2-oxoethyl)-5'-(difluoromethyl)-N-((4-isopropylbenzyl)sulfonyl)-2'-methoxy-[1,1'-biphenyl]-2-carboxamide**

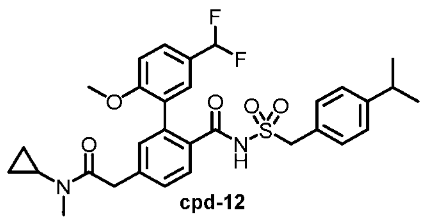

**cpd-12**

LCMS calc. for $C_{31}H_{35}F_2N_2O_5S$ $[M+H]^+$: m/z = 585.2; Found: 585.1 $^1H$ NMR (400 MHz, DMSO-d6) δ 11.80 (s, 1H), 7.58-7.54 (m, 1H), 7.41 (d, J=2.1 Hz, 1H), 7.31-7.17 (m, 7H), 7.15 -6.89 (m, 2H), 4.61 (s, 2H), 3.96 (s, 2H), 3.73 (s, 3H), 2.85-2.75 (m, 4H), 2.81 (s, 3H), 1.20 (d, J=6.9 Hz, 6H), 0.97- 0.66 (m, 4H).

**Example 13: Preparation of compound cpd-13**

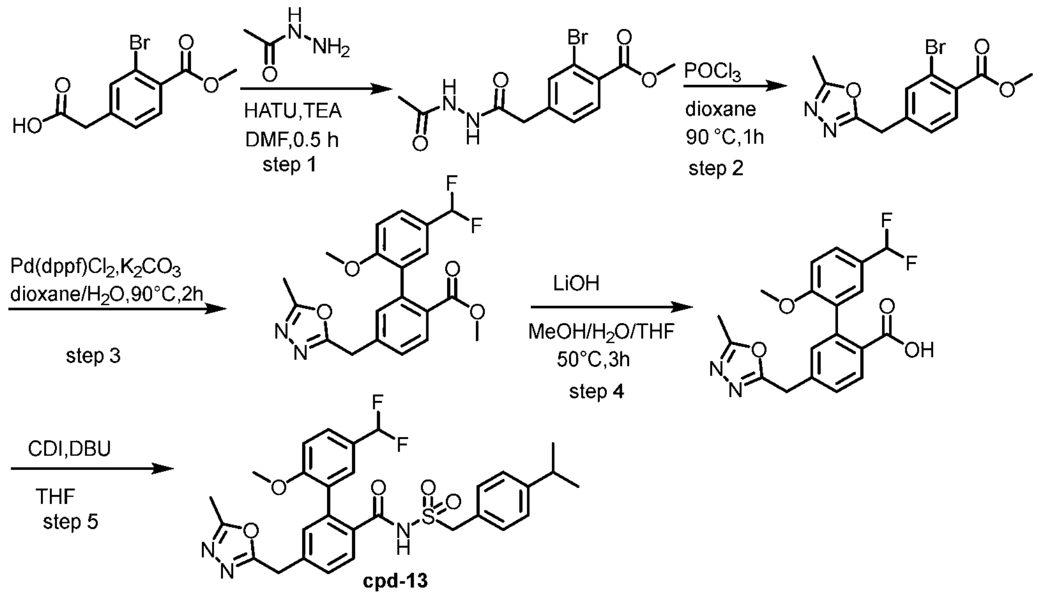


cpd-13

**Step** 1: **Methyl 4-(2-(2-acetylhydrazinyl)-2-oxoethyl)-2-bromobenzoate**

To a solution of 2-(3-bromo-4-(methoxycarbonyl)phenyl)acetic acid (0.5 g, 1.83 mmol) in N,N-dimethylformamide (8.00 mL) at room temperature were added acetyl hydrazine (0.15 g, 2.01 mmol), 2-(7-azabenzotriazol-1-yl)-N,N,N',N'-tetramethyluronium hexafluorophosphate (0.84 g, 2.2 mmol), and triethylamine (0.53 mL, 3.66 mmol). The mixture was reacted at room temperature for 30 minutes. Water (10 mL) was added thereto, and the mixture was added with 1 N aqueous hydrochloric acid to adjust its pH to 5. The mixture was extracted with ethyl acetate (50 mL × 3), washed sequentially with saturated brine (50 mL × 2) and water (50 mL × 2). The combined organic phases were dried over anhydrous sodium sulfate, filtered, and concentrated to obtain a residue. The residue was purified by silica gel column chromatography (methanol:dichloromethane = 0% to 10%) to obtain methyl 4-(2-(2-acetylhydrazinyl)-2-oxoethyl)-2-bromobenzoate (0.3 g, yield: 49.8%). LCMS calc. for $C_{12}H_{14}BrN_2O_4$ $[M+H]^+$: m/z = 329.0/331.0; Found: 329.1/331.1.

**Step 2: Methyl 2-bromo-4-((5-methyl-1,3,4-oxadiazol-2-yl)methyl)benzoate**

To a dioxane (8 mL) solution of methyl 4-(2-(2-acetylhydrazinyl)-2-oxoethyl)-2-bromobenzoate (0.3 g, 0.91 mmol) at room temperature was added phosphorus oxychloride (0.11 mL, 1.18 mmol). The mixture was reacted at 90°C for 1 hour. Water (10 mL) was added thereto, and the mixture was added with an aqueous sodium bicarbonate solution to adjust its pH to 7. The mixture was extracted with ethyl acetate (50 mL × 3), washed sequentially with saturated brine (50 mL × 2) and water (50 mL × 2). The combined organic phases were dried over anhydrous sodium sulfate, filtered, and concentrated to obtain a residue. The residue was purified by silica gel column chromatography (methanol:dichloromethane = 0% ~ 10%) to obtain methyl 2-bromo-4-((5-methyl-1,3,4-oxadiazol-2-yl)methyl)benzoate (0.27 g, yield: 95.2%). LCMS calc. for $C_{12}H_{12}BrN_2O_3$ $[M+H]^+$: m/z = 311.0/313.0; Found: 311.1/313.1.

**Step 3: Methyl 5'-(difluoromethyl)-2'-methoxy-5-((5-methyl-1,3,4-oxadiazol-2-yl)methyl)-[1,1'-biphenyl]-2-carboxylate**

To a solution of methyl 2-bromo-4-((5-methyl-1,3,4-oxadiazol-2-yl)methyl)benzoate (0.27 g, 0.87 mmol) in dioxane (8 mL) and water (1 mL) at room temperature were added 2-[5-(difluoromethyl)-2-methoxy-phenyl]-4,4,5,5-tetramethyl-1,3,2-dioxaborolane (0.49 g, 1.74 mmol), [1,1'-bis(diphenylphosphino)ferrocene]dichloropalladium(II) dichloromethane complex (0.07 g, 0.09 mmol), and potassium carbonate (0.36 g, 2.6 mmol). The mixture was reacted at 90°C under an argon atmosphere for 2 hours. Water (10 mL) was added thereto, and the mixture was added with 1 N aqueous hydrochloric acid to adjust its pH to 5. The mixture was extracted with ethyl acetate (50 mL × 3), washed sequentially with saturated brine (50 mL × 2) and water (50 mL × 2). The combined organic phases were dried over anhydrous sodium sulfate, filtered, and concentrated to obtain a residue. The residue was purified by silica gel column chromatography (methanol:dichloromethane = 0% to 10%) to obtain methyl 5'-(difluoromethyl)-2'-methoxy-5-((5-methyl-1,3,4-oxadiazol-2-yl)methyl)-[1,1'-biphenyl]-2-carboxylate (0.3 g, yield: 89%). LCMS calc. for $C_{20}H_{19}F_2N_2O_4$ $[M+H]^+$: m/z = 389.1; Found: 389.2.

**Step 4: 5'-(Difluoromethyl)-2'-methoxy-5-((5-methyl-1,3,4-oxadiazol-2-yl)methyl)-[1,1'-biphenyl]-2-carboxylic acid**

To a solution of methyl 5'-(difluoromethyl)-2'-methoxy-5-((5-methyl-1,3,4-oxadiazol-2-yl)methyl)-[1,1'-biphenyl]-2-carboxylate (0.27 g, 0.7 mmol) in methanol (3 mL)/tetrahydrofuran (3 mL)/water (3 mL) at room temperature was added lithium hydroxide (0.08 g, 3.48 mmol). The mixture was reacted at 50°C for 3 hours. Water (10 mL) was added thereto, and the mixture was added with 1 N aqueous hydrochloric acid to adjust its pH to 5. The mixture was extracted with ethyl acetate (50 mL × 3), washed sequentially with saturated brine (50 mL × 2) and water (50 mL × 2). The combined organic phases were dried over anhydrous sodium sulfate, filtered, and concentrated to obtain a residue. The residue was purified by silica gel column chromatography (methanol:dichloromethane = 0% to 10%) to obtain 5'-(difluoromethyl)-2'-methoxy-5-((5-methyl-1,3,4-oxadiazol-2-yl)methyl)-[1,1'-biphenyl]-2-carboxylic acid (0.27 g, yield: 98.6%). LCMS calc. for $C_{19}H_{17}F_2N_2O_4$ $[M+H]^+$: m/z =375.1; Found: 375.1.

Step 5: **5'-(Difluoromethyl)-N-((4-isopropylbenzyl)sulfonyl)-2'-methoxy-5-((5-methyl-1,3,4-oxadiazol-2-yl)methyl)-[1,1'-biphenyl]-2-carboxamide**

To a solution of 5'-(difluoromethyl)-2'-methoxy-5-((5-methyl-1,3,4-oxadiazol-2-yl)methyl)-[1,1'-biphenyl]-2-carboxylic acid (100 mg, 0.27 mmol) in tetrahydrofuran (3 mL) at room temperature was added 1,1'-carbonyldiimidazole (65 mg, 0.4 mmol). The reaction mixture was reacted at 50°C for 15 minutes, then cooled to room temperature. (4-Isopropylphenyl)methanesulfonamide (114 mg, 0.53 mmol) and 1,8-diazabicyclo[5.4.0]undec-7-ene (0.08 mL, 0.53 mmol) were added thereto. The reaction mixture was heated to 75°C and reacted for 2 hours. Water (10 mL) was added thereto, and the mixture was added with 1 N aqueous hydrochloric acid to adjust its pH to 5. The mixture was extracted with ethyl acetate (50 mL × 3), washed sequentially with saturated brine (50 mL × 2) and water (50 mL × 2). The combined organic phases were dried over anhydrous sodium sulfate, filtered, and concentrated to obtain a residue. The residue was purified by preparative high performance liquid chromatography (Column: SHIMADZU Prep C18 10μm 20*250mm; Mobile phase: A (0.1% FA in $H_2O$) B (ACN); 0-2 min: 40% B, 2-4 min: 40-80% B, 4-6 min: 80% B, 6-8 min: 80-95% B, 8-12 min: 95% B, 12-12.01 min: 95-40% B, 12.01-15 min: 40% B, Flow=20 mL/min) to obtain 5'-(difluoromethyl)-N-((4-isopropylbenzyl)sulfonyl)-2'-methoxy-5-((5-methyl-1,3,4-oxadiazol-2-yl)methyl)-[1,1'-biphenyl]-2-carboxamide (30.4 mg, yield: 19.8%). LCMS calc. for $C_{29}H_{30}F_2N_3O_5S$ $[M+H]^+$: m/z =570.2; Found: 570.2. $^1H$ NMR (400 MHz, DMSO-$d_6$) $\delta$ 11.84 (s, 1H), 7.60-7.53 (m, 1H), 7.46-7.39 (m, 1H), 7.35 (s, 2H), 7.31 (s, 1H), 7.25 (d, J = 8.0 Hz, 2H), 7.20 (d, J = 8.0 Hz, 2H), 7.18-6.86 (m, 2H), 4.60 (s, 2H), 4.34 (s, 2H), 3.72 (s, 3H), 2.97-2.80 (m, 1H), 2.45 (s, 3H), 1.20 (d, J = 6.8 Hz, 6H).

**Example 14: Preparation of compound cpd-14**

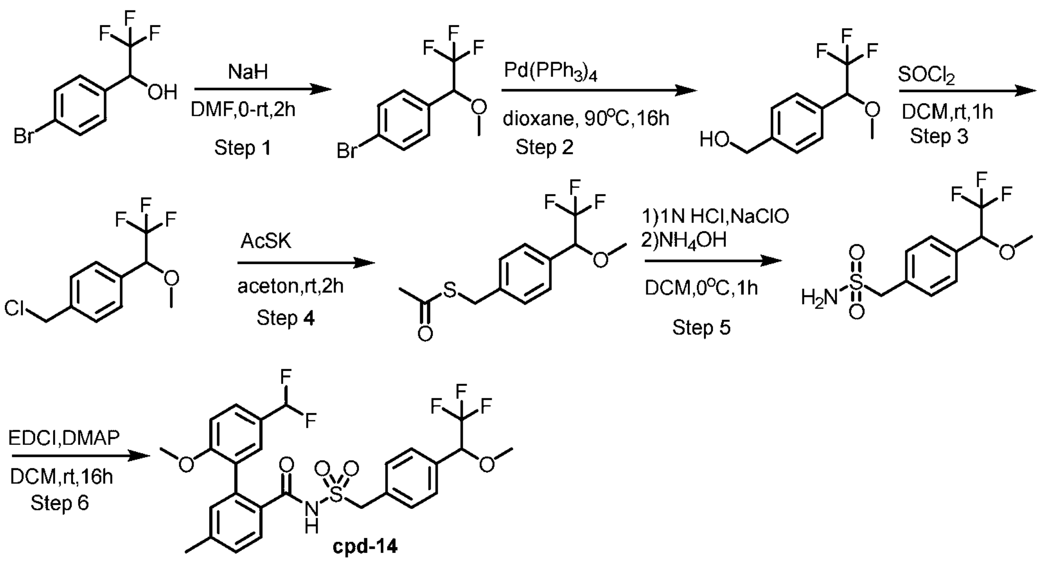


**cpd-14**

**Step 1: 1-Bromo-4-(2,2,2-trifluoro-1-methoxyethyl)benzene**

1-(4-bromophenyl)-2,2,2-trifluoroethan-1-ol (2.5 g, 9.8 mmol) was dissolved in N,N-dimethylformamide (20 mL),

followed by addition of sodium hydride (0.59 g, 14.7 mmol, 60% purity) at 0 °C. The mixture was reacted at 0°C under a nitrogen atmosphere for 0.5 hours. Then iodomethane (0.92 mL, 14.7 mmol) was added, and the reaction was continued at 20°C under a nitrogen atmosphere for 2 hours. The reaction mixture was quenched with saturated aqueous ammonium chloride solution, then extracted with ethyl acetate (100 mL × 2). The combined organic phases were dried over anhydrous sodium sulfate, filtered, and concentrated to obtain a residue. The residue was purified by silica gel column chromatography (ethyl acetate:petroleum ether = 0% to 20%) to obtain 1-bromo-4-(2,2,2-trifluoro-1-methoxyethyl)benzene (1.6 g, yield: 60.7%).

**Step 2: (4-(2,2,2-Trifluoro-1-methoxyethyl)phenyl)methanol**

1-Bromo-4-(2,2,2-trifluoro-1-methoxyethyl)benzene (1.6 g, 6.27 mmol) was dissolved in dioxane (20 mL), and then (tributylstannyl)methanol (3.02 g, 9.41 mmol) and tetrakis(triphenylphosphine)palladium (0.72 g, 0.63 mmol) were added to the reaction mixture. After the reaction was completed, the reaction mixture was quenched with an aqueous potassium fluoride solution, then extracted with ethyl acetate (100 mL × 2). The combined organic phases were dried over anhydrous sodium sulfate, filtered, and concentrated to obtain a residue. The residue was purified by silica gel column chromatography (ethyl acetate:petroleum ether = 0% to 30%) to obtain (4-(2,2,2-trifluoro-1-methoxyethyl)phenyl)methanol (0.9 g, yield: 65.2%).

**Step 3: 1-(Chloromethyl)-4-(2,2,2-trifluoro-1-methoxyethyl)benzene**

(4-(2,2,2-Trifluoro-1-methoxyethyl)phenyl)methanol (0.5 g, 2.27 mmol) was dissolved in dichloromethane (8 mL), followed by addition of thionyl chloride (0.82 mL, 11.4 mmol). The mixture was reacted at room temperature for 1 hour, and concentrated under reduced pressure to obtain crude 1-(chloromethyl)-4-(2,2,2-trifluoro-1-methoxyethyl)benzene (0.54 g, crude yield: 99.7%).

**Step 4: *S*-(4-(2,2,2-Trifluoro-1-methoxyethyl)benzyl) ethanethioate**

1-(Chloromethyl)-4-(2,2,2-trifluoro-1-methoxyethyl)benzene (0.54 g, 2.26 mmol) was dissolved in acetone (10 mL), followed by addition of potassium thioacetate (0.39 g, 3.39 mmol). The mixture was reacted at room temperature for 2 hours. After the reaction was completed, the mixture was filtered, and the filtrate was concentrated to obtain a residue. The residue was purified by silica gel column chromatography (ethyl acetate:petroleum ether = 0% to 20%) to obtain *S*-(4-(2,2,2-trifluoro-1-methoxyethyl)benzyl)ethanethioate (0.5 g, yield: 79.4%).

**Step 5: (4-(2,2,2-Trifluoro-1-methoxyethyl)phenyl)methanesulfonamide**

*S*-(4-(2,2,2-trifluoro-1-methoxyethyl)benzyl) ethanethioate (0.5 g, 1.8 mmol) was dissolved in dichloromethane (8 mL). 1 N dilute hydrochloric acid **(10.8 mL, 10.8 mmol)** and then a 10% aqueous sodium hypochlorite solution **(8.02 g, 10.8 mmol)** were added to the reaction mixture. After reacting for 1 hour, the reaction mixture was added with concentrated aqueous ammonia to adjust the pH to 11, and reacted for an additional 0.5 hours. The reaction mixture was diluted with water (50 mL) and extracted with ethyl acetate (50 mL × 3). The combined organic phases were dried over anhydrous sodium sulfate, filtered, and concentrated to obtain a residue. The residue was purified by silica gel column chromatography (methanol:dichloromethane = 0% to 10%) to obtain (4-(2,2,2-trifluoro-1-methoxyethyl)phenyl)methanesulfonamide (0.3 g, yield: 59.4%). LCMS calc. for $C_{10}H_{11}F_3NO_3S$ $[M-H]^-$: m/z =282.0; Found: 282.1.

**Step 6: 5'-(Difluoromethyl)-2'-methoxy-5-methyl-N-((4-(2,2,2-trifluoro-1-methoxyethyl)benzyl)sulfonyl)-[1,1'-biphenyl]-2-carboxamide**

5'-(Difluoromethyl)-2'-methoxy-5-methyl-[1,1'-biphenyl]-2-carboxylic acid (100 mg, 0.34 mmol) was dissolved in dichloromethane (3 mL), followed by addition of (4-(2,2,2-trifluoro-1-methoxyethyl)phenyl)methanesulfonamide (116 mg, 0.41 mmol), 1-ethyl-(3-dimethylaminopropyl)carbodiimide hydrochloride (78.7 mg, 0.41 mmol), and 4-dimethylaminopyridine (83.6 mg, 0.68 mmol). The mixture was reacted at room temperature for 16 hours. After the reaction was completed, the reaction mixture was concentrated under reduced pressure. The residue was purified by silica gel column chromatography (dichloromethane:methanol = 0% to 10%) and preparative high-performance liquid chromatography (Column: XBridge Prep C18 5μm OBD 19*150mm, Mobile phase: A(10 mM $NH_4HCO_3$ in Water) B (ACN), 0-3min: 40%B, 3-7min: 40-85%B, 7-11min: 85-90%B, 11-13min: 90-95%B, 13-16min: 95%B, 16-16.5min: 95-40%B, 16.5-21min: 40%B, Flow =15mL/min) to obtain 5'-(difluoromethyl)-2'-methoxy-5-methyl-N-((4-(2,2,2-trifluoro-1-methoxyethyl)benzyl)sulfonyl)-[1,1'-biphenyl]-2-carboxamide (68.9 mg, yield: 36.1%). LCMS calc. for $C_{26}H_{25}F_5NO_5S$ $[M+H]^+$: m/z = 558.1; Found: 558.4. $^1H$ NMR (400 MHz, DMSO-*d*6) δ 11.84 (s, 1H), 7.59-7.53 (m, 1H), 7.49-7.41 (m, 3H), 7.38 (d, *J* = 8.0 Hz, 2H),

7.22-6.87 (m, 5H), 5.10 (q, *J* = 7.0 Hz, 1H), 4.69 (s, 2H), 3.72 (s, 3H), 3.35 (s, 3H), 2.38 (s, 3H).

**Example 15: Preparation of compound cpd-15**

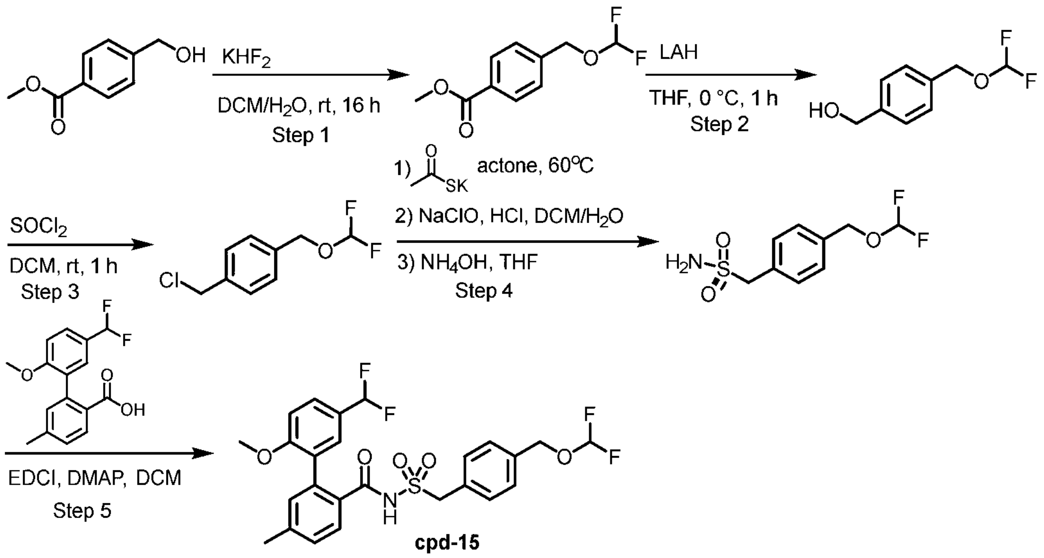


cpd-15

**Step 1: Methyl 4-(difluoromethoxy)methylbenzoate**

Methyl 4-(hydroxymethyl)benzoate (1.0 g, 6.02 mmol) was dissolved in dichloromethane (10 mL), followed by addition of potassium hydrogen fluoride (1.88 g, 24.1 mmol), (bromodifluoromethyl)trimethylsilane (2.44 g, 12 mmol), and water (10 mL). The mixture was reacted at 25°C for 16 hours. The mixture was added with water (40 mL) and extracted twice with dichloromethane (50 mL). The combined organic phases were sequentially washed with water (100 mL) and saturated brine (100 mL), dried over anhydrous sodium sulfate, filtered, and concentrated to obtain a residue. The residue was purified by silica gel column chromatography (ethyl acetate:petroleum ether = 0% to 10%) to obtain methyl 4-(difluoromethoxy)methylbenzoate (0.32 g, yield: 24.6%). LCMS calc. for $C_{10}H_{11}F_2O_3$ $[M+H]^+$: m/z =217.1; Found: 217.1.

**Step 2: (4-((Difluoromethoxy)methyl)phenyl)methanol**

To a solution of methyl 4-(difluoromethoxy)methylbenzoate (0.32 g, 1.48 mmol) in tetrahydrofuran (10 mL) under a nitrogen atmosphere at 0°C was added lithium aluminum hydride (67.4 mg, 1.78 mmol). The mixture was reacted at 0°C for 2 hours. After the reaction was completed, the mixture was frozen to -10°C, and water (0.1 mL), 20% sodium hydroxide solution (0.1 mL), and water (0.3 mL) were sequentially added slowly thereto. The mixture was stirred at room temperature for 30 minutes and filtered under reduced pressure. The filtrate was concentrated to obtain (4-((difluoromethoxy)methyl) phenyl)methanol (0.23 g, yield: 82.6%).

**Step 3: 1-(Chloromethyl)-4-((difluoromethoxy)methyl)benzene**

(4-((Difluoromethoxy)methyl)phenyl)methanol (0.7 g, 4.21 mmol) was dissolved in dichloromethane (50 mL), followed by addition of thionyl chloride (330 mg, 2.8 mmol). The mixture was reacted at 20°C for 1 hour. The mixture was concentrated under reduced pressure to obtain a residue, 1-(chloromethyl)-4-(difluoromethoxy)methyl)benzene (0.32 g, crude). The crude product was directly used in the next step.

**Step 4: (4-((Difluoromethoxy)methyl)phenyl)methanesulfonamide**

1-(Chloromethyl)-4-(difluoromethoxy)methyl)benzene (0.32 g, crude) was dissolved in acetone (10 mL), followed by addition of potassium thioacetate (170 mg, 1.49 mmol). The reaction mixture was reacted at 60°C for 1 hour under a nitrogen atmosphere. The reaction mixture was diluted with water (30 mL), and the aqueous phase was extracted twice with ethyl acetate (30 mL). The combined organic phases were washed with water (50 mL) and saturated brine (50 mL),

dried over anhydrous sodium sulfate, filtered, and concentrated to obtain a residue. To the residue was added dichloromethane (5 mL) and 1N dilute hydrochloric acid (5 mL). The mixture was cooled to 0°C, and a 10% aqueous sodium hypochlorite solution (7 mL) was slowly added dropwise thereto. After reacting for 1 hour, the reaction mixture was added with concentrated aqueous ammonia to adjust the pH to 13, and reacted for an additional 0.5 hours. The reaction mixture was diluted with water (50 mL), and the aqueous phase was extracted twice with ethyl acetate (50 mL). The combined organic phases were washed with water (50 mL) and saturated brine (50 mL), dried over anhydrous sodium sulfate, filtered, and concentrated to afford a residue. The residue was purified by silica gel column chromatography (methanol:dichloromethane = 0% to 5%) to obtain (4-((difluoromethoxy)methyl)phenyl)methanesulfonamide (0.21 g, yield: 67.5%). LCMS calc. for $C_9H_{12}F_2NO_3S$ $[M+H]^+$: m/z =252.0; Found: 252.1.

**Step 5: *N*-((4-((Difluoromethoxy)methyl)benzyl)sulfonyl)-5'-(difluoromethyl)-2'-methoxy-5-methyl-[1,1'-biphenyl]-2-carboxamide**

(4-((Difluoromethoxy)methyl)phenyl)methanesulfonamide (150 mg, 0.6 mmol) and 5'-(difluoromethyl)-2'-methoxy-5-methyl-[1,1'-biphenyl]-2-carboxylic acid (145 mg, 0.5 mmol) were dissolved in dichloromethane (6 mL), followed by addition of 1-ethyl-(3-dimethylaminopropyl)carbodiimide hydrochloride (143 mg, 0.75 mmol) and 4-dimethylaminopyridine (122 mg, 0.99 mmol). The mixture was reacted at 20°C for 10 hours. The reaction mixture was concentrated under reduced pressure and purified by silica gel column chromatography (dichloromethane:methanol = 0% to 10%) and preparative high-performance liquid chromatography (Column: XBridge Prep C18 5μm OBD 19*150mm, Mobile phase: A (10 mM $NH_4HCO_3$ in Water) B (ACN), 0-3min: 40%B, 3-7min: 40-85%B, 7-11min: 85-90%B, 11-13min: 90-95%B, 13-16min: 95%B, 16-16.5min: 95-40%B, 16.5-21min: 40%B, Flow =15mL/min) to obtain N-((4-((difluoromethoxy)methyl)benzyl)sulfonyl)-5'-(difluoromethyl)-2'-methoxy-5-methyl-[1,1'-biphenyl]-2-carboxamide (126 mg, yield: 40.2%). LCMS calc. for $C_{25}H_{24}F_4NO_5S$ $[M+H]^+$: m/z = 526.1; Found: 526.3 $^1$H NMR (400 MHz, DMSO-d6) δ 11.78 (s, 1H), 7.60-7.50 (m, 1H), 7.45-7.19 (m, 7H), 7.17-6.60 (m, 4H), 4.93 (s, 2H), 4.66 (s, 2H), 3.72 (s, 3H), 2.38 (s, 3H).

**Example 16: Preparation of compound cpd-16**

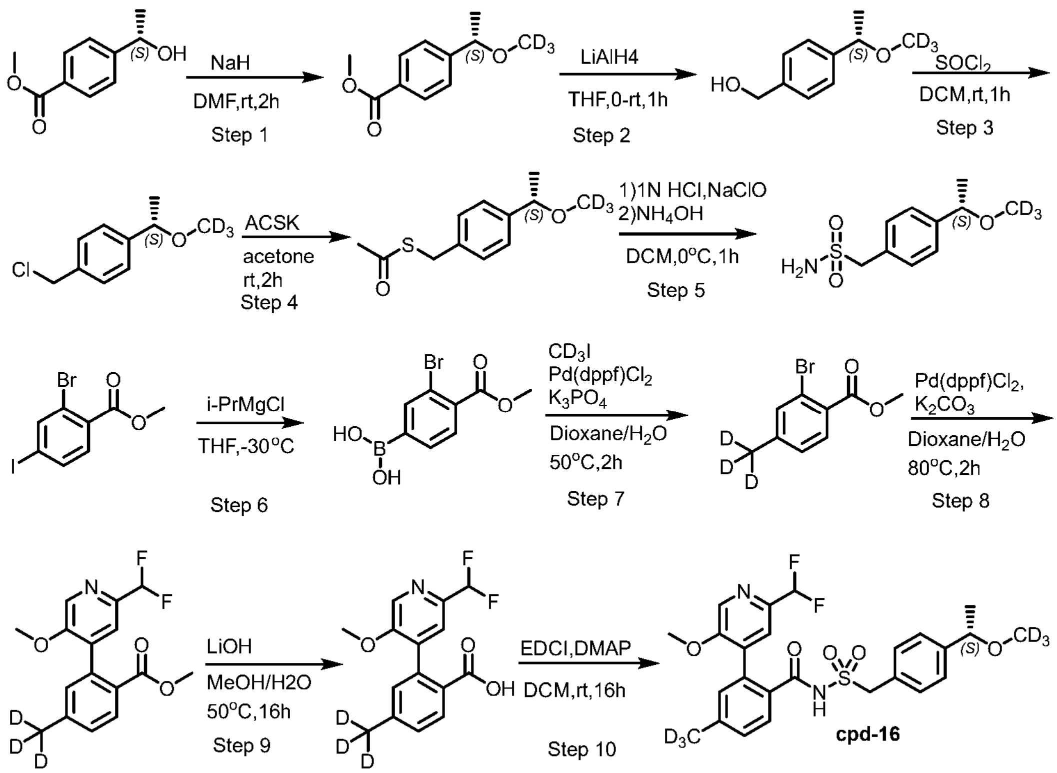


cpd-16

**Step 1: Methyl (S)-4-(1-(methoxy-d3)ethyl)benzoate**

Methyl (S)-4-(1-hydroxyethyl)benzoate (4.0 g, 22.2 mmol) was dissolved in N,N-dimethylformamide (30 mL),

then sodium hydride (0.80 g, 33.3 mmol, 60%) was added to the solution at 0°C. The reaction mixture was stirred at 0°C under a nitrogen atmosphere for 0.5 hours. Deuterated iodomethane (4.83 g, 33.3 mmol) was added to the reaction mixture, which was further stirred under a nitrogen atmosphere at 20°C for 2 hours. The reaction mixture was quenched with saturated aqueous ammonium chloride solution, then extracted with ethyl acetate (100 mL × 3). The combined organic phases were dried over anhydrous sodium sulfate, filtered, and concentrated to obtain a residue. The residue was purified by silica gel column chromatography (ethyl acetate:petroleum ether = 0% to 20%) to obtain methyl (S)-4-(1-(methoxy-d3)ethyl)benzoate (2.7 g, yield: 61.7%).

**Step 2: (S)-(4-(1-(Methoxy-d3)ethyl)phenyl)methanol**

(S)-4-(1-(Methoxy-d3)ethyl)benzoate (2.7 g, 13.7 mmol) was dissolved in tetrahydrofuran (30 mL), followed by addition of lithium aluminum hydride (11 mL, 27.5 mmol, 2.5 mol/L) at 0 °C. The reaction mixture was stirred under a nitrogen atmosphere at 0°C for 1 hour. After the reaction was completed, decahydrate sodium sulfate was slowly added to the reaction mixture to quench the reaction. The mixture was filtered. The filter cake was washed 3 times with ethyl acetate (100 mL), and the combined organic phases were dried over anhydrous sodium sulfate, filtered, and concentrated to obtain a residue. The residue was purified by silica gel column chromatography (ethyl acetate:petroleum ether = 0% to 30%) to obtain (S)-(4-(1-(Methoxy-d3)ethyl)phenyl)methanol (2.0 g, yield: 86.3%).

**Step 3: (S)-1-(chloromethyl)-4-(1-(methoxy-d3)ethyl)benzene**

(S)-(4-(1-(Methoxy-d3)ethyl)phenyl)methanol **(2.0 g, 11.8 mmol)** was dissolved in dichloromethane (20 mL), and thionyl chloride **(2.57 mL, 35.5 mmol)** was added to the reaction mixture. The reaction mixture was stirred at room temperature for 1 hour, quenched with a saturated sodium carbonate solution, and extracted with dichloromethane (50 mL × 2). The combined organic phases were dried over anhydrous sodium sulfate, filtered, and concentrated to obtain the crude product (S)-1-(chloromethyl)-4-(1-(methoxy-d3)ethyl)benzene (2.1 g, crude).

**Step 4: (S)-S-(4-(1-(Methoxy-d3)ethyl)benzyl) ethanethioate**

(S)-1-(Chloromethyl)-4-(1-(methoxy-d3)ethyl)benzene **(2.1 g, 11.2 mmol)** was dissolved in acetone (20 mL), followed by addition of potassium thioacetate (1.92 g, 16.8 mmol). The reaction mixture was stirred at room temperature for 2 hours. After the reaction was completed, the mixture was filtered, and the filtrate was concentrated to obtain a residue. The residue was purified by silica gel column chromatography (ethyl acetate:petroleum ether = 0% to 20%) to obtain (S)-S-(4-(1-(Methoxy-d3)ethyl)benzyl) ethanethioate (2.0 g, yield: 78.6%).

**Step 5: (S)-(4-(1-(Methoxy-d3)ethyl)phenyl)methanesulfonamide**

(S)-S-(4-(1-(Methoxy-d3)ethyl)benzyl) ethanethioate (2.0 g, 8.8 mmol) was dissolved in dichloromethane (20 mL). 1 N dilute hydrochloric acid (35.2 mL, 35.2 mmol) and then a 10% aqueous sodium hypochlorite solution (26.2 g, 35.2 mmol) were added to the reaction mixture. After stirring for 1 hour, the reaction mixture was added with concentrated aqueous ammonia to adjust the pH to 12, and stirred for an additional 0.5 hours. The reaction mixture was diluted with water (50 mL) and extracted with ethyl acetate (50.00 mL x 3). The combined organic phases were dried over anhydrous sodium sulfate, filtered, and concentrated to obtain a residue. The residue was purified by silica gel column chromatography (methanol:dichloromethane = 0% to 10%) to obtain (S)-(4-(1-(methoxy-d3)ethyl)phenyl)methanesulfonamide (1.0 g, yield: 54.2%). LCMS calc. for $C_{10}H_{11}D_3NO_3S$ $[M-H]^-$: m/z = 231.1; Found: 231.2. $^1$HNMR (400 MHz, DMSO) δ 7.31 (d, J = 8.1 Hz, 2H), 7.26 (d, J = 8.1 Hz, 2H), 6.82 (s, 2H), 4.28 (q, J = 6.4 Hz, 1H), 4.21 (s, 2H), 1.29 (d, J = 6.4 Hz, 3H).

**Step 6: 3-Bromo-4-(methoxycarbonyl)phenylboronic acid**

Methyl 2-bromo-4-iodobenzoate (11.0 g, 32.3 mmol) was dissolved in tetrahydrofuran (100 mL). To the reaction mixture was slowly added a complex of isopropylmagnesium chloride and lithium chloride (27.3 mL, 1.30 mol/L, 35.5 mmol) at -30°C. The reaction mixture was stirred at this temperature for 0.5 hours, and then trimethyl borate (6.71 g, 64.5 mmol) was added to the reaction mixture. The reaction mixture was stirred at -30°C for 2 hours. After the reaction was completed, the reaction mixture was added with 1N dilute hydrochloric acid (20 mL), subsequently stirred for 10 minutes and then extracted with ethyl acetate (200 mL x 2). The combined organic phases were dried over anhydrous sodium sulfate, filtered, and concentrated to obtain a crude product. The crude product was slurried with dichloromethane (20 mL) and filtered to obtain crude 3-bromo-4-(methoxycarbonyl)phenylboronic acid (8.0 g, yield: 95.8%).

**Step 7: Methyl 2-bromo-4-(methyl-d3)benzoate**

3-Bromo-4-(methoxycarbonyl)phenylboronic acid (8.0 g, 30.9 mmol) was dissolved in dioxane (80 mL) and water (8.0 mL), followed by addition of deuterated iodomethane (6.72 g, 46.6 mmol), 1,1'-bis(diphenylphosphino)ferrocene palladium dichloride (2.26 g, 3.09 mmol), and potassium phosphate (16.4 g, 77.3 mmol). The reaction mixture was heated to 50°C and stirred under an argon atmosphere for 2 hours. After the reaction was completed, the reaction mixture was quenched with water, then extracted with ethyl acetate (100 mL × 2). The combined organic phases were dried over anhydrous sodium sulfate, filtered, and concentrated to obtain a residue. The residue was purified by silica gel column chromatography (ethyl acetate:petroleum ether = 0% to 20%) to obtain methyl 2-bromo-4-(methyl-d3)benzoate (3.0 g, yield: 41.8%).

**Step 8: Methyl 2-(2-(difluoromethyl)-5-methoxypyridin-4-yl)-4-(methyl-d3)benzoate**

Methyl 2-bromo-4-(methyl-d3)benzoate (3.0 g, 13.0 mmol) was dissolved in dioxane (30 mL) and water (5.0 mL). To the reaction mixture were added 2-(difluoromethyl)-5-methoxy-4-(4,4,5,5-tetramethyl-1,3,2-dioxaborolan-2-yl)pyridine (3.7 g, 13.0 mmol), potassium carbonate (4.76 g, 34.5 mmol), and 1,1'-bis(diphenylphosphino)ferrocene palladium dichloride (1.01 g, 1.3 mmol). The reaction mixture was heated to 80°C and stirred for 2 hours under an argon atmosphere. After the reaction was completed, the reaction mixture was quenched with water, then extracted with ethyl acetate (100 mL × 2). The combined organic phases were dried over anhydrous sodium sulfate, filtered, and concentrated to obtain a residue. The residue was purified by silica gel column chromatography (ethyl acetate:petroleum ether = 0% to 30%) to obtain methyl 2-(2-(difluoromethyl)-5-methoxypyridin-4-yl)-4-(methyl-d3)benzoate (4.0 g, yield : 93.5%).

**Step 9: 2-(2-(Difluoromethyl)-5-methoxypyridin-4-yl)-4-(methyl-d3)benzoic acid**

Methyl 2-(2-(difluoromethyl)-5-methoxypyridin-4-yl)-4-(methyl-d3)benzoate (4.0 g, 12.9 mmol) was dissolved in methanol (30 mL) and water (5.0 mL). Lithium hydroxide (0.93 g, 38.7 mmol) was added to the reaction mixture, which was then heated to 50°C and stirred for 16 hours. After the reaction was completed, the reaction mixture was added with 1N dilute hydrochloric acid to adjust the pH to 5, and extracted with ethyl acetate (100 mL x3). The combined organic phases were dried over anhydrous sodium sulfate, filtered, and concentrated to obtain a residue. The residue was purified by silica gel column chromatography (methanol:dichloromethane = 0% to 10%) to obtain 2-(2-(Difluoromethyl)-5-methoxypyridin-4-yl)-4-(methyl-d3)benzoic acid (3.0 g, yield: 78.6%). LCMS calc. for $C_{15}H_9D_3F_2NO_3$ $[M-H]^-$: m/z = 295.1; Found:295.1. $^1$HNMR (400 MHz, DMSO) δ 12.65 (s, 1H), 8.43 (s, 1H), 7.79 (d, J = 7.9 Hz, 1H), 7.49 (s, 1H), 7.34 (dd, J = 7.9, 1.7 Hz, 1H), 7.15 (d, J = 1.6 Hz, 1H), 6.94 (t,J = 55.1 Hz, 1H), 3.83 (s, 3H).

**Step 10: (S)-2-(2-(Difluoromethyl)-5-methoxypyridin-4-yl)-N-((4-(1-(methoxy-d3)ethyl)benzyl)sulfonyl)-4-(methyl-d3)benzamide**

2-(2-(Difluoromethyl)-5-methoxypyridin-4-yl)-4-(methyl-d3)benzoic acid (100 mg, 0.34 mmol) was dissolved in dichloromethane (4.0 mL), followed by addition of (S)-(4-(1-(methoxy-d3)ethyl)phenyl)methanesulfonamide (94.1 mg, 0.41 mmol), 1-ethyl-(3-dimethylaminopropyl)carbodiimide hydrochloride (78.7 mg, 0.41 mmol), and 4-dimethylaminopyridine (83.6 mg, 0.68 mmol). The reaction mixture was stirred at room temperature for 16 hours. After the reaction was completed, the reaction mixture was concentrated under reduced pressure. The residue was purified by silica gel column chromatography (dichloromethane:methanol = 0% to 10%) and preparative high-performance liquid chromatography (Column: XBridge Prep C18 5μm OBD 19*150mm, Mobile phase: A (10 mM $NH_4HCO_3$ in water) B (ACN), 0-3min: 40%B, 3-7min: 40-85%B, 7-11min: 85-90%B, 11-13min: 90-95%B, 13-16min: 95%B, 16-16.5min: 95-40%B, 16.5-21min: 40%B, Flow =15mL/min) to obtain (S)-2-(2-(difluoromethyl)-5-methoxypyridin-4-yl)-N-((4-(1-(methoxy-d3)ethyl)benzyl)sulfonyl)-4-(methyl-d3)benzamide (40.0 mg, yield: 23.2%). LCMS calc. for $C_{25}H_{21}D_6F_2N_2O_5S$ $[M+H]^+$: m/z = 511.2; Found: 511.3. 1H NMR (400 MHz, DMSO-d6) δ 11.92 (s, 1H), 8.45 (s, 1H), 7.56 (s, 1H), 7.37-7.22 (m, 7H), 6.96 (t, J = 55.1 Hz, 1H), 4.66 (s, 2H), 4.33 (q, J = 6.4 Hz, 1H), 3.86 (s, 3H), 1.32 (d, J = 6.4 Hz, 3H).

**Example 17: Preparation of compound cpd-17**

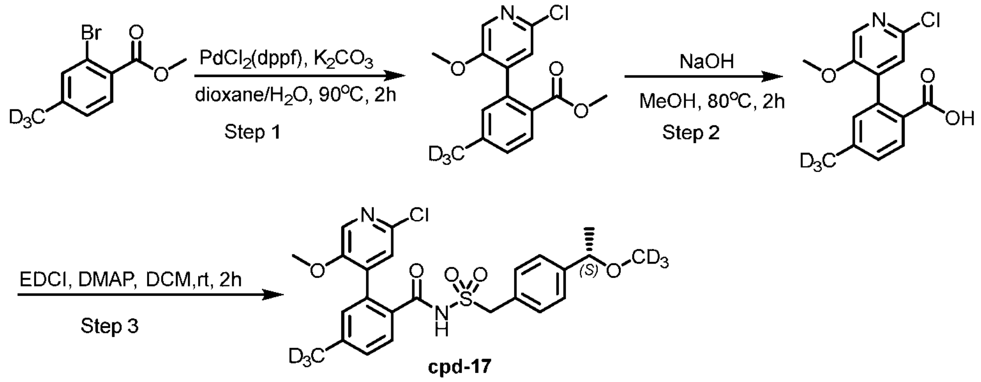


**cpd-17**

**Step 1: Methyl 2-(2-chloro-5-methoxypyridin-4-yl)-4-(methyl-d3)benzoate**

Methyl 2-bromo-4-(methyl-d3)benzoate (250 mg, 1.07 mmol) was dissolved in 1,4-dioxane (5 mL) and water (1 mL), followed by addition of (2-chloro-5-methoxypyridin-4-yl)boronic acid (202 mg, 1.07 mmol), potassium carbonate (447 mg, 3.23 mmol), and 1,1'-bis(diphenylphosphino)ferrocene palladium dichloride (78.8 mg, 0.11 mmol). The mixture was reacted at 90°C for 2 hours. Water (20 mL) was added, and the mixture was extracted three times with ethyl acetate (20 mL). The combined organic phases were sequentially washed with water (100 mL) and saturated brine (100 mL), dried over anhydrous sodium sulfate, filtered, and concentrated to obtain methyl 2-(2-chloro-5-methoxypyridin-4-yl)-4-(methyl-d3)benzoate (300 mg, crude). LCMS calc. for $C_{15}H_{12}D_3ClNO_3$ $[M+H]^+$: m/z =295.1; Found: 295.1.

**Step 2:2-(2-Chloro-5-methoxypyridin-4-yl)-4-(methyl-d3)benzoic acid**

To a solution of methyl 2-(2-chloro-5-methoxypyridin-4-yl)-4-(methyl-d3)benzoate (300 mg, 1.02 mmol) in methanol (10 mL) was added sodium hydroxide (201 mg, 5.1 mmol). The mixture was then reacted at 80°C for 2 hours. After the reaction was completed, the mixture was concentrated under reduced pressure, and subsequently added with water (20 mL). The resulting mixture was then extracted three times with a mixture of ethyl acetate and petroleum ether (20 mL, 1:1). The aqueous phase was added with 1N dilute hydrochloric acid (20 mL) to adjust the pH to 5, and extracted three times with ethyl acetate (50 mL). The combined organic phases were washed with water (50 mL) and saturated brine (50 mL), dried over anhydrous sodium sulfate, filtered, and concentrated to obtain 2-(2-chloro-5-methoxypyridin-4-yl)-4-(methyl-d3)benzoic acid (210 mg, yield: 76.3%). LCMS calc. for $C_{14}H_{10}D_3ClNO_3$ $[M+H]^+$: m/z =281.1; Found: 281.1.

**Step 3: (S)-2-(2-Chloro-5-methoxypyridin-4-yl)-N-((4-(1-(methoxy-d3)ethyl)benzyl)sulfonyl)-4-(methyl-d3)benzamide**

2-(2-Chloro-5-methoxypyridin-4-yl)-4-(methyl-d3)benzoic acid (65 mg, 0.23 mmol) and (S)-(4-(1-(methoxy-d3) ethyl)phenyl)methanesulfonamide (53.8 mg, 0.23 mmol) were dissolved in dichloromethane (4 mL), followed by addition of 1-ethyl-(3-dimethylaminopropyl)carbodiimide hydrochloride (66.6 mg, 0.35 mmol) and 4-dimethylaminopyridine (42.4 mg, 0.35 mmol). The mixture was reacted at 25°C for 2 hours. The reaction mixture was concentrated under reduced pressure and purified by preparative high-performance liquid chromatography (Gemini-C18 150 x 21.2 mm, 5 μm, Mobile phase: A (0.1% FA in Water) B (ACN), 0-10 min: 40% B, 10-10.5 min: 40-95% B, 10.5-12.5 min: 95-90% B, 12.5-13 min: 95-10% B, 13-15 min: 40% B, Flow = 20 mL/min) to obtain (S)-2-(2-Chloro-5-methoxypyridin-4-yl)-N-((4-(1-(methoxy-d3) ethyl)benzyl)sulfonyl)-4-(methyl-d3)benzamide (21.5 mg, yield: 18.6%). LCMS calc. for $C_{24}H_{20}D_6ClN_2O_5S$ $[M+H]^+$: m/z = 495.2; Found: 495.3. $^1H$ NMR (400 MHz, ) δ 11.87 (s, 1H), 8.12 (s, 1H), 7.34 (s, 1H), 7.30 (t, J = 5.7 Hz, 2H), 7.27 (d, J = 1.7 Hz, 2H), 7.24 (d, J = 8.2 Hz, 2H), 7.21 (d, J = 1.5 Hz, 1H), 4.64 (s, 2H), 4.29 (q, J = 6.4 Hz, 1H), 3.75 (s, 3H), 1.28 (d, J = 6.4 Hz, 3H).

**Example 18: Preparation of compound cpd-18**

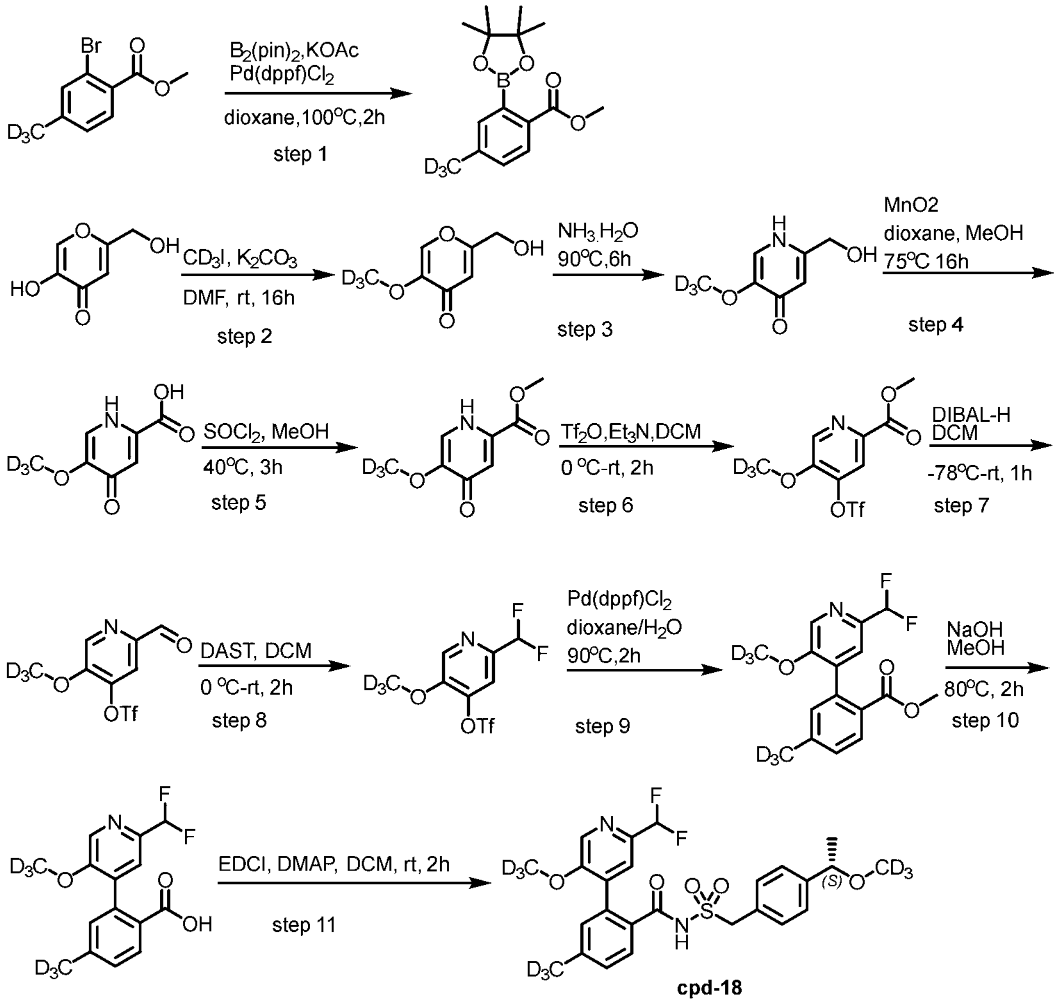


**cpd-18**

**Step 1: Methyl 2-(4,5-dimethyl-1,3,2-dioxaborolan-2-yl)-4-(methyl-d3)benzoate**

Methyl 2-bromo-4-(methyl-d3)benzoate (1 g, 4.32 mmol) was dissolved in 1,4-dioxane (10 mL), followed by addition of bis(pinacolato)diboron (2 g, 8.64 mmol), potassium acetate (1.50 g, 13.0 mmol), and 1,1'-bis(diphenylphosphino)ferrocene palladium dichloride (158 mg, 0.22 mmol). The mixture was reacted at 100°C for 2 hours. Water (30 mL) was added, and the mixture was extracted three times with ethyl acetate (30 mL). The combined organic phases were washed sequentially with water (20 mL) and saturated brine (30 mL), dried over anhydrous sodium sulfate, filtered, and concentrated to obtain a residue. The residue was purified by silica gel column chromatography (petroleum ether:ethyl acetate = 0% to 25%) to obtain methyl 2-(4,5-dimethyl-1,3,2-dioxaborolan-2-yl)-4-(methyl-d3)benzoate (1 g, yield: 82.8%). LCMS calc. for $C_{15}H_{19}D_3BO_4$ $[M+H]^+$: m/z =280.2; Found: 280.3.

**Step 2: 2-(hydroxymethyl)-5-(methoxy-d3)-4H-pyran-4-one**

5-Hydroxy-2-(hydroxymethyl)-4H-pyran-4-one (5.0 g, 35.2 mmol), deuterated iodomethane (6.0 g, 42.2 mmol), and potassium carbonate (15.0 g, 106 mmol) were dissolved in N,N-dimethylformamide (10 mL). The mixture was reacted at 25°C for 16 hours. After the reaction was completed, the mixture was filtered. The filtrate was concentrated under reduced pressure to obtain 2-(hydroxymethyl)-5-(methoxy-d3)-4H-pyran-4-one (3.0 g, crude). LCMS calc. for $C_7H_6D_3O_4$ $[M+H]^+$: m/z =160.1; Found: 160.1.

**Step 3: 2-(Hydroxymethyl)-5-(methoxy-d3)pyridin-4(1H)-one**

2-(hydroxymethyl)-5-(methoxy-d3)-4H-pyran-4-one (3.0 g, 18.9 mmol) was dissolved in an aqueous ammonia solution (30 mL). The mixture was reacted at 90°C for 6 hours. After the reaction was completed, the mixture was further concentrated under reduced pressure to obtain 2-(hydroxymethyl)-5-(methoxy-d3)pyridin-4(1H)-one (2.0 g, yield: 60%). LCMS calc. for $C_7H_7D_3NO_3$ $[M+H]^+$: m/z =159.1; Found: 159.1.

**Step 4: 5-(Methoxy-d3)-4-oxo-1,4-dihydropyridine-2-carboxylic acid**

2-(Hydroxymethyl)-5-(methoxy-d3)pyridin-4(1H)-one (2.0 g, 12.7 mmol) was dissolved in methanol and 1,4-dioxane (20 mL), followed by addition of manganese dioxide (11 g, 127 mmol). The mixture was reacted at 75°C for 16 hours. After the reaction was completed, the mixture was concentrated under reduced pressure. The resulting filtrate was concentrated to obtain5-(methoxy-d3)-4-oxo-1,4-dihydropyridine-2-carboxylic acid(2.0 g, 73%). LCMS calc. for $C_7H_5D_3NO_4$ $[M+H]^+$: m/z =173.1; Found: 173.1.

**Step 5: Methyl 5-(methoxy-d3)-4-oxo-1,4-dihydropyridine-2-carboxylate**

5-(Methoxy-d3)-4-oxo-1,4-dihydropyridine-2-carboxylic acid (2 g, 11.6 mmol) was dissolved in methanol (20 mL), followed by addition of thionyl chloride (1.66 g, 14.0 mmol). The mixture was reacted at 40°C for 3 hours. The reaction mixture was concentrated to obtain methyl 5-(methoxy-d3)-4-oxo-1,4-dihydropyridine-2-carboxylate (3.0 g, crude). LCMS calc. for $C_8H_7D_3NO_4$ $[M+H]^+$: m/z =187.1; Found: 187.0.

**Step 6: Methyl 5-(methoxy-d3)-4-(((trifluoromethyl)sulfonyl)oxy)picolinate**

Under a nitrogen atmosphere at 0°C, methyl 5-(methoxy-d3)-4-oxo-1,4-dihydropyridine-2-carboxylate (3.0 g, 16.0 mmol) and triethylamine (5.0 mL, 32.1 mmol) were dissolved in dichloromethane (30 mL), followed by addition of trifluoromethanesulfonic anhydride (4.0 mL, 19.3 mmol). The mixture was reacted under a nitrogen atmosphere at 25°C for 2 hours. The reaction mixture was diluted with water (50 mL) and extracted three times with dichloromethane (50 mL). The combined organic phases were washed with water (50 mL) and saturated brine (50 mL), dried over anhydrous sodium sulfate, filtered, and concentrated to obtain a residue. The residue was purified by silica gel column chromatography (ethyl acetate:petroleum ether = 0% to 10%) to obtain methyl 5-(methoxy-d3)-4-(((trifluoromethyl)sulfonyl)oxy)picolinate (270 mg, yield: 5.3%). LCMS calc. for $C_9H_6D_3F_3NO_6S$ $[M+H]^+$: m/z =319.0; Found: 319.0.

**Step 7: 2-Formyl-5-(methoxy-d3)pyridin-4-yl trifluoromethanesulfonate**

To a solution of methyl 5-(methoxy-d3)-4-(((trifluoromethyl)sulfonyl)oxy)picolinate in dichloromethane (5 mL) (270 mg, 0.85 mmol) under a nitrogen atmosphere at -78°C was slowly added dropwise diisobutylaluminum hydride (1 mL, 1.0 mmol, 1 mol/L). The mixture was reacted at 25°C for 1 hour. The reaction mixture was cooled to -78°C, quenched with a saturated aqueous ammonium chloride solution (10 mL), and warmed to room temperature. The aqueous phase was extracted three times with dichloromethane (20 mL). The combined organic phases were washed with water (20 mL) and saturated brine (20 mL), dried over anhydrous sodium sulfate, filtered, and concentrated to obtain a residue. The residue was purified by silica gel column chromatography (ethyl acetate:petroleum ether = 20% to 50%) to obtain 2-formyl-5-(methoxy-d3)pyridin-4-yl trifluoromethanesulfonate (80 mg, yield: 26.3%). LCMS calc. for $C_8H_4D_3F_3NO_5S$ $[M+H]^+$: m/z =289.0; Found: 289.0.

**Step 8: 2-(Difluoromethyl)-5-(methoxy-d3)pyridin-4-yl trifluoromethanesulfonate**

To a solution of 2-formyl-5-(methoxy-d3)pyridin-4-yl trifluoromethanesulfonate (80 mg, 0.27 mmol) in dichloromethane (4 mL) under nitrogen atmosphere at 0°C was slowly added dropwise diethylaminosulfur trifluoride (130 mg, 0.81 mmol). The mixture was reacted at 25°C for 2 hours. The reaction mixture was diluted with water (20 mL). The aqueous phase was extracted three times with dichloromethane (20 mL). The combined organic phases were washed with water (20 mL) and saturated brine (10 mL), dried over anhydrous sodium sulfate, filtered, and concentrated to obtain a residue. The residue was purified by silica gel column chromatography (ethyl acetate:petroleum ether = 0% to 30%) to obtain 2-(difluoromethyl)-5-(methoxy-d3)pyridin-4-yl trifluoromethanesulfonate (50 mg, yield: 53.7%). LCMS calc. for $C_8H_4D_3F_5NO_4S$ $[M+H]^+$: m/z =311.0; Found: 311.0.

**Step 9: Methyl 2-(2-(difluoromethyl)-5-(methoxy-d3)pyridin-4-yl)-4-(methyl-d3)benzoate**

2-(Difluoromethyl)-5-(methoxy-d3)pyridin-4-yl trifluoromethanesulfonate (50 mg, 0.16 mmol) was dissolved in 1,4-dioxane (5.0 mL) and water (0.5 mL), followed by addition of methyl 2-(4,5-dimethyl-1,3,2-dioxaborolan-2-yl)-4-(methyl-d3)benzoate (61 mg, 0.24 mmol), potassium carbonate (66 mg, 0.48 mmol), and 1,1'-bis(diphenylphosphino)ferrocene palladium dichloride (12 mg, 0.016 mmol). The mixture was reacted at 90°C for 2 hours. Water (20 mL) was added, and the mixture was extracted three times with ethyl acetate (20 mL). The combined organic phases were washed sequentially with water (20 mL) and saturated brine (20 mL), dried over anhydrous sodium sulfate, filtered, and concentrated to obtain a residue. The residue was purified by silica gel column chromatography (petroleum ether:ethyl

acetate = 0% to 10%) to obtain methyl 2-(2-(difluoromethyl)-5-(methoxy-d3)pyridin-4-yl)-4-(methyl-d3)benzoate (45 mg, yield: 80.8%). LCMS calc. for $C_{16}H_{10}D_6F_2NO_3$ $[M+H]^+$: m/z =314.1; Found: 314.1.

**Step 10: 2-(2-(Difluoromethyl)-5-(methoxy-d3)pyridin-4-yl)-4-(methyl-d3)benzoic acid**

methyl 2-(2-(difluoromethyl)-5-(methoxy-d3)pyridin-4-yl)-4-(methyl-d3)benzoate (45 mg, 0.14 mmol) was dissolved in methanol (3.0 mL), and then sodium hydroxide (17 mg, 0.42 mmol) was added. The mixture was reacted at 80°C for 2 hours. After the reaction was completed, the mixture was concentrated under reduced pressure, and subsequently added with water (20 mL). The resulting mixture was then extracted three times with ethyl acetate (20 mL). The aqueous phase was added with 2N dilute hydrochloric acid (20 mL) to adjust the pH to 6, and extracted three times with ethyl acetate (20 mL). The combined organic phases were washed with water (10 mL) and saturated brine (10 mL), dried over anhydrous sodium sulfate, filtered, and concentrated and purified to obtain 2-(2-(difluoromethyl)-5-(methoxy-d3)pyridin-4-yl)-4-(methyl-d3)benzoic acid (40 mg, yield: 95.5%). LCMS calc. for $C_{15}H_8D_6F_2NO_3$ $[M+H]^+$: m/z = 300.1; Found: 300.1.

**Step 11: (S)-2-(2-(Difluoromethyl)-5-(methoxy-d3)pyridin-4-yl)-N-((4-(1-(methoxy-d3)ethyl)benzyl)sulfonyl)-4-(methyl-d3)benzamide**

2-(2-Chloro-5-methoxypyridin-4-yl)-4-(methyl-d3)benzoic acid (40 mg, 0.13 mmol) and (S)-(4-(1-(methoxy-d3) ethyl)phenyl)methanesulfonamide (37 mg, 0.16 mmol) were dissolved in dichloromethane (4 mL), followed by addition of 1-ethyl-(3-dimethylaminopropyl)carbodiimide hydrochloride (31 mg, 0.16 mmol) and 4-dimethylaminopyridine (20 mg, 0.16 mmol). The mixture was reacted at 25°C for 2 hours. The reaction mixture was concentrated under reduced pressure and purified by preparative high-performance liquid chromatography (Gemini-C18 150 x 21.2 mm, 5 μm, Mobile phase: A (0.1% FA in Water) B (ACN), 0-10 min: 50% B, 10-10.5 min: 50-95% B, 10.5-12.5 min: 95-95% B, 12.5-13 min: 95-50% B, 13-15 min: 50% B, Flow = 20 mL/min) to obtain (S)-2-(2-(difluoromethyl)-5-(methoxy-d3)pyridin-4-yl)-N-((4-(1-(methoxy-d3)ethyl)benzyl)sulfonyl)-4-(methyl-d3)benzamide (22 mg, yield: 32.9%). LCMS calc. for $C_{25}H_{18}D_9F_2N_2O_5S$ $[M+H]^+$: m/z = 514.2; Found: 514.2. $^1$HNMR (400 MHz, DMSO) δ = 11.92 (s, 1H), 8.44 (s, 1H), 7.56 (s, 1H), 7.36-7.25 (m, 6H), 7.23 (s, 1H), 7.10-6.83 (m, 1H), 4.66 (s, 2H), 4.33 (q, J=6.4, 1H),1.32 (d, J=6.4, 3H).

**Example 19: Preparation of compound cpd-19**

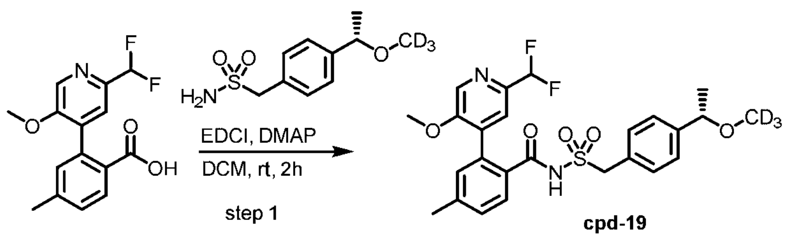


cpd-19

**Step 1: (S)-2-(2-(Difluoromethyl)-5-methoxypyridin-4-yl)-N-((4-(1-(methoxy-d3)ethyl)benzyl)sulfonyl)-4-methylbenzamide**

2-(2-(Difluoromethyl)-5-methoxypyridin-4-yl)-4-methylbenzoic acid (100 mg, 0.34 mmol) and (S)-(4-(1-(methoxy-d3)ethyl)phenyl)methanesulfonamide (79 mg, 0.34 mmol) were dissolved in dichloromethane (5.0 mL), followed by addition of 1-ethyl-(3-dimethylaminopropyl)carbodiimide hydrochloride (78 mg, 0.41 mmol) and 4-dimethylaminopyridine (50 mg, 0.41 mmol). The mixture was reacted at 25°C for 2 hours. The reaction mixture was concentrated under reduced pressure, dissolved in ethyl acetate (30 mL), and sequentially washed with 1N dilute hydrochloric acid (5 mL) and saturated brine (10 Ml). The organic phase was dried and concentrated. The resulting residue was purified by preparative high-performance liquid chromatography (Gemini-C18 150 x 21.2 mm, 5 μm, Mobile phase: A (0.1% FA in Water) B (ACN), 0-10 min: 49% B, 10-10.5 min: 49-95% B, 10.5-12.5 min: 95-95% B, 12.5-13 min: 95-49% B, 13-15 min: 49% B, Flow = 20 mL/min) to obtain (S)-2-(2-(difluoromethyl)-5-methoxypyridin-4-yl)-N-((4-(1-(methoxy-d3)ethyl)benzyl)sulfonyl)-4-methylbenzamide (46 mg, yield: 26.2%). LCMS calc. for $C_{25}H_{24}D_3F_2N_2O_3S$ $[M+H]^+$: m/z = 508.2; Found: 508.2. $^1$HNMR (400 MHz, DMSO) δ 11.93 (s, 1H), 8.45 (s, 1H), 7.56 (s, 1H), 7.36-7.26 (m, 6H), 7.24 (s, 1H), 6.97 (t, J = 55.1 Hz, 1H), 4.67 (s, 2H), 4.33 (q, J = 6.4Hz, 1H), 3.86 (s, 3H), 2.39 (s, 3H), 1.32 (d, J = 6.4 Hz, 3H).

**Example 20: Preparation of compound cpd-20**

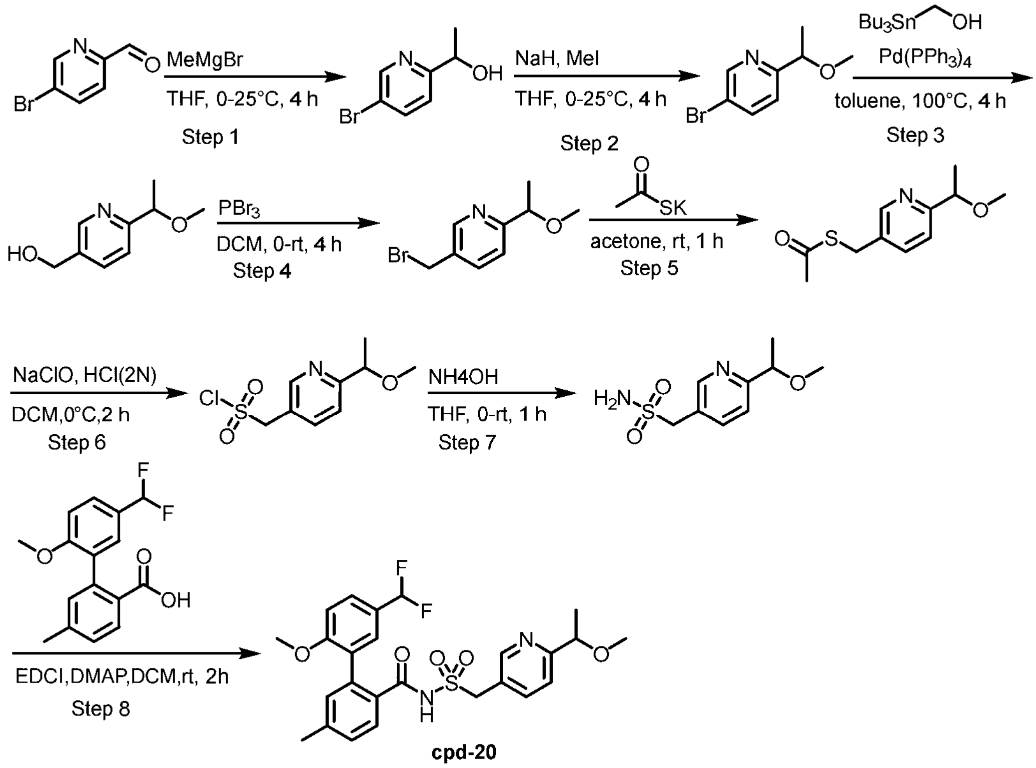


**cpd-20**

**Step 1: 1-(5-Bromopyridin-2-yl)ethan-1-ol**

To a solution of 5-bromopicolinaldehyde (3.5 g, 18.8 mmol) in tetrahydrofuran (40 mL) under a nitrogen atmosphere at 0°C was slowly added methylmagnesium bromide (28 mL, 28.2 mmol). The mixture was reacted at 25°C for 4 hours. The mixture was added with a saturated aqueous solution of ammonium chloride (20 mL) and extracted three times with dichloromethane (50 mL). The combined organic phases were sequentially washed with water (30 mL) and saturated brine (30 mL), dried over anhydrous sodium sulfate, filtered, and concentrated to obtain 1-(5-bromopyridin-2-yl)ethan-1-ol (3.0 g, yield: 71.2%). LCMS calc. for $C_7H_9BrNO$ $[M+H]^+$: m/z =202.0/204.0; Found: 202.0/204.0.

**Step 2: 5-Bromo-2-(1-methoxyethyl)pyridine**

Under a nitrogen atmosphere at 0°C, 1-(5-bromopyridin-2-yl)ethan-1-ol (3.0 g, 14.8 mmol) was dissolved in tetrahydrofuran (30 mL), followed by addition of sodium hydride (1.0 g, 22.2 mmol, 60%). The mixture was reacted at 0°C for 30 minutes. Iodomethane (3 g, 22.20 mmol) was added to the reaction mixture, which was reacted at 25°C for 4 hours. The mixture was added with a saturated aqueous ammonium chloride solution (30 mL) and extracted three times with ethyl acetate (50 mL). The combined organic phases were successively washed with water (30 mL) and saturated brine (30 mL), dried over anhydrous sodium sulfate, filtered, and concentrated to obtain a residue. The residue was purified by silica gel column chromatography (ethyl acetate:petroleum ether = 0% to 10%) to obtain 5-bromo-2-(1-methoxyethyl)pyridine (2.0 g, yield: 56.6%). LCMS calc. for $C_8H_{11}BrNO$ $[M+H]^+$: m/z =216.0/218.0; Found: 216.1/218.1.

**Step 3: (6-(1-Methoxyethyl)pyridin-3-yl)methanol**

5-Bromo-2-(1-methoxyethyl)pyridine (2.0 g, 9.3 mmol) and (tributylstannyl)methanol (3.6 g, 11.2 mmol) were dissolved in anhydrous toluene (20 mL), followed by addition of tetrakis(triphenylphosphine)palladium (537 mg, 0.46 mmol). The mixture was reacted at 100°C for 4 hours. Water (30 mL) was added, and the mixture was extracted three times with ethyl acetate (30 mL). The combined organic phases were sequentially washed with water (30 mL) and saturated brine (30 mL), dried over anhydrous sodium sulfate, filtered, and concentrated. The residue was purified by silica gel column chromatography (ethyl acetate:petroleum ether = 20%-50%) to obtain (6-(1-methoxyethyl)pyridin-3-yl)methanol

(800 mg, yield: 46.6%). LCMS calc. for $C_9H_{14}NO_2$ $[M+H]^+$: m/z =168.1; Found: 168.2.

**Step 4: 5-(Bromomethyl)-2-(1-methoxyethyl)pyridine**

Under a nitrogen atmosphere at 0°C, (6-(1-methoxyethyl)pyridin-3-yl)methanol (800 mg, 4.78 mmol) was dissolved in dichloromethane (10 mL), followed by addition of phosphorus tribromide (2.0 g, 7.18 mmol). The mixture was reacted at 25°C for 4 hours. The reaction mixture was poured into a sodium bicarbonate solution (20 mL) and extracted three times with dichloromethane (30 mL). The combined organic phases were sequentially washed with water (30 mL) and saturated brine (30 mL), dried over anhydrous sodium sulfate, filtered, and concentrated to obtain 5-(bromomethyl)-2-(1-methoxyethyl)pyridine(850 mg, yield: 69.5%). LCMS calc. for $C_9H_{13}BrNO$ $[M+H]^+$: m/z =230.0/232.0; Found: 230.0/232.0.

**Step 5: S-((6-(1-Methoxyethyl)pyridin-3-yl)methyl) ethanethioate**

5-(Bromomethyl)-2-(1-methoxyethyl)pyridine (850 mg, 3.71 mmol) was dissolved in acetone (10 mL), followed by addition of potassium thioacetate (595 mg, 5.22 mmol). The reaction mixture was reacted at 25°C for 1 hour under a nitrogen atmosphere. The reaction mixture was diluted with water (30 mL), and extracted three times with ethyl acetate (30 mL). The combined organic phases were washed with water (50 mL) and saturated brine (50 mL), dried over anhydrous sodium sulfate, filtered, and concentrated to obtain a residue. The residue was purified by silica gel column chromatography (ethyl acetate:petroleum ether = 0% to 20%) to obtain S-((6-(1-methoxyethyl)pyridin-3-yl)methyl) ethanethioate (580 mg, yield: 51.8%). LCMS calc. for $C_{11}H_{16}NO_2S$ $[M+H]^+$: m/z =226.1; Found: 226.1.

**Step 6: (6-(1-Methoxyethyl)pyridin-3-yl)methanesulfonyl chloride**

To a solution of S-((6-(1-methoxyethyl)pyridin-3-yl)methyl) ethanethioate (580 mg, 2.57 mmol) in dichloromethane (10 mL) under a nitrogen atmosphere at 0°C was added 2N dilute hydrochloric acid (1 mL), followed by slow dropwise addition of an aqueous sodium hypochlorite solution (1.40 g, 15.44 mmol). The mixture was reacted for 2 hours. The reaction mixture was diluted with water (20 mL). The aqueous phase was extracted three times with dichloromethane (20 mL). The combined organic phases were washed with water (30 mL) and saturated brine (30 mL), dried over anhydrous sodium sulfate, filtered, and concentrated to obtain (6-(1-methoxyethyl)pyridin-3-yl)methanesulfonyl chloride (360 mg, yield: 50.4%).

**Step 7: (S)-(4-(1-(Methoxy-d3)ethyl)phenyl)methanesulfonamide**

To aqueous ammonia (2 mL) under a nitrogen atmosphere at 0°C was dropwise added a solution of (6-(1-methoxyethyl)pyridin-3-yl)methanesulfonyl chloride (360 mg, 1.44 mmol) in tetrahydrofuran (4.0 mL). The mixture was reacted at 25°C for 1 hour. The reaction mixture was directly concentrated to obtain a residue. The residue was purified by reverse phase column chromatography (acetonitrile: water (0.1% FA) = 0% to 20%) to obtain (S)-(4-(1-(methoxy-d3)ethyl) phenyl)methanesulfonamide (80 mg, yield: 21.6%). LCMS calc. for $C_9H_{15}N_2O_3S$ $[M+H]^+$: m/z = 231.1; Found: 231.0.

**Step 8: 5'-(Difluoromethyl)-2'-methoxy-N-(((6-(1-methoxyethyl)pyridin-3-yl)methyl)sulfonyl)-5-methyl-[1,1'-biphenyl]-2-carboxamide**

(S)-(4-(1-(Methoxy-d3)ethyl)phenyl)methanesulfonamide (80 mg, 0.34 mmol) and 5'-(difluoromethyl)-2'-methoxy-5-methyl-[1,1'-biphenyl]-2-carboxylic acid (79 mg, 0.34 mmol) were dissolved in dichloromethane (3.0 mL), followed by addition of 1-ethyl-(3-dimethylaminopropyl)carbodiimide hydrochloride (80 mg, 0.42 mmol) and 4-dimethylaminopyridine (85 mg, 0.69 mmol). The mixture was reacted at 25°C for 2 hours. The reaction mixture was concentrated under reduced pressure and purified by preparative high-performance liquid chromatography (Gemini-C18 150 x 21.2 mm, 5 μm, Mobile phase: A (0.1% FA in Water) B (ACN), 0-10 min: 40-60% B, 10-10.5 min: 60-95% B, 10.5-12.5 min: 95-95% B, 12.5-13 min: 95-40% B, 13-15 min: 40% B, Flow = 20 mL/min) to obtain 5'-(difluoromethyl)-2'-methoxy-N-(((6-(1-methoxyethyl)pyridin-3-yl)methyl)sulfonyl)-5-methyl-[1,1'-biphenyl]-2-carboxamide (70 mg, yield: 39.2%). LCMS calc. for $C_{25}H_{27}F_2N_2O_5S$ $[M+H]^+$: m/z = 505.2; Found: 505.2. $^1H$ NMR (400 MHz, DMSO) δ 11.88 (s, 1H), 8.39 (s, 1H), 7.73 (d, J = 7.9 Hz, 1H), 7.56 (d, J = 8.3 Hz, 1H), 7.44 (d, J = 7.7 Hz, 2H), 7.26 (dd, J = 22.2, 7.7 Hz, 2H), 7.17-6.89 (m, 1H), 7.14 (dd, J = 16.5, 6.7 Hz, 2H), 4.72 (s, 2H), 4.38 (dd, J = 12.9, 6.4 Hz, 1H), 3.73 (s, 3H), 3.21 (s, 3H), 2.38 (s, 3H), 1.36 (d, J = 6.5 Hz, 3H).

**Example 21: Preparation of compound cpd-21**

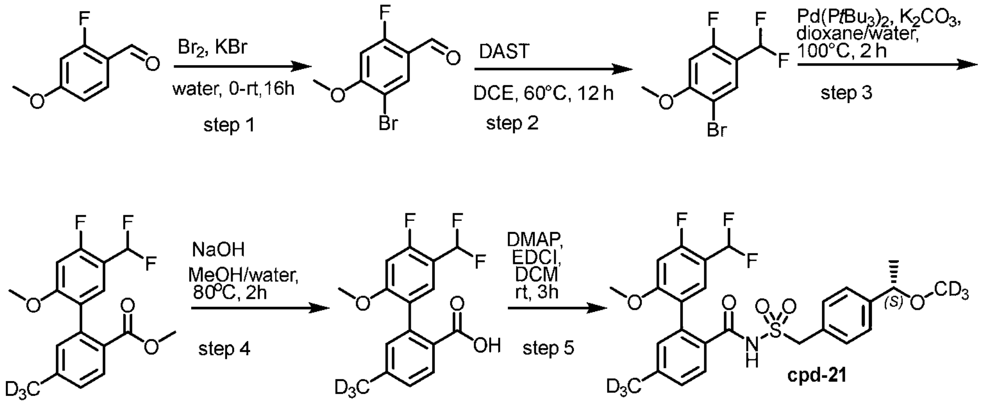


cpd-21

**Step 1: 5-Bromo-2-fluoro-4-methoxybenzaldehyde**

To a solution of 2-fluoro-4-methoxybenzaldehyde (2.0 g, 13.0 mmol) and potassium bromide (8.0 g, 64.9 mmol) in water (30 mL) under a nitrogen atmosphere at 0°C was slowly added dropwise bromine (4.0 g, 26.0 mmol). The mixture was then reacted at 25°C for 16 hours. After the reaction was completed, the reaction was quenched by adding a saturated aqueous solution of sodium thiosulfate (30 mL). The aqueous phase was extracted three times with ethyl acetate (40 mL). The combined organic phases were washed with water (20 mL) and saturated brine (20 mL), dried over anhydrous sodium sulfate, filtered, and concentrated to obtain a residue. The residue was purified by silica gel column chromatography (ethyl acetate:petroleum ether = 0% to 30%) to obtain 5-bromo-2-fluoro-4-methoxybenzaldehyde (2.0 g, 59.5%). LCMS calc. for $C_8H_7BrFO_2$ $[M+H]^+$: m/z =233.0/235.0; Found: 233.0/235.0.

**Step 2: 1-Bromo-5-(difluoromethyl)-4-fluoro-2-methoxybenzene**

5-Bromo-2-fluoro-4-methoxybenzaldehyde (1.0 g, 4.31 mmol) was dissolved in 1,2-dichloroethane (12 mL), followed by slow dropwise addition of diethylaminosulfur trifluoride (2.0 g, 12.9 mmol). The mixture was reacted at 60°C for 12 hours. The reaction mixture was poured into water (30 mL) and extracted three times with dichloromethane (30 mL). The combined organic phases were washed with water (20 mL) and saturated brine (10 mL), dried over anhydrous sodium sulfate, filtered, and concentrated to obtain a residue. The residue was purified by silica gel column chromatography (ethyl acetate:petroleum ether = 0% to 40%) to obtain 1-bromo-5-(difluoromethyl)-4-fluoro-2-methoxybenzene (1.0 g, 68.5%). $^1$HNMR (400 MHz, $CDCl_3$) $\delta$ 7.75 (d, $J$ = 7.5 Hz, 1H), 6.80 (t, $J$ = 56 Hz, 1H), 6.66 (d, $J$ = 2.1 Hz, 1H), 3.92 (s, 3H).

**Step 3: Methyl 5'-(difluoromethyl)-4'-fluoro-2'-methoxy-5-(methyl-d3)-[1,1'-biphenyl]-2-carboxylate**

1-Bromo-5-(difluoromethyl)-4-fluoro-2-methoxybenzene (300 mg, 1.17 mmol) was dissolved in 1,4-dioxane (5 mL) and water (1 mL), followed by addition of methyl 4-(methyl-d3)-2-(4,4,5,5-tetramethyl-1,3,2-dioxaborolan-2-yl) benzoate (394 mg, 1.41 mmol), potassium carbonate (325 mg, 2.35 mmol), and bis(tri-tert-butylphosphine)palladium(0) (60 mg, 0.12 mmol). The mixture was reacted at 100°C for 2 hours. Water (20 mL) was added, and the mixture was extracted three times with ethyl acetate (20 mL). The combined organic phases were washed sequentially with water (20 mL) and saturated brine (20 mL), dried over anhydrous sodium sulfate, filtered, and concentrated to obtain a residue. The residue was purified by silica gel column chromatography (petroleum ether:ethyl acetate = 0% to 40%) to obtain methyl 5'-(difluoromethyl)-4'-fluoro-2'-methoxy-5-(methyl-d3)-[1,1'-biphenyl]-2-carboxylate (160 mg, yield: 37.4%). LCMS calc. for $C_{17}H_{13}D_3F_3O_3$ $[M+H]^+$: m/z =328.1; Found: 328.1.

**Step 4: 5'-(Difluoromethyl)-4'-fluoro-2'-methoxy-5-(methyl-d3)-[1,1'-biphenyl]-2-carboxylic acid**

Methyl 5'-(difluoromethyl)-4'-fluoro-2'-methoxy-5-(methyl-d3)-[1,1'-biphenyl]-2-carboxylate (160 mg, 0.49 mmol) was dissolved in methanol (3 mL) and water (1 mL), followed by addition of sodium hydroxide (117 mg, 2.93 mmol). The mixture was reacted at 80°C for 2 hours. After the reaction was completed, the mixture was concentrated under reduced pressure, and subsequently added with water (20 mL). The resulting mixture was then extracted three times with ethyl acetate (20 mL). The aqueous phase was added with 1N dilute hydrochloric acid to adjust the pH to 5, and extracted three times with ethyl acetate (30 mL). The combined organic phases were sequentially washed with water (30 mL) and saturated brine (30 mL), dried over anhydrous sodium sulfate, filtered, and concentrated and purified to obtain 5'-(difluoromethyl)-4'-fluoro-2'-methoxy-5-(methyl-d3)-[1,1'-biphenyl]-2-carboxylic acid (110 mg, yield: 64.6%). LCMS calc. for $C_{16}H_{11}D_3F_3O_3$ $[M+H]^+$: m/z = 314.1; Found: 314.1.

**Step 5: (S)-5'-(Difluoromethyl)-4'-fluoro-2'-methoxy-N-((4-(1-(methoxy-d3)ethyl)benzyl)sulfonyl)-5-(methyl-d3)-[1,1'-biphenyl]-2-carboxamide**

5'-(Difluoromethyl)-4'-fluoro-2'-methoxy-5-(methyl-d3)-[1,1'-biphenyl]-2-carboxylic acid (110 mg, 0.35 mmol) and (S)-(4-(1-(methoxy-d3)ethyl)phenyl)methanesulfonamide (82 mg, 0.35 mmol) were dissolved in dichloromethane (4 mL), followed by addition of 1-ethyl-(3-dimethylaminopropyl)carbodiimide hydrochloride (134 mg, 0.7 mmol) and 4-dimethylaminopyridine (51 mg, 0.42 mmol). The mixture was reacted at 25°C for 3 hours. After the reaction was completed, the mixture was diluted with dichloromethane (30 mL) and sequentially washed with 1N dilute hydrochloric acid (20 mL) and saturated brine (10 Ml). The organic phase was dried over anhydrous sodium sulfate, filtered, and concentrated under reduced pressure. The resulting residue was purified by preparative high-performance liquid chromatography (Gemini-C18 150 x 21.2 mm, 5 μm, Mobile phase: A (0.1% FA in Water) B (ACN), 0-10 min: 55% B, 10-10.5 min: 55-95% B, 10.5-12.5 min: 95-95% B, 12.5-13 min: 95-55% B, 13-15 min: 55% B, Flow = 20 mL/min) to obtain (S)-5'-(difluoromethyl)-4'-fluoro-2'-methoxy-N-((4-(1-(methoxy-d3)ethyl)benzyl)sulfonyl)-5-(methyl-d3)-[1,1'-biphenyl]-2-carboxamide (82 mg, yield: 42.0%). LCMS calc. for $C_{26}H_{21}D_6F_3NO_5S$ $[M+H]^+$: m/z = 528.2; Found: 528.1. $^1$HNMR (400 MHz, DMSO) δ 11.77 (s, 1H), 7.45 (d, J = 8.0 Hz, 1H), 7.32-7.21 (m, 6H), 7.31-7.03 (m, 1H), 7.15 (s, 1H), 7.09 (d, J = 12.5 Hz, 1H), 4.65 (s,2H), 4.32 (q, J = 6.4 Hz, 1H), 3.73 (s, 3H), 1.32 (d, J = 6.4 Hz, 3H).

**Example 22: Preparation of compound cpd-22**

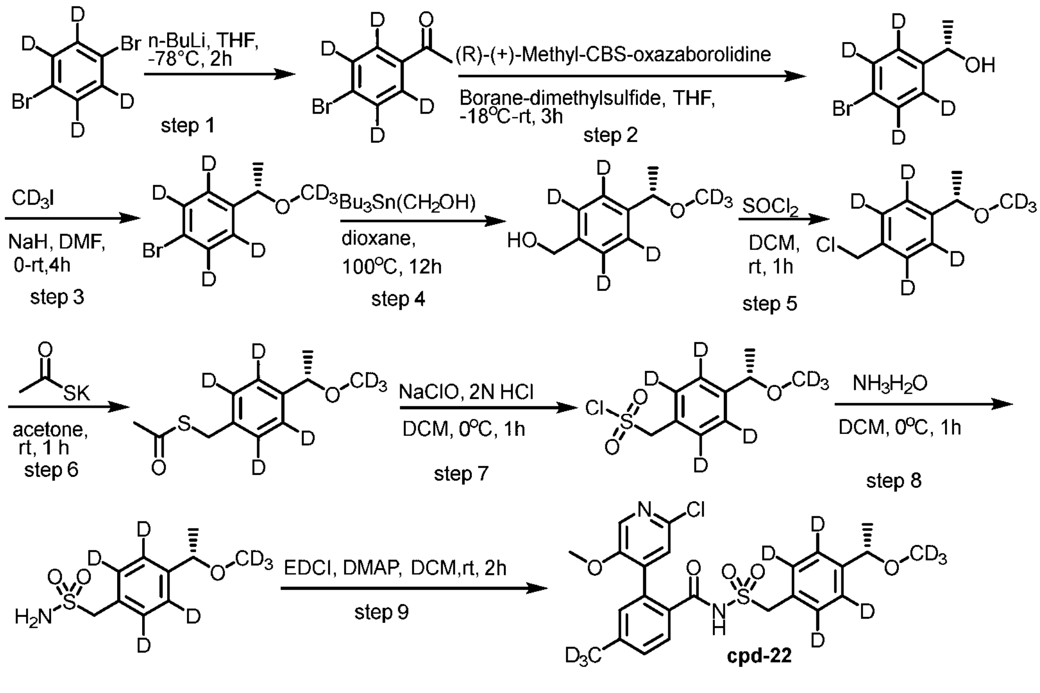


**Step 1: 1-(4-Bromophenyl-2,3,5,6-$d_4$)ethan-1-one**

Under a nitrogen atmosphere at -78°C, 1,4-dibromobenzene-2,3,5,6-$d_4$ (10 g, 41.7 mmol) and a solution of n-butyllithium in hexane (17 mL, 41.7 mmol, 2.5 mol/L) were dissolved in tetrahydrofuran (60 mL). The mixture was stirred at -78°C for 30 minutes, and then N-methoxy-N-methylacetamide (3.8 g, 37.5 mmol) was added. The mixture was further reacted at 25°C for 2 hours. After the reaction was completed, the mixture was added with a saturated aqueous solution of ammonium chloride (20 mL) and extracted three times with dichloromethane (50 mL). The combined organic phases were sequentially washed with water (30 mL) and saturated brine (30 mL), dried over anhydrous sodium sulfate, filtered, and concentrated to obtain a residue. The residue was purified by silica gel column chromatography (petroleum ether:ethyl acetate = 0% to 30%) to obtain 1-(4-bromophenyl-2,3,5,6-$d_4$)ethan-1-one (6.0 g, yield: 65.2%). $^1$H NMR (400 MHz, CDCl3) δ 2.59 (s, 3H).

**Step 2: (S)-1-(4-bromophenyl-2,3,5,6-$d_4$)ethan-1-ol**

Under a nitrogen atmosphere at -18°C, 1-(4-bromophenyl-2,3,5,6-$d_4$)ethan-1-one (6.0 g, 29.7 mmol) was dissolved in tetrahydrofuran (60 mL). A toluene solution of (R)-2-methyl-CBS-oxazaborolidine (3.1 mL, 3.1 mmol, 1 mol/L) and borane-dimethyl sulfide complex (22 mL, 22.0 mmol, 1 mol/L) were slowly added. The mixture was then reacted at 25°C for 3 hours. After the reaction was completed, the reaction mixture was added with methanol (50 mL) and subsequently concentrated to obtain a residue. The residue was purified by silica gel column chromatography (petroleum ether:ethyl acetate = 0% to 30%) to obtain (S)-1-(4-bromophenyl-2,3,5,6-$d_4$)ethan-1-ol (3.0 g, yield: 44.7%). $^1$HNMR (400 MHz, DMSO) $\delta$ 5.22 (s, 1H), 4.70 (q, J = 6.5 Hz, 1H), 1.29 (d, J = 6.5 Hz, 3H).

**Step 3: (S)-1-bromo-4-(1-(methoxy-$d_3$)ethyl)benzene-2,3,5,6-$d_4$**

Under a nitrogen atmosphere at 0°C, (S)-1-(4-bromophenyl-2,3,5,6-$d_4$)ethan-1-ol (3.0 g, 14.60 mmol) was dissolved in N,N-dimethylformamide (30 mL), followed by addition of sodium hydride (530 mg, 21.9 mmol, 60%). The mixture was reacted at 0°C for 30 minutes. Iodomethane-$d_3$ (3.0 g, 21.9 mmol) was added to the reaction mixture, which was reacted at 25°C for 4 hours. The mixture was added with a saturated aqueous ammonium chloride solution (30 mL) and extracted three times with ethyl acetate (50 mL). The combined organic phases were sequentially washed with water (30 mL) and saturated brine (30 mL), dried over anhydrous sodium sulfate, filtered, and concentrated to obtain a residue. The residue was purified by silica gel column chromatography (petroleum ether:ethyl acetate = 0% to 10%) to obtain (S)-1-bromo-4-(1-(methoxy-$d_3$)ethyl)benzene-2,3,5,6-$d_4$ (2.7 g, yield: 74.6%). $^1$HNMR (400 MHz, DMSO) $\delta$ 4.31 (q, J = 6.4 Hz, 1H), 1.30 (d, J = 6.4 Hz, 3H).

**Step 4: (S)-(4-(1-(Methoxy-$d_3$)ethyl)phenyl-2,3,5,6-$d_4$)methanol**

To a solution of (S)-1-bromo-4-(1-(methoxy-$d_3$)ethyl)benzene-2,3,5,6-$d_4$ (2.7 g, 12.2 mmol) in 1,4-dioxane (30 mL) were added (tributylstannyl)methanol (5.8 g, 18.3 mmol) and tetrakis(triphenylphosphine)palladium (1.4 g, 1.22 mmol). The mixture was reacted at 100°C for 12 hours. After the reaction was completed, the mixture was added with water (50 mL) and then extracted three times with ethyl acetate (50 mL). The combined organic phases were sequentially washed with water (30 mL) and saturated brine (30 mL), dried over anhydrous sodium sulfate, filtered, and concentrated to obtain a residue. The residue was purified by silica gel column chromatography (petroleum ether:ethyl acetate = 0% to 30%) to obtain (S)-(4-(1-(methoxy-$d_3$)ethyl)phenyl-2,3,5,6-$d_4$)methanol (1.3 g, yield: 55.7%). LCMS calc. for $C_{10}H_8D_7O_2$ $[M+H]^+$: m/z =174.1; Found: 139.2.$[M-OCD_3]^+$

**Step 5: (S)-1-(Chloromethyl)-4-(1-(methoxy-$d_3$)ethyl)benzene-2,3,5,6-$d_4$**

(S)-(4-(1-(Methoxy-$d_3$)ethyl)phenyl-2,3,5,6-$d_4$)methanol (1.3 g, 7.47 mmol) was dissolved in dichloromethane (15 mL), followed by addition of thionyl chloride (1.06 g, 8.96 mmol). The mixture was reacted at 25°C for 2 hours. The mixture was added with water (30 mL) and extracted three times with dichloromethane (30 mL). The combined organic phases were sequentially washed with water (30 mL) and saturated brine (30 mL), dried over anhydrous sodium sulfate, filtered, and concentrated to obtain (S)-1-(chloromethyl)-4-(1-(methoxy-$d_3$)ethyl)benzene-2,3,5,6-$d_4$ (1.4 g, crude). The crude product was directly used in the next step.

**Step 6: (S)-S-(4-(1-(Methoxy-$d_3$)ethyl)benzyl) ethanethioate-2,3,5,6-$d_4$**

(S)-1-(Chloromethyl)-4-(1-(methoxy-$d_3$)ethyl)benzene-2,3,5,6-$d_4$ (1.4 g, crude) was dissolved in acetone (20 mL), followed by addition of potassium thioacetate (1.26 g, 11.0 mmol). The mixture was reacted at 25°C for 1 hour under a nitrogen atmosphere. The reaction mixture was diluted with water (30 mL) and extracted three times with ethyl acetate (30 mL). The combined organic phases were washed with water (50 mL) and saturated brine (50 mL), dried over anhydrous sodium sulfate, filtered, and concentrated to obtain (S)-S-(4-(1-(methoxy-$d_3$)ethyl)benzyl) ethanethioate-2,3,5,6-$d_4$ (1.2 g, yield: 56.1%). LCMS calc. for $C_{12}H_{10}D_7O_2S$ $[M+H]^+$: m/z =232.1; Found: 198.1$[M-OCD_3]^+$.

**Step 7: (S)-(4-(1-(Methoxy-$d_3$)ethyl)phenyl-2,3,5,6-$d_4$)methanesulfonyl chloride**

To a solution of (S)-S-(4-(1-(methoxy-$d_3$)ethyl)benzyl) ethanethioate-2,3,5,6-$d_4$ (1.2 g, 5.19 mmol) in dichloromethane (20 mL) under a nitrogen atmosphere at 0°C was added 2N dilute hydrochloric acid (16 mL), followed by slow dropwise addition of an aqueous sodium hypochlorite solution (2.32 g, 31.1 mmol). The mixture was reacted for 1 hour. The reaction mixture was diluted with water (30 mL). The aqueous phase was extracted three times with dichloromethane (30 mL). The combined organic phases were washed with water (30 mL) and saturated brine (30 mL), dried over anhydrous

sodium sulfate, filtered, and concentrated to obtain (S)-(4-(1-(methoxy-$d_3$)ethyl)phenyl-2,3,5,6-$d_4$)methanesulfonyl chloride (1.2 g, yield: 72.4%).

**Step 8: (S)-(4-(1-(Methoxy-d3)ethyl)phenyl-2,3,5,6-d4)methanesulfonamide**

To a mixture of tetrahydrofuran (16 mL) and aqueous ammonia (8 mL) under a nitrogen atmosphere at 0°C was added (S)-(4-(1-(methoxy-d3)ethyl)phenyl-2,3,5,6-d4)methanesulfonyl chloride (1.2 g, 4.7 mmol). The mixture was reacted at 25°C for 1 hour. The reaction mixture was diluted with water (30 mL) and extracted three times with dichloromethane (30 mL). The combined organic phases were washed with water (50 mL) and saturated brine (50 mL), dried over anhydrous sodium sulfate, filtered, and concentrated to obtain a residue. The residue was purified by silica gel column chromatography (petroleum ether:ethyl acetate = 0% to 50%) to obtain (S)-(4-(1-(methoxy-d3)ethyl)phenyl-2,3,5,6-d4)methanesulfonamide (270 mg, yield: 23.4%). LCMS calc. for $C_{10}H_9D_7NO_3S$ $[M+H]^+$: m/z = 237.1; Found: 254.2 $[M+NH_4]^+$. $^1$HNMR (400 MHz, DMSO) δ 6.84 (s, 2H), 4.32 (q, J = 6.4 Hz, 1H), 4.25 (s, 2H), 1.33 (d, J = 6.4 Hz, 3H).

**Step 9: (S)-2-(2-Chloro-5-methoxypyridin-4-yl)-N-(4-(1-(methoxy-d3)ethyl)(phenyl-2,3,5,6-d4)methylsulfonyl)-4-(methyl-d3)benzamide**

2-(2-Chloro-5-methoxypyridin-4-yl)-4-(methyl-d3)benzoic acid (40 mg, 0.14 mmol) and (S)-(4-(1-(methoxy-d3)ethyl)phenyl-2,3,5,6-d4)methanesulfonamide (34 mg, 0.14 mmol) were dissolved in dichloromethane (3 mL), followed by addition of 1-ethyl-(3-dimethylaminopropyl)carbodiimide hydrochloride (33 mg, 0.17 mmol) and 4-dimethylaminopyridine (21 mg, 0.17 mmol). The mixture was reacted at 25°C for 2 hours. The reaction mixture was concentrated under reduced pressure and purified by preparative high-performance liquid chromatography (Gemini-C18 150 x 21.2 mm, 5 μm, Mobile phase: A (0.1% FA in Water) B (ACN), 0-10 min: 50% B, 10-10.5 min: 50-95% B, 10.5-12.5 min: 95-95% B, 12.5-13 min: 95-50% B, 13-15 min: 50% B, Flow = 20 mL/min) to obtain (S)-2-(2-chloro-5-methoxypyridin-4-yl)-N-(4-(1-(methoxy-d3)ethyl)(phenyl-2,3,5,6-d4)methylsulfonyl)-4-(methyl-d3)benzamide (30 mg, yield: 40.1%). LCMS calc. for $C_{24}H_{16}D_{10}ClN_2O_5S$ $[M+H]^+$: m/z = 499.2; Found: 499.2. $^1$H NMR (400 MHz, DMSO) δ = 11.91 (s, 1H), 8.16 (s, 1H), 7.38 (s, 1H), 7.36-7.28 (m, 2H), 7.24 (s, 1H), 4.68 (s, 2H), 4.33 (q, J=6.4, 1H), 3.78 (s, 3H), 1.32(d, J=6.4, 3H).

**Example 23: Preparation of compound cpd-23**

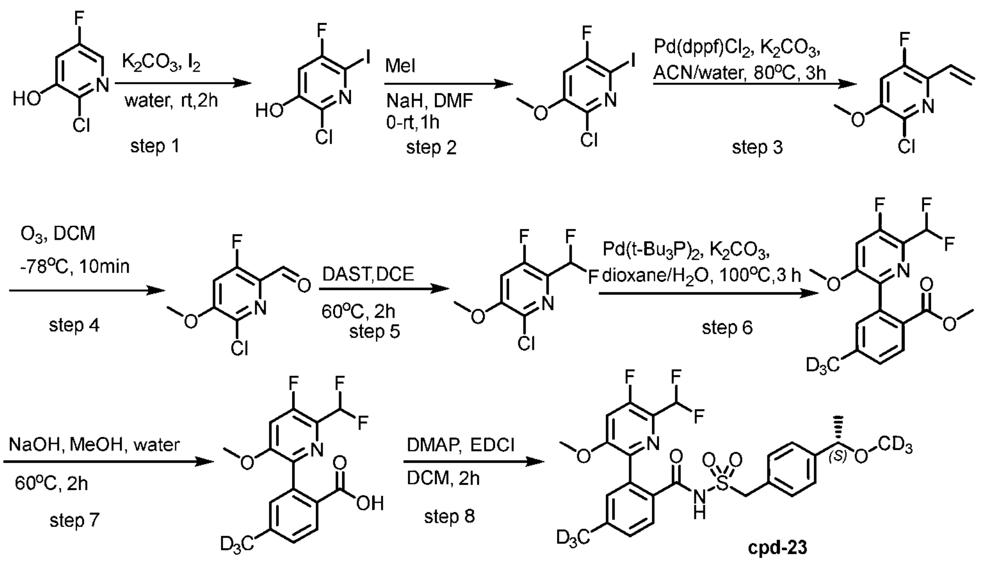


**Step 1: 2-Chloro-5-fluoro-6-iodopyridin-3-ol**

2-Chloro-5-fluoropyridin-3-ol (900 mg, 6.10 mmol), iodine (1.70 g, 6.71 mmol) and potassium carbonate (1.70 g, 12.2 mmol) were dissolved in water (10 mL). The mixture was reacted at 25°C for 2 hours. After the reaction was completed, the reaction mixture was diluted with water (30 mL). The aqueous phase was extracted three times with ethyl acetate (30 mL). The combined organic phases were washed with water (20 mL) and saturated brine (20 mL), dried over anhydrous sodium sulfate, filtered, and concentrated to obtain a residue. The residue was purified by silica gel column

chromatography (ethyl acetate:petroleum ether = 0% to 30%) to obtain 2-chloro-5-fluoro-6-iodopyridin-3-ol (1.0 g, 53.9%). LCMS calc. for $C_5H_3ClFINO$ [M+H]$^+$: m/z =273.9; Found: 273.9.

**Step 2: 2-Chloro-5-fluoro-6-iodo-3-methoxypyridine**

Under a nitrogen atmosphere at 0°C, 2-chloro-5-fluoro-6-iodopyridin-3-ol (1.0 g, 3.70 mmol) was dissolved in N,N-dimethylformamide (10 mL), followed by addition of sodium hydride (190 mg, 4.80 mmol, 60%) in portions. The mixture was reacted at 0°C for 30 minutes. Iodomethane (346 mL, 5.55 mmol, 2.28 g/L) was added to the reaction mixture, which was reacted at room temperature for 1 hour. After the reaction was completed, the reaction was quenched by adding a saturated ammonium chloride solution (10 mL). The aqueous phase was extracted three times with ethyl acetate (30 mL). The combined organic phases were washed with water (20 mL) and saturated brine (20 mL), dried over anhydrous sodium sulfate, filtered, and concentrated to obtain a residue. The residue was purified by silica gel column chromatography (ethyl acetate:petroleum ether = 0% to 30%) to obtain 2-chloro-5-fluoro-6-iodo-3-methoxypyridine (780 mg, 64.8%). LCMS calc. for $C_6H_5ClFINO$ [M+H]$^+$: m/z =287.9; Found: 287.9.

**Step 3: 2-Chloro-5-fluoro-3-methoxy-6-vinylpyridine**

2-Chloro-5-fluoro-6-iodo-3-methoxypyridine (580 mg, 2.02 mmol) was dissolved in acetonitrile (5 mL) and water (1 mL), followed by addition of potassium vinyltrifluoroborate (405 mg, 3.03 mmol), potassium carbonate (558 mg, 4.04 mmol), and 1,1'-bis(diphenylphosphino)ferrocene palladium dichloride (148 mg, 0.2 mmol). The mixture was reacted at 80°C for 3 hours. Water (20 mL) was added, and the mixture was extracted three times with ethyl acetate (20 mL). The combined organic phases were washed sequentially with water (20 mL) and saturated brine (20 mL), dried over anhydrous sodium sulfate, filtered, and concentrated to obtain a residue. The residue was purified by silica gel column chromatography (petroleum ether:ethyl acetate = 0% to 30%) to obtain 2-chloro-5-fluoro-3-methoxy-6-vinylpyridine (300 mg, yield: 71.3%). LCMS calc. for $C_8H_8ClFNO$ [M+H]$^+$: m/z =188.0; Found: 188.1.

**Step 4: 6-Chloro-3-fluoro-5-methoxypicolinaldehyde**

2-Chloro-5-fluoro-3-methoxy-6-vinylpyridine (300 mg, 1.60 mmol) was dissolved in dichloromethane (5 mL). The mixture was cooled to -78°C, and subjected to ozone for 10 minutes. After the reaction was completed, the reaction mixture was quenched by adding dimethyl sulfide (1 mL) dropwise, then concentrated to obtain 6-chloro-3-fluoro-5-methoxypicolinaldehyde (400 mg, crude), which was used directly in the next step. LCMS calc. for $C_7H_6ClFNO_2$ [M+H]$^+$: m/z =190.0; Found: 190.0.

**Step 5: 2-Chloro-6-(difluoromethyl)-5-fluoro-3-methoxypyridine**

6-Chloro-3-fluoro-5-methoxypicolinaldehyde (400 mg, 2.12 mmol) was dissolved in 1,2-dichloroethane (5 mL), followed by slow dropwise addition of diethylaminosulfur trifluoride (1.40 g, 8.48 mmol). The mixture was reacted at 60°C for 2 hours. The reaction mixture was diluted with water (20 mL). The aqueous phase was extracted three times with dichloromethane (20 mL). The combined organic phases were washed with water (20 mL) and saturated brine (10 mL), dried over anhydrous sodium sulfate, filtered, and concentrated to obtain a residue. The residue was purified by silica gel column chromatography (ethyl acetate:petroleum ether = 0% to 60%) to obtain 2-chloro-6-(difluoromethyl)-5-fluoro-3-methoxypyridine (250 mg, yield: 50.4%). LCMS calc. for $C_7H_6ClF_3NO$ [M+H]$^+$: m/z =212.0; Found: 212.0.

**Step 6: Methyl 2-(6-(difluoromethyl)-5-fluoro-3-methoxypyridin-2-yl)-4-(methyl-d3)benzoate**

2-Chloro-6-(difluoromethyl)-5-fluoro-3-methoxypyridine (140 mg, 0.66 mmol) was dissolved in 1,4-dioxane (4 mL) and water (0.5 mL), followed by addition of methyl 4-(methyl-d3)-2-(4,4,5,5-tetramethyl-1,3,2-dioxaborolan-2-yl) benzoate (203 mg, 0.73 mmol), potassium carbonate (183 mg, 1.32 mmol), and bis(tri-tert-butylphosphine)palladium (34 mg, 0.07 mmol). The mixture was reacted at 100°C for 3 hours. Water (20 mL) was added, and the mixture was extracted three times with ethyl acetate (20 mL). The combined organic phases were washed sequentially with water (20 mL) and saturated brine (20 mL), dried over anhydrous sodium sulfate, filtered, and concentrated to obtain a residue. The residue was purified by silica gel column chromatography (petroleum ether:ethyl acetate = 0% to 30%) to obtain methyl 2-(6-(difluoromethyl)-5-fluoro-3-methoxypyridin-2-yl)-4-(methyl-d3)benzoate (150 mg, yield: 62.1%). LCMS calc. for $C_{16}H_{12}D_3F_3NO_3$ [M+H]$^+$: m/z =329.1; Found: 329.1.

**Step 7: 2-(6-(Difluoromethyl)-5-fluoro-3-methoxypyridin-2-yl)-4-(methyl-d3)benzoic acid**

Methyl 2-(6-(difluoromethyl)-5-fluoro-3-methoxypyridin-2-yl)-4-(methyl-d3)benzoate (120 mg, 0.37 mmol) was dissolved in methanol (2 mL) and water (0.5 mL), followed by addition of sodium hydroxide (88 mg, 2.19 mmol). The mixture was reacted at 60°C for 2 hours. After the reaction was completed, the mixture was concentrated under reduced pressure, and subsequently added with water (20 mL). The resulting mixture was then extracted three times with ethyl acetate (20 mL). The aqueous phase was added with 2N dilute hydrochloric acid (20 mL) to adjust the pH to 6, and extracted three times with ethyl acetate (50 mL). The combined organic phases were washed with water (20 mL) and saturated brine (20 mL), dried over anhydrous sodium sulfate, filtered, and concentrated and purified to obtain 2-(6-(difluoromethyl)-5-fluoro-3-methoxypyridin-2-yl)-4-(methyl-d3)benzoic acid (120 mg, crude). LCMS calc. for $C_{15}H_{10}D_3F_3NO_3$ $[M+H]^+$: m/z = 315.1; Found: 315.1.

**Step 8: (S)-2-(6-(Difluoromethyl)-5-fluoro-3-methoxypyridin-2-yl)-N-((4-(1-(methoxy-d3)ethyl)benzyl)sulfonyl)-4-(methyl-d3)benzamide**

2-(6-(Difluoromethyl)-5-fluoro-3-methoxypyridin-2-yl)-4-(methyl-d3)benzoic acid (50 mg, 0.16 mmol) and (S)-(4-(1-(methoxy-d3)ethyl)phenyl)methanesulfonamide (37 mg, 0.16 mmol) were dissolved in dichloromethane (4 mL), followed by addition of 1-ethyl-(3-dimethylaminopropyl)carbodiimide hydrochloride (61 mg, 0.32 mmol) and 4-dimethylaminopyridine (23 mg, 0.19 mmol). The mixture was reacted at 25°C for 2 hours. After the reaction was completed, the mixture was diluted with 1N dilute hydrochloric acid (10 mL) to adjust the pH to 4. The aqueous phase was washed three times with dichloromethane (20 mL). The combined organic phases were washed with water (20 mL) and saturated brine (20 mL), dried over anhydrous sodium sulfate, filtered, and concentrated under reduced pressure. The resulting residue was purified by preparative high-performance liquid chromatography (Gemini-C18 150 x 21.2 mm, 5 μm, Mobile phase: A (0.1% FA in Water) B (ACN), 0-10 min: 52% B, 10-10.5 min: 52-95% B, 10.5-12.5 min: 95-95% B, 12.5-13 min: 95-52% B, 13-15 min: 52% B, Flow = 20 mL/min) to obtain (S)-2-(6-(Difluoromethyl)-5-fluoro-3-methoxypyridin-2-yl)-N-((4-(1-(methoxy-d3)ethyl)benzyl)sulfonyl)-4-(methyl-d3)benzamide (25 mg, yield: 28.2%). LCMS calc. for $C_{25}H_{20}D_6F_3N_2O_5S$ $[M+H]^+$: m/z = 529.1; Found: 529.1. $^1$HNMR (400 MHz, DMSO) δ 12.03 (s, 1H), 7.70 (d, J = 12.1 Hz, 1H), 7.39-7.24 (m, 7H), 7.21-6.92 (m, 1H), 4.64 (s, 2H), 4.33 (q, J = 6.4 Hz, 1H), 3.82 (s, 3H), 1.33 (d, J = 6.4 Hz, 3H).

**Example 24: Preparation of compound cpd-24**

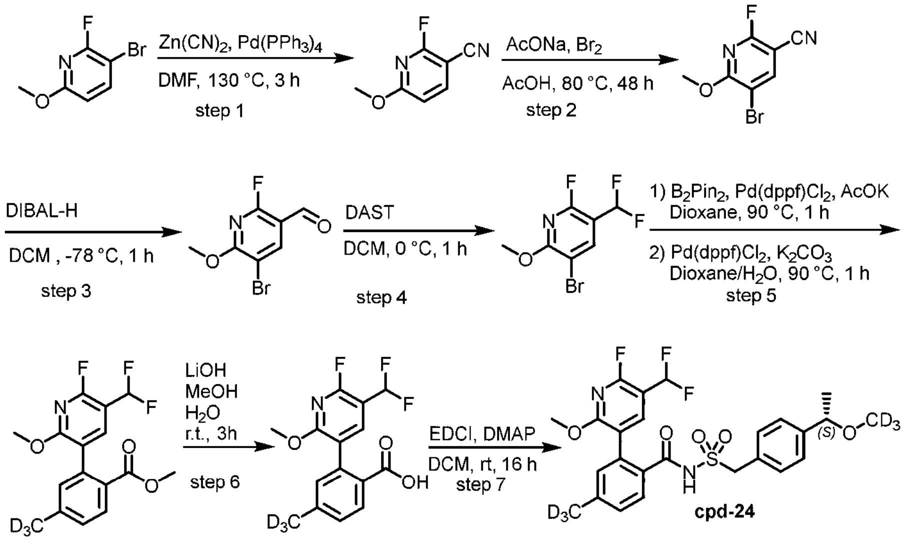


**Step 1: 2-Fluoro-6-methoxynicotinonitrile**

To a solution of 3-bromo-2-fluoro-6-methoxypyridine (6.0 g, 29.1 mmol) in N,N-dimethylformamide (40 mL) were added zinc cyanide (2.05 g, 14.5 mmol) and tetrakis(triphenylphosphine)palladium (1.68 g, 1.46 mmol). The reaction mixture was stirred at 130°C under a nitrogen atmosphere for 3 hours. After the reaction was completed, the reaction mixture was poured into water (400 mL) and extracted twice with ethyl acetate (50 mL). The combined organic phases

were washed with saturated brine, dried over anhydrous sodium sulfate, filtered, and concentrated in vacuo. The residue was purified by silica gel column chromatography (ethyl acetate:petroleum ether = 5% to 10%) to obtain 2-fluoro-6-methoxynicotinonitrile (3.91 g, 88.3%). $^1$H NMR (400 MHz, DMSO-d6) δ 8.36 (t, J = 9.3, 8.5 Hz, 1H), 6.99 (d, J = 8.5, 1.4 Hz, 1H), 3.94 (s, 3H).

**Step 2: 5-Bromo-2-fluoro-6-methoxynicotinonitrile**

To a solution of 2-fluoro-6-methoxynicotinonitrile (2.91 g, 19.1 mmol) and sodium acetate (3.14 g, 38.3 mmol) in acetic acid (10 mL) was added liquid bromine (2.94 mL, 57.4 mmol). The mixture was stirred at 80°C under a nitrogen atmosphere for 48 hours. After the reaction was completed, the reaction mixture was poured into a saturated sodium sulfite solution (100 mL), stirred for ten minutes, and then extracted twice with ethyl acetate (30 mL). The combined organic phases were dried over anhydrous sodium sulfate, filtered, and concentrated in vacuo. The residue was purified by silica gel column chromatography (ethyl acetate:petroleum ether = 5% to 10%) to obtain 5-bromo-2-fluoro-6-methoxynicotinonitrile (1.8 g, yield: 40.7%). $^1$H NMR (400 MHz, DMSO-d6) δ 8.80(d, J = 8.0 Hz, 1H), 4.01 (s, 3H).

**Step 3: 5-Bromo-2-fluoro-6-methoxynicotinaldehyde**

To a solution of 5-bromo-2-fluoro-6-methoxynicotinonitrile (1.8 g, 7.79 mmol) in dichloromethane (20 mL) at -78°C was added diisobutylaluminum hydride (1.69 mL, 9.35 mmol). The reaction mixture was stirred at -78°C for 1 hour. After the reaction was completed, the reaction mixture was poured into a saturated aqueous ammonium chloride solution (100 mL) and extracted twice with dichloromethane (30 mL). The combined organic phases were dried over anhydrous sodium sulfate, filtered, and concentrated in vacuo. The residue was purified by silica gel column chromatography (ethyl acetate:petroleum ether = 5% to 10%) to obtain 5-Bromo-2-fluoro-6-methoxynicotinaldehyde (0.98 g, yield: 53.8%). $^1$H NMR (400 MHz, DMSO-d6) δ 9.98 (s, 1H), 8.47 (d, J = 8.4 Hz, 1H), 4.04 (s, 3H).

**Step 4: 3-Bromo-5-(difluoromethyl)-6-fluoro-2-methoxypyridine**

To a solution of 5-bromo-2-fluoro-6-methoxynicotinaldehyde (980 mg, 4.19 mmol) in dichloromethane (10 mL) at 0°C was added diethylaminosulfur trifluoride (2.03 g, 12.6 mmol). The reaction mixture was stirred at 0°C for 3 hours. After the reaction was completed, the reaction mixture was poured into a saturated aqueous solution of sodium bicarbonate (100 mL) and extracted twice with dichloromethane (50 mL). The combined organic phases were dried over anhydrous sodium sulfate, filtered, and concentrated in vacuo. The residue was purified by silica gel column chromatography (ethyl acetate:petroleum ether = 5% to 10%) to obtain 3-bromo-5-(difluoromethyl)-6-fluoro-2-methoxypyridine (757 mg, yield: 70.6%). $^1$H NMR (400 MHz, DMSO-d6) δ 8.41(dt, J=8.4, 0.9 Hz, 1H), 7.12(t, J = 54.0 Hz, 1H), 3.97(s, 3H).

**Step 5: Methyl 2-(5-(difluoromethyl)-6-fluoro-2-methoxypyridin-3-yl)-4-(methyl-d3)benzoate**

To a solution of 3-bromo-5-(difluoromethyl)-6-fluoro-2-methoxypyridine (300 mg, 1.17 mmol) in 1,4-dioxane (4 mL) were added bis(pinacolato)diboron (387 mg, 1.52 mmol), potassium acetate (345 mg, 3.52 mmol), and 1,1'-bis(diphenylphosphino)ferrocene palladium dichloride (85.8 mg, 0.12 mmol). The reaction mixture was stirred at 90°C under a nitrogen atmosphere for 1 hour. After the reaction was completed, the mixture was cooled to room temperature, and then 1,1'-bis(diphenylphosphino)ferrocene palladium dichloride (85.8 mg, 0.12 mmol), methyl 2-bromo-4-(methyl-d3) benzoate (272 mg, 1.17 mmol), water (1.0 mL), and potassium carbonate (486 mg, 3.52 mmol) were added to the reaction mixture. The reaction mixture was stirred at 90°C under a nitrogen atmosphere for 1 hour. After the reaction was completed, the reaction mixture was concentrated and purified by silica gel column chromatography (ethyl acetate:petroleum ether = 5% to 10%) to obtain methyl 2-(5-(difluoromethyl)-6-fluoro-2-methoxypyridin-3-yl)-4-(methyl-d3)benzoate (260 mg, 67.6%). LCMS calc. for $C_{16}H_{12}D_3F_3NO_3$ $[M+H]^+$: m/z =329.1; Found: 329.1.

**Step 6: 2-(5-(Difluoromethyl)-6-fluoro-2-methoxypyridin-3-yl)-4-(methyl-d3)benzoic acid**

To a solution of methyl 2-(5-(difluoromethyl)-6-fluoro-2-methoxypyridin-3-yl)-4-(methyl-d3)benzoate (260 mg, 0.79 mmol) in methanol (4.0 mL) were added lithium hydroxide (37.9 mg, 1.58 mmol) and water (1.0 mL). The reaction mixture was stirred at 25°C for 1 hour. After the reaction was completed, the reaction mixture was poured into water (20 mL). The pH was adjusted to 6 using 1 M hydrochloric acid. The aqueous phase was extracted twice with ethyl acetate (30 mL). The combined organic phases were dried over anhydrous sodium sulfate, filtered, and concentrated in vacuo. The crude residue was purified by silica gel column chromatography (methanol:dichloromethane = 5% to 10%) to obtain 2-(5-(difluoromethyl)-6-fluoro-2-methoxypyridin-3-yl)-4-(methyl-d3)benzoic acid (220 mg, 30.9%). LCMS calc. for $C_{15}H_8D_3F_3NO_3$ $[M-H]^-$: m/z = 313.1; Found: 313.1.

**Step 7: (S)-2-(5-(Difluoromethyl)-6-fluoro-2-methoxypyridin-3-yl)-N-((4-(1-(methoxy-d3)ethyl)benzyl)sulfonyl)-4-(methyl-d3)benzamide**

To a solution of 2-(5-(difluoromethyl)-6-fluoro-2-methoxypyridin-3-yl)-4-(methyl-d3)benzoic acid (150 mg, 0.48 mmol) in dichloromethane (5.0 mL) was added (S)-(4-(1-(methoxy-d3)ethyl)phenyl)methanesulfonamide (131 mg, 0.57 mmol), 1-ethyl-(3-dimethylaminopropyl)carbodiimide hydrochloride (137 mg, 0.72 mmol), and 4-dimethylaminopyridine (116 mg, 0.95 mmol). The reaction mixture was stirred at 25°C for 3 hours. After the reaction was completed, the reaction mixture was purified by silica gel column chromatography (methanol:dichloromethane = 5% to 10%) to obtain a crude product. The crude product was further purified by preparative high-performance liquid chromatography (SHIMADZU Prep C18 10μm 20*250mm, mobile phase: A (0.1% FA in water) B (ACN): 40% B, 0.5-2 min: 60% B, 2-4 min: 60-75% B, 4-6 min: 75% B, 6-7 min: 75-95% B, 7-12 min: 95% B, 12-12.01 min: 95-40% B, 12.01-15.5 min: 40% B (Flow = 20 mL/min)) to obtain (S)-2-(5-(difluoromethyl)-6-fluoro-2-methoxypyridin-3-yl)-N-((4-(1-(methoxy-d3)ethyl)benzyl)sulfonyl)-4-(methyl-d3)benzamide (18 mg, 14.0%). LCMS calc. for $C_{25}H_{18}D_6F_3N_2O_5S$ $[M-H]^-$: m/z = 527.2; Found: 527.1 $^1H$ NMR (400 MHz, DMSO-d6) δ 11.87 (s, 1H), 8.01 (d, J = 9.1 Hz, 1H), 7.37-7.07 (m, 8H), 4.68 (s, 2H), 4.42-4.17 (m, 1H), 3.82 (s, 3H), 1.32 (d, J = 6.4 Hz, 3H).

**Biological assays**

**Polθ ATPase activity assay**

The inhibitory efficiency of the compounds of formula (I) against Polθ ATPase activity was determined using the ADP-Glo Kinase Assay kit (Promega, V9101).

Compounds were dissolved in 100% DMSO and serially diluted. 0.1 μL of each dilution was added to a 384-well plate. The starting (final) concentration was 100 nM, followed by a 3-fold serial dilution for a total of 10 concentration points in duplicate (n=2). 5 μL of purified truncated POLQ-N protein (aa 1-894, N-terminal 6-His tag) was added to each well. After centrifugation at 1000 rpm for 1 minute, the plate was incubated at 25°C for 10 minutes. 5 μL of substrate reaction solution (containing ATP & ssDNA) was added to each well, and the reaction was carried out at 25°C for 60 minutes. 5 μL of ADP-Glo Reagent was added to each well. After centrifugation at 1000 rpm for 1 minute, the plate was incubated at 25°C for 40 minutes. 10 μL of detection solution was added to each well. After centrifugation at 1000 rpm for 1 minute, the plate was incubated at 25°C for 40 minutes. Luminescence signals were recorded using a microplate reader (BMG, PHERAstar FSX). The final reaction system contained 1 nM of POLQ-N enzyme; 50 nM of ssDNA (5'-CCAGTGAATTGTTGCTC GGTACCTGCTAAC-3'); 50 μM of ATP; and 1% DMSO.

The inhibition percentage (%) of the compound at each concentration was calculated using the RFU values normalized against the control wells within each assay plate. Curve fitting was performed using XLfit, and the specific results are shown in Table 1.

Table 1: Inhibitory activity of compounds against Polθ ATPase

| Compound No. | ATPase IC50 (nM) |
|---|---|
| Ref. 1 | ++ |
| cpd-3 | + |
| cpd-4 | + |
| cpd-5 | + |
| cpd-6 | + |
| cpd-7 | + |
| cpd-8 | + |
| cpd-9 | + |
| cpd-10 | + |
| cpd-11 | + |
| cpd-12 | + |
| cpd-13 | + |
| cpd-14 | + |
| cpd-15 | ++ |

(continued)

| Compound No. | ATPase IC50 (nM) |
|---|---|
| cpd-16 | + |
| cpd-17 | + |
| cpd-18 | + |
| cpd-19 | + |
| cpd-20 | + |
| cpd-21 | + |
| cpd-22 | + |
| Ref. 1 represents 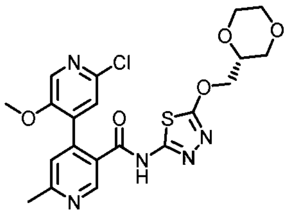 , which is disclosed in WO 2023233295 A1. *+ indicates $IC_{50} \leq 2$ nM; ++ indicates 2 nM < $IC_{50} \leq 10$ nM; +++ indicates 10 nM < $IC_{50} \leq 100$ nM; ++++ indicates 100 nM < $IC_{50} \leq 1000$ nM. | |

The specific values for the inhibitory activity of the compounds in Table 1 against Polθ ATPase are as follows:

| Compound No. | ATPase IC50 (nM) |
|---|---|
| Ref. 1 | 2.2 |
| cpd-3 | 0.71 |
| cpd-4 | 0.65 |
| cpd-5 | 0.50 |
| cpd-6 | 0.60 |
| cpd-7 | 0.71 |
| cpd-8 | 0.72 |
| cpd-9 | 0.52 |
| cpd-10 | 1.1 |
| cpd-11 | 0.53 |
| cpd-12 | 1.0 |
| cpd-13 | 0.88 |
| cpd-14 | 1.6 |
| cpd-15 | 8.2 |
| cpd-16 | 0.39 |
| cpd-17 | 1.4 |
| cpd-18 | 1.4 |
| cpd-19 | 0.45 |
| cpd-20 | 1.4 |

(continued)

| Compound No. | ATPase IC50 (nM) |
|---|---|
| cpd-21 | 1.5 |
| cpd-22 | 1.1 |

The data above show that the compounds of the embodiments of the present disclosure have good inhibitory activity against Polθ ATPase; in particular, the inhibitory activity of certain compounds against Polθ ATPase is superior to that of compound Ref. 1.

**DLD-1 BRCA2-/- cell proliferation assay:**

The anti-proliferative effect of the compounds on the human colorectal adenocarcinoma cell line **DLD-1 BRCA2$^{-/-}$** method.

Instrument and equipment:

| Instrument name | Supplier | Model |
|---|---|---|
| $CO_2$ incubator | Thermo | 3111 |
| Vi-CELL cell counter | Beckman Counter | Vi-CELL XR |
| Biosafety cabinet | Thermo | 1389 |
| Centrifuge | Thermo | ST40R |
| Enspire | Perkinelme | 2300 |
| eline 12-channel electronic pipette | BIOHIT | 730491 |
| Low-temperature freezer (-20°C) | Thermo | ULT3030V |

Reagents and consumables:

DLD-1 (purchased from ATCC, Cat. No.: CCL-221, Batch No.: 70033255);
RPMI1640 (purchased from Invitrogen, Cat. No.: 11875-085);
0.25% Trypsin-EDTA (purchased from Invitrogen, Cat. No.: 25200-072);
Penicillin-Streptomycin solution (purchased from Hyclone, Cat. No.: SV30010);
FBS (purchased from BI, Cat. No.: 04-002-1A);
DMSO (purchased from Sigma, Cat. No.: D4540-1L);
96-well plate (purchased from Corning, Cat. No.: CLS3903);
CellTiter-Glo reagent (purchased from Promega, Cat. No.: G7558).

Experimental method:

The anti-proliferative effect of the compounds on a human colorectal adenocarcinoma cell line was evaluated using an ATP quantification method, wherein the measured activity correlates well with the number of viable cells. The genetically edited cell line DLD-1 BRCA2$^{-/-}$ was cultured in RPMI 1640 medium (Invitrogen, 11875-085) supplemented with 10% (v/v) fetal bovine serum (BI, 04-002-1A). The cell line was cultured according to the standard instructions of the American Type Culture Collection (ATCC) and identified via short tandem repeat (STR) genotyping.

In the proliferation assay, cells were harvested and resuspended in cell culture medium. Cell density was determined using a cell counter. An appropriate volume of cell suspension was aspirated from the centrifuge tube to prepare a plating working solution (150 cells/100 μL for the DLD-1 BRCA2$^{-/-}$ cell line). 100 μL of the cell suspension was seeded into each well of a 96-well plate (Corning, 3603) and incubated overnight in an incubator. The diluted test compound solutions were added to the wells. On the fourth and seventh days of compound incubation, the medium was

replaced with fresh culture medium, and the compounds were re-administered, for a total incubation period of 10 days. Following incubation, the cell culture plates were removed and equilibrated to room temperature. 100 μL of CellTiter-Glo (Promega, G7558) was added to each well, and the plate was incubated at room temperature for 30 minutes. Data were read using a microplate reader (PerkinElmer, Enspire) in 96-well luminescence mode. Data analysis, curve fitting, and reporting were performed using the dose-response one-site 205 model of IDBS XL fit. Specific results are shown in Table 2.

Table 2: Proliferation inhibitory activity of compounds against DLD-1 BRCA2-/- cells

| Compound No. | DLD1 BRCA2$^{-/-}$ $IC_{50}$ (nM) |
|---|---|
| Ref. 1 | 21 |
| cpd-3 | 24 |
| cpd-4 | 17 |
| cpd-5 | 27 |
| cpd-6 | 189 |
| cpd-7 | 59 |
| cpd-8 | 57 |
| cpd-9 | 63 |
| cpd-10 | 514 |
| cpd-11 | 99 |
| cpd-12 | 70 |
| cpd-13 | 202 |
| cpd-14 | 108 |
| cpd-15 | 154 |
| cpd-16 | 24 |
| cpd-17 | 16 |
| cpd-18 | 17 |
| cpd-19 | 21 |
| cpd-21 | 5.2 |
| cpd-22 | 48 |
| cpd-23 | 22 |
| cpd-24 | 33 |

The data above demonstrate that the compounds of the embodiments of the present disclosure exhibit good inhibitory activity against the proliferation of DLD-1 BRCA2$^{-/-}$ cells.

**TMEJ reporter assay:**

The inhibitory effect of the compounds on the Theta-mediated end joining (TMEJ) process was evaluated.

Experimental instrument:

| Instrument | Supplier | Model |
|---|---|---|
| Biological cabinet | ThermoFisher | 307 |
| $CO_2$ incubator | ESCO | CLM-240B-8-CN |
| Cell countess | NanoEnTeK | EVE-MC2 |

(continued)

| Instrument | Supplier | Model |
|---|---|---|
| Echo | LabCyte | 655 |
| Microplate centrifuge | Monad | PlatePro 3200 |
| BMG | BMG LRBTECH | PHERAstar FSX |
| DNA imager | BioRad | ChemiDoc |
| Neon Transfection system | Invitrogen | MPK5000 |

Experimental materials:

| Reagent | Supplier | Cat. No. |
|---|---|---|
| Substrate NSC | ICE | / |
| Nano-Glo Luciferase Assay kit | Promega | N1120 |
| DMEM | Gibco | 11995-065 |
| DMSO | Solarbio | D8371 |
| DPBS | Gibco | 14190250 |
| TrypL Express | Gibco | 25200-072 |
| PenStrep | Gibco | 15140122 |
| 384-well plate | Corning | 3764 |
| Neon™ Transfection System 100 μL Kit | Invitrogen | MPK10096 |

Experimental method:

(1) HEK293T cells were cultured in DMEM supplemented with 10% heat-inactivated fetal bovine serum (FBS) and 1% penicillin-streptomycin (PS).
(2) Prior to startup, the Neon transfection system was disinfected, and E2 and R buffers were equilibrated to room temperature.
(3) Culture medium without antibiotics was pre-warmed.
(4) Cells in the flask reaching 80-90% confluency were trypsinized, neutralized with culture medium, centrifuged, and counted.
(5) For transfection using a 100 μL electroporation tip, $2 \times 10^6$ cells were taken, washed once with DPBS (without $Mg^{2+}$ and $Ca^{2+}$), centrifuged at 1000 rpm for 5 min, and the supernatant was discarded. The number of cells and volume may be increased as required.
(6) The Neon system was set up; a Neon tube was inserted into the Neon pipette and E2 buffer was added.
(7) 10 μg of TMEJ substrate was added to 100 μL of R solution; the volume may be increased as required.
(8) Cells were resuspended in DNA buffer, and electroporation was initiated according to the following parameters:

| | |
|---|---|
| Pulse voltage (V) | 1600 |
| Pulse width (ms) | 10 |
| Pulse times | 3 |

(9) The cells were loaded into antibiotic-free culture medium and mixed gently.
(10) Steps (8)-(9) were repeated as necessary, and cells were seeded into 384-well plates at a density of 40,000 cells/well in 40 μL of culture medium.
(11) The plates were incubated in a humidified $CO_2$ incubator at 37°C for 24 hours.

(12) NanoGlo reagent was warmed to room temperature, and NanoGlo buffer was mixed with the substrate at a ratio of 50:1.
(13) The cell culture plates were equilibrated at room temperature for 10 minutes.
(14) 40 μL of NanoGlo substrate buffer was added to each well, followed by shaking at 300 rpm for 3 minutes protected from light.
(15) Signals were read via a BMG PHERAstar FSX.

Specific results are shown in Table 3.

Table 3: Inhibitory activity of compounds against the Theta-mediated end joining (TMEJ) process in HEK293T cells

| Compound No. | TMEJ in HEK293T $IC_{50}$ (nM) |
|---|---|
| Ref. 1 | 5.0 |
| cpd-4 | 1.0 |
| cpd-5 | 1.0 |
| cpd-8 | 2.0 |
| cpd-11 | 2.0 |
| cpd-16 | 2.0 |
| cpd-17 | 1.8 |
| cpd-18 | 1.3 |
| cpd-19 | 2.0 |
| cpd-21 | 2.3 |
| cpd-22 | 1.3 |

The data above show that the compounds of the embodiments of the present disclosure have good inhibitory activity against the Theta-mediated end joining (TMEJ) process in HEK293T cells, and are superior to the reference compound Ref. 1.

**Thermodynamic solubility determination:**

Experimental materials

(1) The control compound, diclofenac sodium, was purchased from Sigma.
(2) FaSSIF, FeSSIF & FaSSGF (FFF02) powders were purchased from Biorelevant.
(3) 1.5 mL flat-bottom glass vials (purchased from local suppliers); PTFE/silicone septa (BioTech Solutions); PTFE-coated stir bars (V&P Scientific); MultiScreenHTS HV (0.45 μm) 96-well plates (Millipore, MSHVN4510 or MSHVN4550); Eppendorf Thermomixer Comfort; Vacuum Manifold ORVMN96 (Orochem).

Experimental method:

(1) Preparation of FaSSIF

a. Preparation of Buffer A:

0.420 g of NaOH, 3.438 g of $NaH_2PO_4$, and 6.186 g of NaCl were dissolved in approximately 900 mL of ultrapure water, and the pH of the solution was adjusted to 6.5 using 1N NaOH or 1N HCl. The solution was then diluted to 1000 mL with ultrapure water at room temperature.

b. Addition of powder:

2.240 g of FaSSIF, FeSSIF, and FaSSGF powder was added to approximately 500 mL of Buffer A. The mixture was stirred until the powder was completely dissolved. The solution was then diluted to 1000 mL with Buffer A at room

temperature.

c. Preparation for use

The solution was allowed to stand for 2 hours. The solution became slightly opalescent and was ready for use at that point. Note: it was to be used within 48 hours at room temperature or within 24 hours at 37°C.
(2) The test compounds and control compounds were weighed for solubility measurement (approximately 1.0 mg each in three separate 1.5 mL glass vials).
(3) For each compound, one vial served as a standard, and the other two vials were used for solubility measurement in duplicate.
(4) Each compound was placed sequentially into its appropriate 96-well plate. One plate served as the standard plate and the other as the sample plate.
(5) Based on the mass of the sample in each vial on the sample plate, an appropriate volume (approximately 1000 μL) of FaSSIF was added to each vial of the solubility sample plate using a pipette. A stir bar was added to each vial, and the vials were sealed with PTFE/silicone septa.
(6) The solubility sample plate was transferred to an Eppendorf Thermomixer Comfort shaker and shaken at 37°C and 1100 rpm for 24 hours.
(7) Upon completion of the 24-hour period, the septa were removed, the stir bars were removed using a large magnet, and the samples were transferred from the solubility sample plate to a filter plate. The samples were filtered using a vacuum pump. The filtrate was diluted 1000-fold with a mixture of water and acetonitrile (1:1 v/v) (specifically, a 5 μL aliquot of the filtrate was transferred to a new plate, followed by the addition of 5 μL of DMSO and 490 μL of a 1:1 v/v water/acetonitrile mixture; after thorough mixing, a 50 μL aliquot of the diluted sample was transferred to another new plate, and 450 μL of a 1:1 v/v water/acetonitrile mixture was added). 200 μL of the diluted sample was transferred to a new 96-well plate for LC-MS/MS analysis. The dilution factor may be adjusted based on solubility values and LC-MS signal responses.
(8) For the standards, each standard was dissolved to a concentration of 1.0 mg/mL based on the mass of the sample in each vial on the standard plate. An appropriate volume of DMSO was added to each standard vial using a pipette.
(9) The standard vials were covered with a 96-well plate lid, and the standard plate was placed in an Eppendorf Thermomixer Comfort and shaken at 25°C and 1100 rpm for 5 minutes.
(10) After 5 minutes, each compound should be completely dissolved.
(11) A 5 μL aliquot was taken from the 1.0 mg/mL DMSO standard plate and transferred to a new plate. Then, 5 μL of FaSSIF and 490 μL of a mixture of water and acetonitrile (1:1 v/v) were added to obtain a standard at a concentration of 10 μg/mL. From the 10 μg/mL standard plate, a 50 μL aliquot was transferred to a new plate, and 450 μL of a mixture of water and acetonitrile (1:1 v/v) was added to obtain a standard at a concentration of 1 μg/mL. 200 μL of the diluted sample was transferred to a new 96-well plate for LC-MS/MS analysis. The concentration of the standards may be adjusted according to LC-MS signal responses.
(12) The solubility sample plate and the standard plate were placed in an autosampler at 4°C and evaluated by LC-MS/MS analysis.

Data analysis:

All calculations were performed using Microsoft Excel. Analysis and quantification of the sample filtrates were completed via LC-MS/MS by qualitative and quantitative comparison against the peaks of standards with known concentrations. The formula for calculating the solubility values of the control drug and the test substances is as follows:

$$[Sample] = \frac{AREA_{Sample} \times INJ\,VOL_{Std} \times DF_{Sample} \times [STD]}{AREA_{Std} \times INJ\,VOL_{Sample}}$$

DF refers to the dilution factor.
Specific results are shown in Table 4.

Table 4: Thermodynamic solubility of compounds

| Compound No. | FaSSIF (pH 6.5) solubility (μg/mL) |
|---|---|
| Ref. 1 | 31 |
| cpd-16 | 669 |

(continued)

| Compound No. | FaSSIF (pH 6.5) solubility (μg/mL) |
|---|---|
| cpd-17 | 511 |

The data above show that the thermodynamic solubility of the compounds of the embodiments of the present disclosure is significantly superior to that of reference compound Ref. 1.

**Pharmacokinetic study in rats:**

1. Experimental objective: To test the pharmacokinetics of the compounds in *in vivo* experiments using Wistar-Han rats.
2. Experimental instruments and materials:

Animal: Wistar-Han rats (male)

Instrument:

| Instrument name | Manufacturer | Instrument No. |
|---|---|---|
| API4000 Qtrap-Shimadzu 20AT | AB Sciex/Shimadzu | TW-017-0002 |
| Large benchtop refrigerated centrifuge | Eppendorf | TW-007-0017 |
| Electronic balance | Sartorius | TW-001-0013 |
| Liquid handling workstation | Sptlabtech | TW-013-0016 |
| Vortex shaker | DRAGON LAB | TW-005-0013 |
| High-Throughput tissue grinder | Ningbo Scientz Biotechnology Co., Ltd. | TW-011-0002 |

Reagent:

| Name | Manufacturer | Cat. No. | Batch No. |
|---|---|---|---|
| Acetonitrile | Sigma-Aldrich | 34851 | WXBD8733V |
| Methanol | Sigma-Aldrich | 75851G | P2478457 |
| DMSO | Fisher Scientific | D4540 | BCCH3267 |
| Formic acid | Sigma-Aldrich | F112034 | B2320164 |
| PBS | Shanghai Basal Media | B310KJ | K211007 |

Vehicle:

Oral gavage: 1% methylcellulose aqueous solution.

3. Experimental method:

Six male rats were used. On the evening prior to administration, the animals were fasted, with free access to water. During the experimental period, mice had free access to food and water. The animals were administered either by intravenous injection or intragastric gavage, after which the status of the animals was observed and any abnormal manifestations were recorded. Blood was collected via jugular vein puncture at 0.0833, 0.25, 1, 2, 4, 8, and 24 hours following intravenous administration; and at 0.25, 0.5, 1, 2, 4, 6, 8, and 24 hours following intragastric gavage. A 50 μL plasma sample was taken, and 500 μL of acetonitrile (containing dexamethasone as an internal standard) was added to precipitate proteins. The mixture was centrifuged at 4000 rpm for 20 minutes at 4°C. 300 μL of the supernatant was

transferred to a 96-well plate and mixed with 300 μL of Watson water, after which a 5 μL aliquot was used for LC-MS/MS detection.

4. Data processing method and results:

A standard curve was established using the internal standard method, with the theoretical concentrations of the standard curve as the abscissa and the peak area ratio (peak area of test compound / peak area of internal standard) as the ordinate. Linear regression analysis was performed (weighting factor 1/X2) with R2>0.9900. Unknown samples were calculated via the standard curve. Pharmacokinetic parameters were calculated using the non-compartmental analysis (NCA) model of WinNonlin 8.2 software and presented in the report. Parameters include $T_{1/2}$, Tmax, Cmax, and AUC, etc.
Specific results are shown in Table 5.

Table 5: Rat PK of compounds

| Compound | Dose (mg/kg) | $C_{max}$ (μg/mL) | $AUC_{0-t}$ (μg*hr/mL) |
|---|---|---|---|
| cpd-17 | 30 | 8.3 | 11.7 |

The compounds of the embodiments of the present disclosure exhibit high exposure in rats, and the AUC thereof is significantly superior to that of the prodrug compound Example 1 ($AUC_{0-t}$ 8.5 μg*hr/mL) reported in WO 2023233295 A1.
Example 1 represents

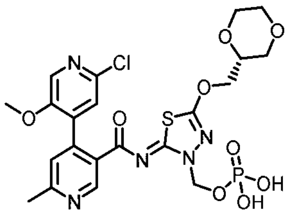
,

which is disclosed in WO 2023233295 A1.

## Claims

1. A compound of formula (I), or an isotopically labeled compound, enantiomer, diastereoisomer, or solvate thereof, or a pharmaceutically acceptable salt thereof, wherein the compound of formula (I) is selected from the following structures:

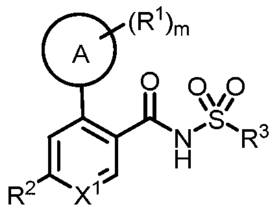

(I)

wherein

$X^1$ is CH or N;
ring A is phenyl or a six-membered heteroaryl containing 1 to 3 N atoms;
m is 0, 1, 2, 3, or 4;
each $R^1$ is independently selected from deuterium, halogen, C1-C6 alkyl, C3-C6 cycloalkyl, C1-C6 haloalkyl, deuterated C1-C6 alkyl, C1-C6 alkoxy, C3-C6 cycloalkoxy, C1-C6 haloalkoxy, deuterated C1-C6 alkoxy, hydroxyl, cyano, C2-C6 alkynyl, and $-NR^{1x}R^{1y}$; $R^{1x}$ and $R^{1y}$ are each independently H, C1-C6 alkyl, C1-C6 haloalkyl, or C3-C6 cycloalkyl;
$R^2$ is selected from hydrogen, cyano, hydroxyl, $-NR^{2x}R^{2y}$, $-CONR^{2x}R^{2y}$, $-NR^{2x}COR^{2y}$, C1-C6 alkyl unsubstituted

or substituted with one or more substituents selected from Group A, C1-C6 alkoxy unsubstituted or substituted with one or more substituents selected from Group A, C2-C6 alkenyl unsubstituted or substituted with one or more substituents selected from Group A, C2-C6 alkynyl unsubstituted or substituted with one or more substituents selected from Group A, 3- to 8-membered heterocyclyl unsubstituted or substituted with one or more substituents selected from Group A, C3-C6 cycloalkyl unsubstituted or substituted with one or more substituents selected from Group A, C6-C10 aryl unsubstituted or substituted with one or more substituents selected from Group A, and 5- to 6-membered heteroaryl unsubstituted or substituted with one or more substituents selected from Group A;

the substituents of Group A comprise: oxo, deuterium, halogen, C1-C6 alkyl, C3-C6 cycloalkyl unsubstituted or substituted with one or more substituents selected from Group A1, C6-C10 aryl unsubstituted or substituted with one or more substituents selected from Group A1, 3- to 8-membered heterocyclyl unsubstituted or substituted with one or more substituents selected from Group A1, 5- to 6-membered heteroaryl unsubstituted or substituted with one or more substituents selected from Group A1, C1-C6 haloalkyl, deuterated C1-C6 alkyl, C1-C6 alkoxy, C3-C6 cycloalkoxy, C1-C6 haloalkoxy, deuterated C1-C6 alkoxy, hydroxyl, cyano, - $NR^{3x}R^{3y}$, -$CONR^{3x}R^{3z}$, and -$NR^{3x}COR^{3z}$;

the substituents of Group A1 comprise: halogen, hydroxyl, cyano, C1-C6 alkyl, C1-C6 haloalkyl, deuterated C1-C6 alkyl, C1-C6 alkoxy, and C1-C6 haloalkoxy;

$R^{2x}$, $R^{2y}$, $R^{3x}$, $R^{3y}$, and $R^{3z}$ are each independently H, C1-C6 alkyl, C1-C6 haloalkyl, deuterated C1-C6 alkyl, or C3-C6 cycloalkyl;

or $R^{2x}$ and $R^{2y}$, together with the N atom to which they are attached, form a 4- to 6-membered heterocyclyl containing 1 to 2 N atoms; said 4- to 6-membered heterocyclyl may be unsubstituted or substituted with one or more substituents selected from oxo, C1-C6 alkyl, C3-C6 cycloalkyl, hydroxyl, amino, halogen, and cyano;

or $R^{3x}$ and $R^{3y}$, together with the N atom to which they are attached, form a 4- to 6-membered heterocyclyl containing 1 to 2 N atoms; said 4- to 6-membered heterocyclyl may be unsubstituted or substituted with one or more substituents selected from oxo, C1-C6 alkyl, C3-C6 cycloalkyl, hydroxyl, amino, halogen, and cyano;

$R^3$ is -$(CR_4R_5)_nR^6$;

$R^4$ and $R^5$ are each independently selected from hydrogen, deuterium, halogen, C1-C6 alkyl, deuterated C1-C6 alkyl, and C1-C6 haloalkyl;

n is 0, 1, 2, or 3;

$R^6$ is C1-C6 alkyl unsubstituted or substituted with one or more substituents selected from Group B, 3- to 8-membered heterocyclyl unsubstituted or substituted with one or more substituents selected from Group B, 5- to 10-membered heteroaryl unsubstituted or substituted with one or more substituents selected from Group B, C3-C6 cycloalkyl unsubstituted or substituted with one or more substituents selected from Group B, or C6-C10 aryl unsubstituted or substituted with one or more substituents selected from Group B;

the substituents of Group B comprise: oxo, deuterium, halogen, hydroxyl, cyano, mercapto, C2-C6 alkenyl, C2-C6 alkynyl, C1-C6 alkyl unsubstituted or substituted with one or more substituents selected from Group C, C1-C6 alkoxy unsubstituted or substituted with one or more substituents selected from Group C, -$(CH_2)_iNR^7R^8$, -$(CH2)_iCONR^7R^8$, -$(CH_2)_iNR^7COR^8$, -$(CH_2)_iSO_2R^7$, -$(CH_2)_iSO_2NR^7R^8$, -$(CH_2)_iOR^7$, C6-C10 aryl, C3-C6 cycloalkyl, 3- to 8-membered heterocyclyl, and 5- to 10-membered heteroaryl;

i is 0, 1, 2, or 3;

$R^7$ and $R^8$ are each independently H, C1-C6 alkyl, C1-C6 haloalkyl, deuterated C1-C6 alkyl, or C3-C6 cycloalkyl;

the substituents of Group C comprise: deuterium, halogen, hydroxyl, cyano, amino, C1-C6 alkoxy, C3-C6 cycloalkyl, and deuterated C1-C6 alkoxy;

the heteroatoms in the 3- to 8-membered heterocyclyl, 5- to 10-membered heteroaryl, and 5- to 6-membered heteroaryl are independently selected from 1, 2, or 3 of N, O, and S.

2. The compound of formula (I) according to claim 1, or an isotopically labeled compound, enantiomer, diastereoisomer, or solvate thereof, or a pharmaceutically acceptable salt thereof, wherein
ring A is phenyl or pyridyl.

3. The compound of formula (I) according to claim 1, or an isotopically labeled compound, enantiomer, diastereoisomer, or solvate thereof, or a pharmaceutically acceptable salt thereof, wherein

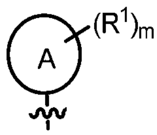

is

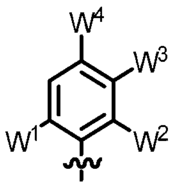, 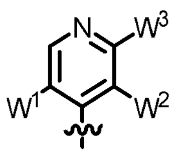, 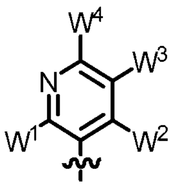, or 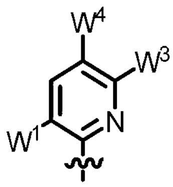;

wherein $W^1$, $W^2$, $W^3$, and $W^4$ are each independently selected from hydrogen, deuterium, halogen, C1-C6 alkyl, C3-C6 cycloalkyl, C1-C6 haloalkyl, deuterated C1-C6 alkyl, C1-C6 alkoxy, C3-C6 cycloalkoxy, C1-C6 haloalkoxy, deuterated C1-C6 alkoxy, hydroxyl, cyano, C2-C6 alkynyl, and $-NR^{1x}R^{1y}$; $R^{1x}$ and $R^{1y}$ are each independently H, C1-C6 alkyl, C1-C6 haloalkyl, or C3-C6 cycloalkyl;
preferably,

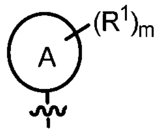

is

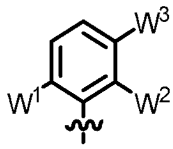 or 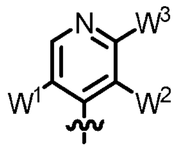;

$W^1$, $W^2$, and $W^3$ are each independently selected from hydrogen, deuterium, halogen, C1-C6 alkyl, C3-C6 cycloalkyl, C1-C6 haloalkyl, deuterated C1-C6 alkyl, C1-C6 alkoxy, C3-C6 cycloalkoxy, C1-C6 haloalkoxy, deuterated C1-C6 alkoxy, hydroxyl, cyano, C2-C6 alkynyl, and $-NR^{1x}R^{1y}$; $R^{1x}$ and $R^{1y}$ are each independently H, C1-C6 alkyl, C1-C6 haloalkyl, or C3-C6 cycloalkyl.

**4.** The compound of formula (I) according to claim 1, or an isotopically labeled compound, enantiomer, diastereoisomer, or solvate thereof, or a pharmaceutically acceptable salt thereof, wherein

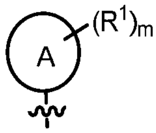

is

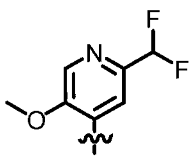, 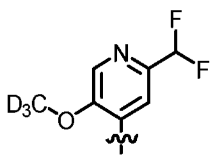, 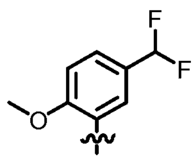, 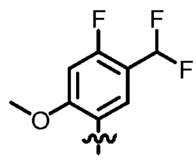,

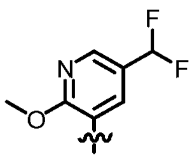, 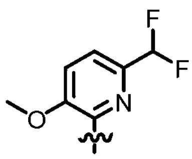, 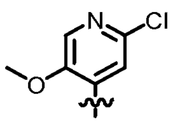, 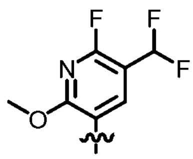, or 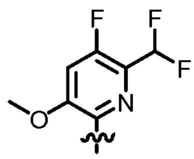;

preferably,

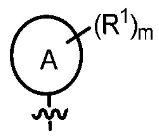

is

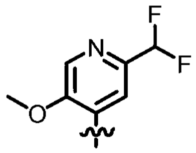
or
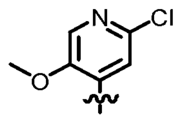
.

**5.** The compound of formula (I) according to claim 1, or an isotopically labeled compound, enantiomer, diastereoisomer, or solvate thereof, or a pharmaceutically acceptable salt thereof, wherein

$R^2$ is selected from C1-C6 alkyl unsubstituted or substituted with one or more substituents selected from Group A, C1-C6 alkoxy unsubstituted or substituted with one or more substituents selected from Group A, C2-C6 alkynyl unsubstituted or substituted with one or more substituents selected from Group A, 3- to 8-membered heterocyclyl unsubstituted or substituted with one or more substituents selected from Group A, C3-C6 cycloalkyl unsubstituted or substituted with one or more substituents selected from Group A, C6-C10 aryl unsubstituted or substituted with one or more substituents selected from Group A, and 5- to 6-membered heteroaryl unsubstituted or substituted with one or more substituents selected from Group A;
the substituents of Group A comprise: oxo, deuterium, halogen, C1-C6 alkyl, C3-C6 cycloalkyl unsubstituted or substituted with one or more substituents selected from Group A1, C6-C10 aryl unsubstituted or substituted with one or more substituents selected from Group A1, 3- to 8-membered heterocyclyl unsubstituted or substituted with one or more substituents selected from Group A1, 5- to 6-membered heteroaryl unsubstituted or substituted with one or more substituents selected from Group A1, C1-C6 haloalkyl, deuterated C1-C6 alkyl, C1-C6 alkoxy, deuterated C1-C6 alkoxy, C3-C6 cycloalkoxy, C1-C6 haloalkoxy, hydroxyl, cyano, - $NR^{3x}R^{3y}$, $-CONR^{3x}R^{3z}$, and $-NR^{3x}COR^{3z}$;
the substituents of Group A1 comprise: halogen, hydroxyl, cyano, C1-C6 alkyl, C1-C6 haloalkyl, deuterated C1-C6 alkyl, C1-C6 alkoxy, C1-C6 haloalkoxy; $R^{3x}$, $R^{3y}$, and $R^{3z}$ are each independently H, C1-C6 alkyl, C1-C6 haloalkyl, deuterated C1-C6 alkyl, or C3-C6 cycloalkyl;
or $R^{3x}$ and $R^{3y}$, together with the N atom to which they are attached, form a 4- to 6-membered heterocyclyl containing 1 to 2 N atoms; said 4- to 6-membered heterocyclyl may be unsubstituted or substituted with one or more substituents selected from oxo, C1-C6 alkyl, C3-C6 cycloalkyl, hydroxyl, amino, halogen, and cyano.

**6.** The compound of formula (I) according to claim 1, or an isotopically labeled compound, enantiomer, diastereoisomer, or solvate thereof, or a pharmaceutically acceptable salt thereof, wherein

$R^2$ is selected from C1-C6 alkyl unsubstituted or substituted with one or more substituents selected from Group A, C1-C6 alkoxy unsubstituted or substituted with one or more substituents selected from Group A, C2-C6 alkynyl unsubstituted or substituted with one or more substituents selected from Group A,

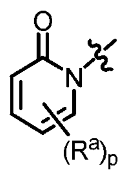
,
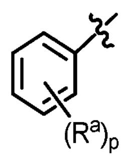
,
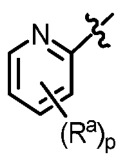
,
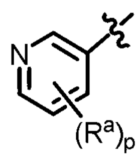
,

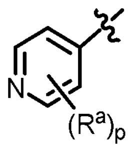 , 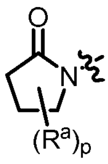 , 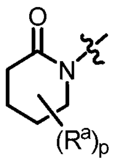 , 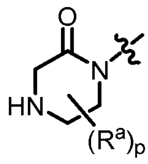 , 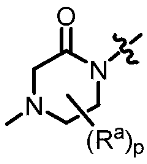 , 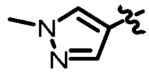 , 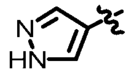 ,

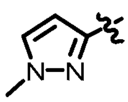 , 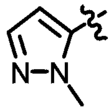 , 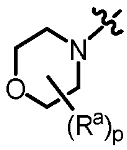 , 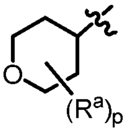 , 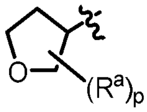 , 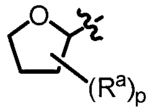 , and 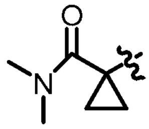 ;

wherein

the substituents of Group A comprise: deuterium, halogen, C1-C6 alkyl, C3-C6 cycloalkyl unsubstituted or substituted with one or more substituents selected from Group A1, C6-C10 aryl unsubstituted or substituted with one or more substituents selected from Group A1, 3- to 8-membered heterocyclyl unsubstituted or substituted with one or more substituents selected from Group A1, 5- to 6-membered heteroaryl unsubstituted or substituted with one or more substituents selected from Group A1, C1-C6 haloalkyl, deuterated C1-C6 alkyl, C1-C6 alkoxy, deuterated C1-C6 alkoxy, C3-C6 cycloalkoxy, C1-C6 haloalkoxy, hydroxyl, cyano, $-NR^{3x}R^{3y}$ $-CONR^{3x}R^{3z}$, and $-NR^{3x}COR^{3z}$;

the substituents of Group A1 comprise: halogen, hydroxyl, cyano, C1-C6 alkyl, C1-C6 haloalkyl, deuterated C1-C6 alkyl, C1-C6 alkoxy, and C1-C6 haloalkoxy; $R^{3x}$, $R^{3y}$, and $R^{3z}$ are each independently H, C1-C6 alkyl, C1-C6 haloalkyl, deuterated C1-C6 alkyl, or C3-C6 cycloalkyl;

or $R^{3x}$ and $R^{3y}$, together with the N atom to which they are attached, form a 4- to 6-membered heterocyclyl containing 1 to 2 N atoms; said 4- to 6-membered heterocyclyl may be unsubstituted or substituted with one or more substituents selected from oxo, C1-C6 alkyl, C3-C6 alkyl, hydroxyl, amino, halogen, and cyano;

$R^a$ is selected from hydrogen, halogen, hydroxyl, amino, cyano, C1-C6 alkyl, C1-C6 haloalkoxy, and C1-C6 alkoxy;

p is 1, 2, or 3.

**7.** The compound of formula (I) according to claim 1, or an isotopically labeled compound, enantiomer, diastereoisomer, or solvate thereof, or a pharmaceutically acceptable salt thereof, wherein

$R^2$ is selected from C1-C6 alkyl, C1-C6 haloalkyl, deuterated C1-C6 alkyl, C1-C6 alkoxy, C1-C6 alkoxy-substituted C1-C6 alkyl,

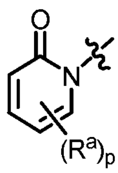 , 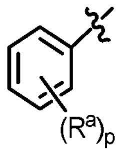 , 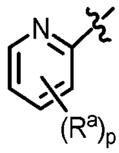 , 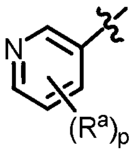 ,

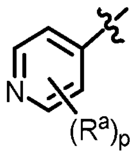 , 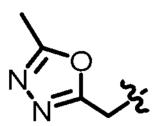 , and 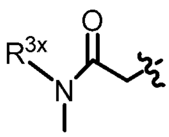 ;

wherein

$R^a$ is selected from hydrogen, C1-C6 alkyl, C1-C6 haloalkyl, or C1-C6 alkoxy; p is 1, 2, or 3; $R^{3x}$ is H, C1-C6 alkyl, C1-C6 haloalkyl, deuterated C1-C6 alkyl, or C3-C6 cycloalkyl.

**8.** The compound of formula (I) according to claim 1, or an isotopically labeled compound, enantiomer, diastereoisomer, or solvate thereof, or a pharmaceutically acceptable salt thereof, wherein
$R^2$ is selected from methyl, methoxy, $-CF_3$, $-CD_3$, $-CH_2CF_3$, $-CH_2OCH_3$,

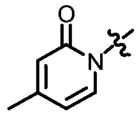

,

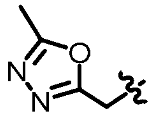, 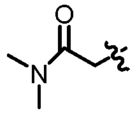, and 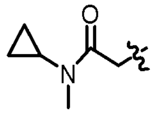.

9. The compound of formula (I) according to claim 1, or an isotopically labeled compound, enantiomer, diastereoisomer, or solvate thereof, or a pharmaceutically acceptable salt thereof, wherein

$R^3$ is $-(CH_2)_nR^6$;
n is 0, 1, 2, or 3;
$R^6$ is 3- to 8-membered heterocyclyl unsubstituted or substituted with one or more substituents selected from Group B, 5- to 10-membered heteroaryl unsubstituted or substituted with one or more substituents selected from Group B, C3-C6 cycloalkyl unsubstituted or substituted with one or more substituents selected from Group B, or C6-C10 aryl unsubstituted or substituted with one or more substituents selected from Group B;
the substituents of Group B comprise: oxo, deuterium, halogen, hydroxyl, cyano, mercapto, C2-C6 alkenyl, C2-C6 alkynyl, C1-C6 alkyl unsubstituted or substituted with one or more substituents selected from Group C, C1-C6 alkoxy unsubstituted or substituted with one or more substituents selected from Group C, $-(CH_2)_iNR^7R^8$, $-(CH2)_iCONR^7R^8$, $-(CH_2)_iNR^7COR^8$, $-(CH_2)_iSO_2R^7$, $-(CH_2)_iSO_2NR^7R^8$, and $-(CH_2)_iOR^7$.
i is 0, 1, 2, or 3;
$R^7$ and $R^8$ are each independently H, C1-C6 alkyl, C1-C6 haloalkyl, deuterated C1-C6 alkyl, or C3-C6 cycloalkyl;
the substituents of Group C comprise: deuterium, halogen, hydroxyl, cyano, amino, C1-C6 alkoxy, C3-C6 cycloalkyl, and deuterated C1-C6 alkoxy;
the heteroatoms in the 5- to 10-membered heteroaryl and 5- to 6-membered heteroaryl are independently selected from 1, 2, or 3 of N, O, and S.

10. The compound of formula (I) according to claim 1, or an isotopically labeled compound, enantiomer, diastereoisomer, or solvate thereof, or a pharmaceutically acceptable salt thereof, wherein $R^3$ is selected from -C1-C3 alkyl,

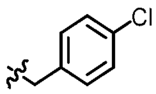, 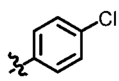,

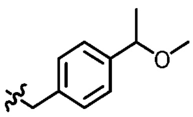, 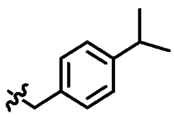, 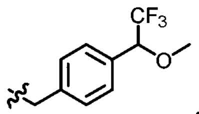, 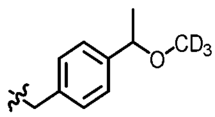, and 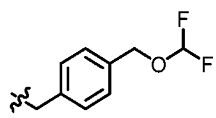.

11. The compound of formula (I) according to claim 1, or an isotopically labeled compound, enantiomer, diastereoisomer, or solvate thereof, or a pharmaceutically acceptable salt thereof, wherein the compound of formula (I) is selected from the following structures:

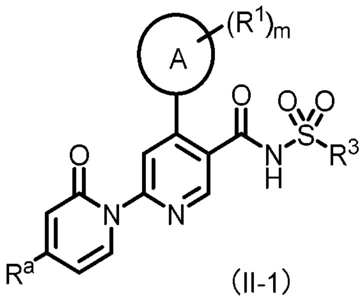
(II-1)

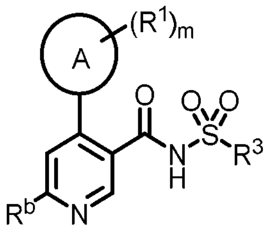
(II-2)

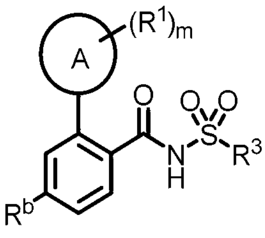
(II-3)

wherein

$R^a$ is selected from hydrogen, halogen, hydroxyl, amino, cyano, C1-C6 alkyl, C1-C6 haloalkyl, and C1-C6 alkoxy; $R^b$ is selected from C1-C6 alkyl unsubstituted or substituted with one or more substituents selected from Group A and C1-C6 alkoxy unsubstituted or substituted with one or more substituents selected from Group A; the substituents of Group A comprise: deuterium, halogen, C1-C6 alkyl, C1-C6 haloalkyl, deuterated C1-C6 alkyl, C1-C6 alkoxy, C1-C6 haloalkoxy, deuterated C1-C6 alkoxy, hydroxyl, cyano, amino, $-CONR^{3x}R^{3z}$, $-NR^{3x}COR^{3z}$, and unsubstituted or C1-C6 alkyl-substituted 5- to 6-membered heteroaryl;
ring A, $R^1$, $R^3$, $R^{3x}$, $R^{3z}$, and m are as defined in claim 1.

**12.** The compound of formula (I) according to claim 1, or an isotopically labeled compound, enantiomer, diastereoisomer, or solvate thereof, or a pharmaceutically acceptable salt thereof, wherein the compound of formula (I) is selected from the following structures:

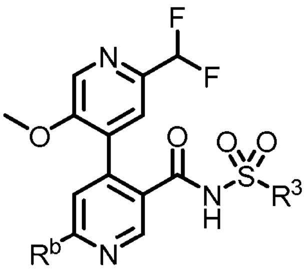

(III-1)

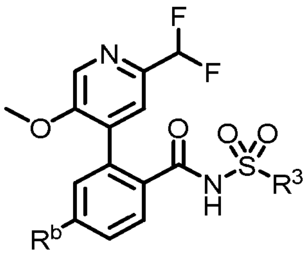

(III-2)

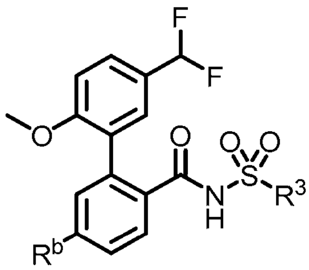

(III-3)

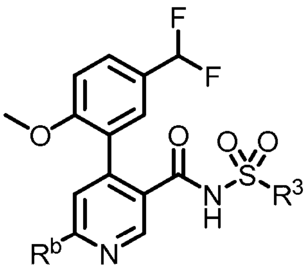

(III-4)

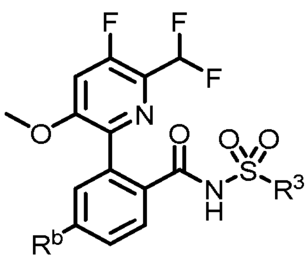

(III-5)

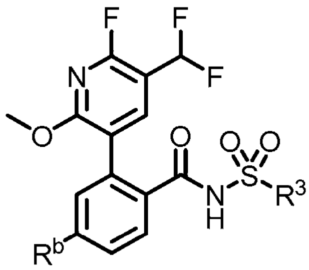

(III-6)

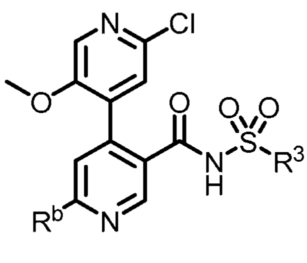

(III-7)

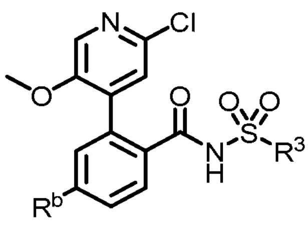

(III-8)

wherein

$R^b$ is selected from C1-C6 alkyl unsubstituted or substituted with one or more substituents selected from Group A and C1-C6 alkoxy unsubstituted or substituted with one or more substituents selected from Group A; the substituents of Group A comprise: deuterium, halogen, C1-C6 alkyl, C1-C6 haloalkyl, deuterated C1-C6 alkyl, C1-C6 alkoxy, C1-C6 haloalkoxy, deuterated C1-C6 alkoxy, hydroxyl, cyano, amino, $-CONR^{3x}R^{3z}$, $-NR^{3x}COR^{3z}$, and unsubstituted or C1-C6 alkyl-substituted 5- to 6-membered heteroaryl; $R^{3x}$ and $R^{3z}$ are each independently H, C1-C6 alkyl, C1-C6 haloalkyl, deuterated C1-C6 alkyl, or C3-C6 cycloalkyl;
$R^3$ is $-(CR_4R_5)_nR^6$;

$R^4$ and $R^5$ are each independently selected from hydrogen, deuterium, halogen, C1-C6 alkyl, deuterated C1-C6 alkyl, and C1-C6 haloalkyl;
n is 0, 1, 2, or 3;
$R^6$ is C1-C6 alkyl unsubstituted or substituted with one or more substituents selected from Group B, 3- to 8-membered heterocyclyl unsubstituted or substituted with one or more substituents selected from Group B, 5- to 10-membered heteroaryl unsubstituted or substituted with one or more substituents selected from Group B, C3-C6 cycloalkyl unsubstituted or substituted with one or more substituents selected from Group B, or C6-C10 aryl unsubstituted or substituted with one or more substituents selected from Group B;
the substituents of Group B comprise: oxo, deuterium, halogen, hydroxyl, cyano, mercapto, C2-C6 alkenyl, C2-C6 alkynyl, C1-C6 alkyl unsubstituted or substituted with one or more substituents selected from Group C, C1-C6 alkoxy unsubstituted or substituted with one or more substituents selected from Group C, $-(CH_2)_iNR^7R^8$, $-(CH2)_iCONR^7R^8$, $-(CH_2)_iNR^7COR^8$, $-(CH_2)_iSO_2R^7$, $-(CH_2)_iSO_2NR^7R^8$, $-(CH_2)_iOR^7$, C6-C10 aryl, C3-C6 cycloalkyl, 3- to 8-membered heterocyclyl, and 5- to 10-membered heteroaryl;
i is 0, 1, 2, or 3;
$R^7$ and $R^8$ are each independently H, deuterium, C1-C6 alkyl, C1-C6 haloalkyl, or C3-C6 cycloalkyl;
the substituents of Group C comprise: deuterium, halogen, hydroxyl, cyano, amino, C1-C6 alkoxy, C3-C6 cycloalkyl, and deuterated C1-C6 alkoxy;
the heteroatoms in the 3- to 8-membered heterocyclyl and 5- to 10-membered heteroaryl are independently selected from 1, 2, or 3 of N, O, and S.

**13.** The compound of formula (I) according to claim 12, or an isotopically labeled compound, enantiomer, diastereoisomer, or solvate thereof, or a pharmaceutically acceptable salt thereof, wherein

$R^b$ is selected from C1-C6 alkyl unsubstituted or substituted with one or more substituents selected from Group A and C1-C6 alkoxy unsubstituted or substituted with one or more substituents selected from Group A; the substituents of Group A comprise: deuterium, halogen, C1-C6 alkyl, C1-C6 haloalkyl, deuterated C1-C6 alkyl, C1-C6 alkoxy, deuterated C1-C6 alkoxy, hydroxyl, cyano, amino, $-CONR^{3x}R^{3z}$, $-NR^{3x}COR^{3z}$, and unsubstituted or C1-C6 alkyl-substituted 5- to 6-membered heteroaryl; $R^{3x}$ and $R^{3z}$ are each independently H, C1-C6 alkyl, C1-C6 haloalkyl, deuterated C1-C6 alkyl, or C3-C6 cycloalkyl;
$R^3$ is $-(CR_4R_5)_nR^6$;
$R^4$ and $R^5$ are each independently selected from hydrogen, deuterium, halogen, and methyl;
n is 0, 1, 2, or 3;
$R^6$ is C1-C6 alkyl unsubstituted or substituted with one or more substituents selected from Group B, 3- to 8-membered heterocyclyl unsubstituted or substituted with one or more substituents selected from Group B, 5- to 10-membered heteroaryl unsubstituted or substituted with one or more substituents selected from Group B, C3-C6 cycloalkyl unsubstituted or substituted with one or more substituents selected from Group B, or C6-C10 aryl unsubstituted or substituted with one or more substituents selected from Group B;
the substituents of Group B comprise: oxo, deuterium, halogen, hydroxyl, cyano, mercapto, C2-C6 alkenyl, C2-C6 alkynyl, C1-C6 alkyl unsubstituted or substituted with one or more substituents selected from Group C, C1-C6 alkoxy unsubstituted or substituted with one or more substituents selected from Group C, $-(CH_2)_iNR^7R^8$, $-(CH_2)_iCONR^7R^8$, $-(CH_2)_iNR^7COR^8$, $-(CH_2)_iSO_2R^7$, $-(CH_2)_iSO_2NR^7R^8$, and $-(CH_2)_iOR^7$;
i is 0, 1, 2, or 3;
$R^7$ and $R^8$ are each independently H, deuterium, C1-C6 alkyl, C1-C6 haloalkyl, or C3-C6 cycloalkyl;
the substituents of Group C comprise: deuterium, halogen, hydroxyl, cyano, amino, C1-C6 alkoxy, C3-C6 cycloalkyl, and deuterated C1-C6 alkoxy;
the heteroatoms in the 3- to 8-membered heterocyclyl and 5- to 10-membered heteroaryl are independently selected from 1, 2, or 3 of N, O, and S.

**14.** The compound of formula (I) according to claim 12, or an isotopically labeled compound, enantiomer, diastereoisomer, or solvate thereof, or a pharmaceutically acceptable salt thereof, wherein

$R^b$ is selected from C1-C6 alkyl unsubstituted or substituted with one or more substituents selected from Group A and C1-C6 alkoxy unsubstituted or substituted with one or more substituents selected from Group A; the substituents of Group A comprise: deuterium, halogen, C1-C6 alkyl, C1-C6 alkoxy, $-CONR^{3x}R^{3z}$, $-NR^{3x}COR^{3z}$, and unsubstituted or C1-C6 alkyl-substituted 5- to 6-membered heteroaryl; $R^{3x}$ and $R^{3z}$ are each independently H, C1-C6 alkyl, C1-C6 haloalkyl, deuterated C1-C6 alkyl, or C3-C6 cycloalkyl;
$R^3$ is $-(CH_2)_nR^6$;
n is 0, 1, or 2;

$R^6$ is C1-C6 alkyl unsubstituted or substituted with one or more substituents selected from Group B, 3- to 6-membered heterocyclyl unsubstituted or substituted with one or more substituents selected from Group B, 6-membered heteroaryl unsubstituted or substituted with one or more substituents selected from Group B, C3-C6 cycloalkyl unsubstituted or substituted with one or more substituents selected from Group B, or phenyl unsubstituted or substituted with one or more substituents selected from Group B;
the substituents of Group B comprise: deuterium, halogen, hydroxyl, cyano, C1-C6 alkyl unsubstituted or substituted with one or more substituents selected from Group C, C1-C6 alkoxy, C1-C6 haloalkoxy, deuterated C1-C6 alkoxy, amino, and $-(CH_2)_iOR^7$; the substituents of Group C comprise: deuterium, halogen, hydroxyl, cyano, amino, C1-C6 alkoxy, C3-C6 cycloalkyl, deuterated C1-C6 alkoxy; i is 1 or 2; $R^7$ is C1-C6 haloalkyl;
the heteroatoms in the 3- to 6-membered heterocyclyl and 6-membered heteroaryl are independently selected from 1, 2, or 3 of N, O, and S.

**15.** The compound of formula (I) according to claim 12, or an isotopically labeled compound, enantiomer, diastereoisomer, or solvate thereof, or a pharmaceutically acceptable salt thereof, wherein

$R^b$ is selected from C1-C6 alkyl unsubstituted or substituted with one or more substituents selected from Group A and C1-C6 alkoxy unsubstituted or substituted with one or more substituents selected from Group A; the substituents of Group A comprise: deuterium, halogen, C1-C6 alkyl, C1-C6 alkoxy, $-CONR^{3x}R^{3z}$, and $-NR^{3x}COR^{3z}$; $R^{3x}$ and $R^{3z}$ are each independently H, C1-C6 alkyl, C1-C6 haloalkyl, deuterated C1-C6 alkyl, or C3-C6 cycloalkyl;
$R^3$ is $-(CH_2)_nR^6$;
n is 0 or 1;
$R^6$ is C1-C6 alkyl unsubstituted or substituted with one or more substituents selected from Group B, 3- to 6-membered heterocyclyl unsubstituted or substituted with one or more substituents selected from Group B, 6-membered heteroaryl unsubstituted or substituted with one or more substituents selected from Group B, C3-C6 cycloalkyl unsubstituted or substituted with one or more substituents selected from Group B, or phenyl unsubstituted or substituted with one or more substituents selected from Group B;
the substituents of Group B comprise: deuterium, halogen, hydroxyl, cyano, C1-C6 alkyl, C1-C6 haloalkyl, deuterated C1-C6 alkyl,

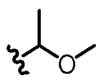, 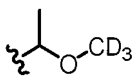, 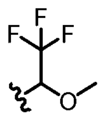, and 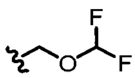;

the heteroatoms in the 3- to 6-membered heterocyclyl and 6-membered heteroaryl are independently selected from 1, 2, or 3 of N, O, and S.

**16.** The compound of formula (I) according to any one of claims 1 to 10, or an isotopically labeled compound, enantiomer, diastereoisomer, or solvate thereof, or a pharmaceutically acceptable salt thereof, wherein the compound of formula (I) is selected from the following structures:

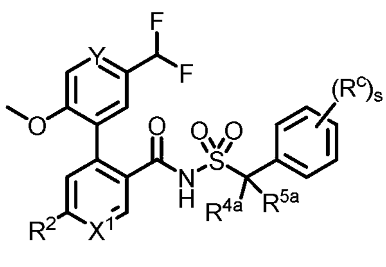

(IV-1)

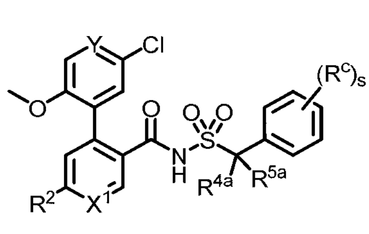

(IV-1a)

wherein

$X^1$ and Y are each independently N or CH;
$R^{4a}$ and $R^{5a}$ are each independently selected from hydrogen, deuterium, C1-C6 alkyl, and deuterated C1-C6 alkyl;

s is 0, 1, 2, 3, or 4;

each $R^c$ is independently selected from deuterium, halogen, hydroxyl, cyano, mercapto, C2-C6 alkenyl, C2-C6 alkynyl, C1-C6 alkyl unsubstituted or substituted with one or more substituents selected from Group C, C1-C6 alkoxy unsubstituted or substituted with one or more substituents selected from Group C, $-(CH_2)_iNR^7R^8$, $-(CH_2)_iCONR^7R^8$, $-(CH_2)_iNR^7COR^8$, $-(CH_2)_iSO_2R^7$, $-(CH_2)_iSO_2NR^7R^8$, and $-(CH_2)_iOR^7$;

i is 0, 1, 2, or 3;

$R^7$ and $R^8$ are each independently H, deuterium, C1-C6 alkyl, C1-C6 haloalkyl, or C3-C6 cycloalkyl;

the substituents of Group C comprise: deuterium, halogen, hydroxyl, cyano, amino, C1-C6 alkoxy, C3-C6 cycloalkyl, and deuterated C1-C6 alkoxy;

$R^2$ is as defined in the corresponding claims.

**17.** The compound of formula (I) according to claim 16, or an isotopically labeled compound, enantiomer, diastereoisomer, or solvate thereof, or a pharmaceutically acceptable salt thereof, wherein the compound of formula (I) is selected from the following structures:

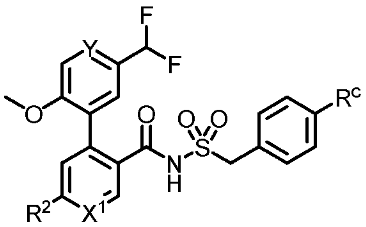

(IV-2)

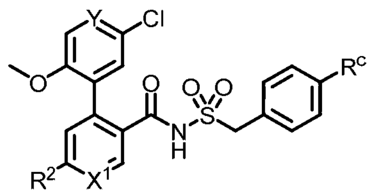

(IV-2a)

wherein

$X^1$ and Y are each independently N or CH; preferably, $X^1$ is CH and Y is N;

$R^c$ is selected from halogen, hydroxyl, cyano, mercapto, C1-C6 alkyl unsubstituted or substituted with one or more substituents selected from Group C, C1-C6 alkoxy unsubstituted or substituted with one or more substituents selected from Group C, and $-(CH_2)_iOR^7$;

i is 0, 1, 2, or 3;

$R^7$ and $R^8$ are each independently H, deuterium, C1-C6 alkyl, C1-C6 haloalkyl, or C3-C6 cycloalkyl;

the substituents of Group C comprise: deuterium, halogen, hydroxyl, cyano, amino, C1-C6 alkoxy, C3-C6 cycloalkyl, and deuterated C1-C6 alkoxy;

$R^2$ is as defined in claim 16; preferably, $R^2$ is $CH_3$ or $CD_3$.

**18.** The compound of formula (I) according to claim 1, or an isotopically labeled compound, enantiomer, diastereoisomer, or solvate thereof, or a pharmaceutically acceptable salt thereof, wherein the compound of formula (I) is selected from the following compounds:

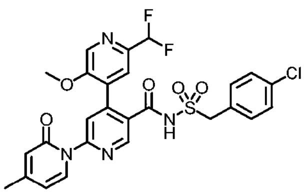
,
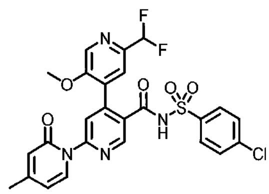
,
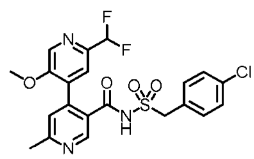
,
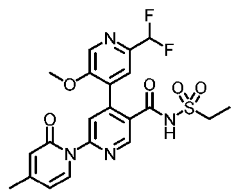
,
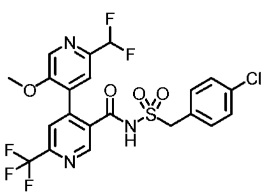
,
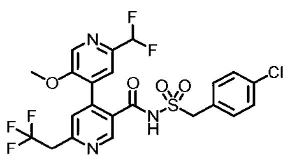
,
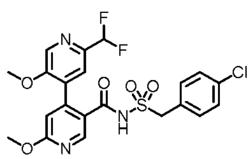
,
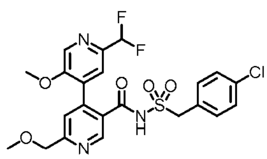
,

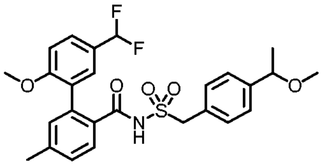, 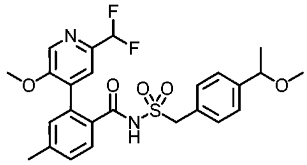, 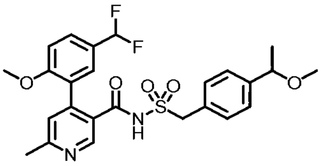,

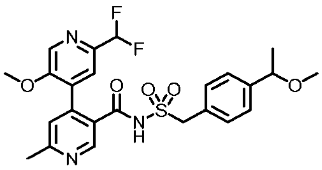, 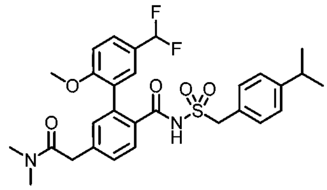, 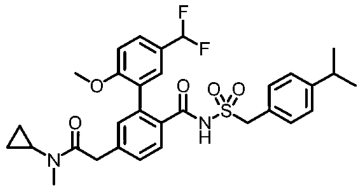,

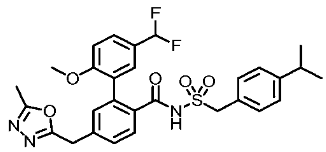, 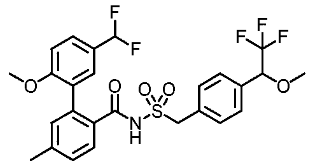, 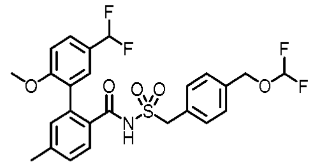,

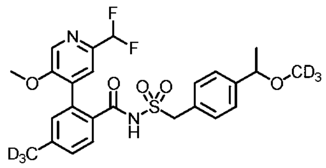, 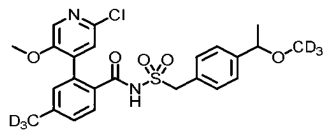, 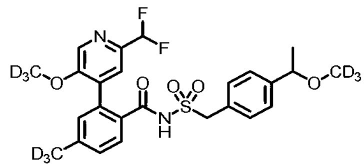,

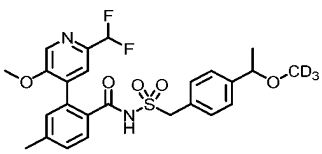, 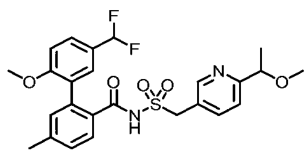, 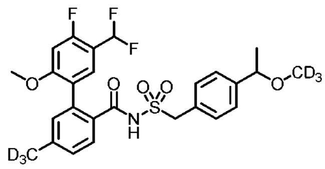,

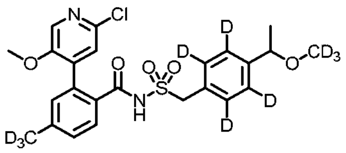, 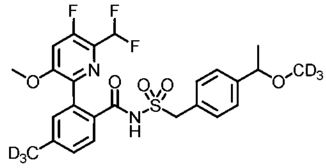, and 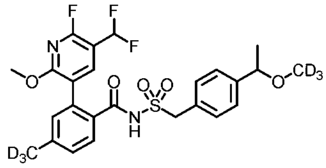.

**19.** A pharmaceutical composition comprising the compound of formula (I) according to any one of claims 1 to 18, or an isotopically labeled compound, enantiomer, diastereoisomer, or solvate thereof, or a pharmaceutically acceptable salt thereof, as well as a pharmaceutical excipient.

**20.** A use of the compound of formula (I) according to any one of claims 1 to 18, or an isotopically labeled compound, enantiomer, diastereoisomer, or solvate thereof, or a pharmaceutically acceptable salt thereof in the manufacture of a DNA polymerase $\theta$ inhibitor or a medicament; said medicament is one for treating and/or preventing diseases by inhibiting DNA polymerase $\theta$.

**21.** A use of the compound of formula (I) according to any one of claims 1 to 18, or an isotopically labeled compound, enantiomer, diastereoisomer, or solvate thereof, or a pharmaceutically acceptable salt thereof in the manufacture of a medicament for treating and/or preventing cancer.

**22.** A method of treating and/or preventing cancer, comprising administering to a patient a therapeutically effective amount of the compound of formula (I) according to any one of claims 1 to 18, or an isotopically labeled compound,

enantiomer, diastereoisomer, or solvate thereof, or a pharmaceutically acceptable salt thereof.

**INTERNATIONAL SEARCH REPORT**

International application No. **PCT/CN2024/119017**

**A. CLASSIFICATION OF SUBJECT MATTER**

C07D213/81(2006.01)i; A61P35/00(2006.01)i; C07D257/08(2006.01)i; A61K31/444(2006.01)i

According to International Patent Classification (IPC) or to both national classification and IPC

**B. FIELDS SEARCHED**

Minimum documentation searched (classification system followed by classification symbols)

IPC:C07D,A61P,A61K

Documentation searched other than minimum documentation to the extent that such documents are included in the fields searched

Electronic data base consulted during the international search (name of data base and, where practicable, search terms used)

CNTXT, ENTXT, VEN, STN(REGISTRY, CAPLUS, MARPAT), CNKI, 万方, Wanfang, Web of Science: 宇道生物, N-磺酰甲酰胺, 聚合酶0, 癌症, TMEJ, 修复, NUTSHELL BIOTECH, N-sulfonylcarboxamides, Pol0, cancer, Polymerase theta-mediated end joining, repir+, reparation, 结构式检索, structural formula search

**C. DOCUMENTS CONSIDERED TO BE RELEVANT**

| Category* | Citation of document, with indication, where appropriate, of the relevant passages | Relevant to claim No. |
|---|---|---|
| X | CN 104892535 A (QIDONG DONGYUE PHARMACEUTICAL CO., LTD. et al.) 09 September 2015 (2015-09-09)<br>description, paragraph 0007 | 1-3, 9 |
| X | LI, Nan et al. "Rapid Synthesis of Spirodienones via Electrochemical Dearomative Spirocyclization in Flow"<br>*Organic Chemistry Frontiers*, Vol. 9, No. (23), 13 October 2022 (2022-10-13), pp. 6586-6591<br>page 6587, Scheme 2 | 1-3, 9 |
| X | PERSON Florent et al. "N-Tosylcarboxamide as a Transformable Directing Group for Pd-Catalyzed C-H Ortho-Arylation"<br>*Organic Letters*, Vol. 14, No. (7), 22 March 2012 (2012-03-22), pp. 1827-1829<br>page 1828, Table 1, and Scheme 1 | 1-3, 5-9, 11 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **12 December 2024** | **23 December 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/119017**

**C. DOCUMENTS CONSIDERED TO BE RELEVANT**

| Category* | Citation of document, with indication, where appropriate, of the relevant passages | Relevant to claim No. |
|---|---|---|
| X | MIURA Hiroki et al. "Ruthenium-Catalyzed Addition of Aromatic Amides to Internal Alkynes and Subsequent Intramolecular Cyclization for the Atom-Economical Synthesis of Isoindolinones" *Journal of Organic Chemistry,* Vol. 82, No. (2), 21 December 2016 (2016-12-21), pp. 1231-1239<br>Supporting Information, and page 13 | 1-3, 9 |
| X | American Chemical Society. "RN NUMBER" *STN REGISTRY database,* 15 November 2022 (2022-11-15), pp. 1-9<br>compounds with RNs being 2852791-22-9, 2852791-24-1, 2852791-25-2, 2852791-26-3, 2814533-94-1, 2812107-08-5, 2811398-51-1, 2371052-39-8, 1808882-54-3, 1808852-30-3, 1808849-67-3, 1808723-26-3, 1808323-63-8, 2814391-14-3, 2800420-16-8, 2771616-07-8, 2637295-57-7, 2636997-73-2 and 2636990-61-7, and the structural formulae thereof | 1-3, 9-10 |
| X | ZHANG Sheng et al. "Electrochemical Formation of N-Acyloxy Amidyl Radicals and Their Application Regioselective Intramolecular Amination of sp2 and sp3 C-H Bonds" *Organic Letters,* Vol. 20, No. (12), 04 June 2018 (2018-06-04), pp. 3443-3446<br>page 3444, figure 2 | 1-3, 9 |
| A | WO 2020082921 A1 (ABBISKO THERAPEUTICS CO., LTD.) 30 April 2020 (2020-04-30)<br>claims | 1-22 |

Form PCT/ISA/210 (second sheet) (July 2022)

**INTERNATIONAL SEARCH REPORT**

International application No.
**PCT/CN2024/119017**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **22**
because they relate to subject matter not required to be searched by this Authority, namely:

Claim 22 relates to a method for treating a human or animal body. Therefore, the subject matter of claim 22 is a subject matter for which no search is required by the International Searching Authority. Nevertheless, a search is still carried out on the basis of the pharmaceutical use of the compound, etc.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2024/119017**

| Patent document cited in search report | Publication date (day/month/year) | Patent family member(s) | Publication date (day/month/year) |
|---|---|---|---|
| CN 104892535 A | 09 September 2015 | None | |
| WO 2020082921 A1 | 30 April 2020 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 2023112025210
- CN 2024103752865
- WO 2023233295 A1


### Non-patent literature cited in the description


- **HANAHAN, D.** ; **WEINBERG, R.A.** Hallmarks of cancer: The next generation. *Cell*, 2011, vol. 144
- **MADDISON ROSE et al.** PARP Inhibitors: Clinical Relevance, Mechanisms of Action and Tumor Resistance.. *Front. Cell Dev. Biol.*, vol. 8, 564601
- **CHANG HHY et al.** Non-homologous DNA end joining and alternative pathways to double-strand break repair.. *Nat Rev Mol Cell Biol.*, 2017, vol. 18 (8), 495-506
- **SFEIR A** ; **SYMINGTON LS.** Microhomology-Mediated End Joining: A Back-up Survival Mechanism or Dedicated Pathway. *Trends Biochem Sci.*, 2015, vol. 40 (11), 701-714
- **KENT T et al.** Mechanism of microhomology-mediated end-joining promoted by human DNA polymerase θ. *Nat Struct Mol Biol.*, 2015, vol. 22 (3), 230-7
- **BEAGAN K et al.** Drosophila DNA polymerase theta utilizes both helicase-like and polymerase domains during microhomology-mediated end joining and interstrand crosslink repair.. *PLoS Genet.*, 2017, vol. 13 (5), e1006813
- **MATEOS-GOMEZ et al.** The Helicase Domain of Polθ Counteracts RPA to Promote Alt-NHEJ.. *Nat. Struct. Mol. Biol.*, 2017, vol. 24 (12), 1116-1123
- **CECCALDI, R. et al.** Homologous-recombination-deficient tumours are dependent on Polθ-mediated repair.. *Nature*, 2015, vol. 518, 258-262
- **CECCALDI R et al.** Homologous-recombination-deficient tumours are dependent on Polθ-mediated repair.. *Nature.*, 2015, vol. 518 (7538), 258-62
- **SHIMA N et al.** The mouse genomic instability mutation chaos1 is an allele of Polq that exhibits genetic interaction with Atm.. *Mol Cell Biol.*, 2004, vol. 24 (23), 10381-9
- **WANG Z et al.** DNA polymerase θ (POLQ) is important for repair of DNA double-strand breaks caused by fork collapse. *J Biol Chem.*, 2019, vol. 294 (11), 3909-3919
- **CECCALDI R et al.** Homologous-recombination-deficient tumors are dependent on Polθ-mediated repair.. *Nature*, 2015, vol. 518 (7538), 258-62
- **MATEOS-GOMEZ PA et al.** *Nature*, 2015, vol. 518 (7538), 254-7
- **Z. WANG et al.** DNA polymerase (POLQ) is important for repair of DNA double-strand breaks caused by fork collapse.. *J. Biol. Chem.*, 2019, vol. 294, 3909-3919
- **R.J. KUMAR et al.** Hyperactive end joining repair mediates resistance to DNA damaging therapy in p53- deficient cells.. *bio Rxiv*, 03 April 2020
- **P. HEINRICH STAHL** ; **CAMILLE G. WERMUTH**. Handbook of Pharmaceutical Salts: Properties, Selection, and Use. 2011
- Pharmacopoeia of the People's Republic of China. 2020
- **PAUL J SHESKEY** ; **BRUNO C HANCOCK** ; **GARY P MOSS** ; **DAVID J GOLDFARB**. Handbook of Pharmaceutical Excipients. 2020